(19) **Europäisches Patentamt / European Patent Office / Office européen des brevets**

(11) **EP 3 400 050 B1**

(12) # EUROPEAN PATENT SPECIFICATION

(45) Date of publication and mention of the grant of the patent:
**23.12.2020 Bulletin 2020/52**

(21) Application number: **17735765.4**

(22) Date of filing: **04.01.2017**

(51) Int Cl.:
*A61D 7/00* (2006.01)  *A61P 5/24* (2006.01)
*A61P 15/00* (2006.01)  *A61M 37/00* (2006.01)
*C08L 53/02* (2006.01)  *A61K 47/32* (2006.01)
*A61F 6/14* (2006.01)  *A61K 9/00* (2006.01)
*A61P 43/00* (2006.01)  *A61M 31/00* (2006.01)

(86) International application number:
**PCT/AU2017/050005**

(87) International publication number:
**WO 2017/117627 (13.07.2017 Gazette 2017/28)**

(54) **DRUG RELEASE DEVICE AND USE**

WIRKSTOFFFREISETZUNGSVORRICHTUNG UND VERWENDUNG

DISPOSITIF DE LIBÉRATION DE MÉDICAMENT ET UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.01.2016 AU 2016900002**

(43) Date of publication of application:
**14.11.2018 Bulletin 2018/46**

(73) Proprietor: **Jurox Pty. Ltd.**
**Rutherford, NSW 2320 (AU)**

(72) Inventors:
• **SIMPSON, Graham**
**Tuggerah**
**New South Wales 2259 (AU)**
• **LOGAN, Paul**
**Charlestown**
**New South Wales 2290 (AU)**
• **GRAHAM, Janease**
**Abersglasslyn MSW, 2320 (AU)**
• **RANASINGHE, Rane**
**Lakelands**
**New South Wales 2282 (AU)**

(74) Representative: **Mewburn Ellis LLP**
**Aurora Building**
**Counterslip**
**Bristol BS1 6BX (GB)**

(56) References cited:
EP-A1- 1 864 655        WO-A2-2012/099406
AR-A1- 054 310          US-A- 4 585 451
US-A1- 2001 020 151     US-A1- 2002 107 330
US-A1- 2003 235 602     US-A1- 2004 142 012
US-A1- 2004 142 012     US-A1- 2009 043 276
US-A1- 2010 285 097     US-A1- 2012 034 306
US-A1- 2014 188 057     US-A1- 2015 342 894
US-A1- 2015 342 894     US-B1- 6 264 638

• **Anonymous: "Overview of materials for Styrenic Elastomer (TES", MatWeb - Material Property Data, XP009513291, Retrieved from the Internet: URL:http://www.matweb.com/search/DataSheet.aspx?MatGUID=2f57f75f8afa43e39ece09ab127bd0f5 [retrieved on 2017-02-16]**
• **"Overview of materials for Low Density Polyethylene (LDPE), Film Grade", MatWeb - Material Property Data, 12 November 2017 (2017-11-12), XP055433970, Retrieved from the Internet: URL:http://www.matweb.com/search/DataSheet.aspx?MatGUID=9ff98d958a714b2a8a00990a929d6f14 [retrieved on 2017-02-16]**

**Description**

**CROSS-REFERENCE TO RELATED APPLICATIONS**

The present application claims priority from Australian Provisional Patent Application No 2016900002 filed on 4 January 2016.

**TECHNICAL FIELD**

The present disclosure relates to a drug release device and its use in delivering one or more drugs to an animal.

**BACKGROUND**

Drug delivery to an animal may occur via a variety of routes including by injection, enteral delivery and mucosal delivery. The administration of drugs via the mucosal route provides a therapeutic effect as the permeation of significant amounts of a drug is permitted through the mucosal membrane. The mucosal routes for drug delivery include buccal/oral route, nasal route, ocular route, vaginal route and gastrointestinal route.

Drug delivery can be achieved using a drug release device. For example drug delivery to the vaginal mucosal membrane of a cow can be achieved using an intra vaginal device carrying the drug to be administered. Intra vaginal devices can be used to synchronise the estrus cycles of dairy and beef cattle and allow artificial insemination to be performed at a convenient and predictable time to a number of cattle. Estrus synchronisation and Artificial Insemination (AI) regimes are of great value to the beef and dairy industry as they effectively shorten the breeding and calving seasons thereby allowing for optimal use of time, labour and resources.

There are disadvantages associated with the limited number of drug release products for animals that are commercially available.

There is a need for alternate devices that are able to release one or more drugs to an animal.

Any discussion of documents, acts, materials, devices, articles or the like which has been included in the present specification is not to be taken as an admission that any or all of these matters form part of the prior art base or were common general knowledge in the field relevant to the present invention as it existed before the priority date of each claim of this application.

US2015/342894 relates to drug delivery systems and related methods of use. The drug delivery systems can include one or more particles, each of which can include a biologically active compound dispersed therein. The drug delivery systems can also be configured to exhibit zero-order or near-zero-order release of the biologically active compound. The particles can be cylindrical or rod-like in shape, and can include a polymeric inner matrix having a biologically active compound dispersed therein, and a polymeric outer layer disposed at least partially around the inner matrix.

**SUMMARY**

Disclosed herein is a drug release device containing one or more drugs that is adapted to contact a mucosal membrane of an animal and is capable of releasing the one or more drugs to the animal. The device may be configured to a geometry that enables positioning at the site of a mucosal membrane of an animal such as retention in a body cavity of an animal, or implantation parentally in an animal. Unexpectedly, it has been found that a device with a drug delivery component made of a specific Thermoplastic Elastomer (TPE) polymer formulation impregnated with the one or more drugs, provides an improved device that is capable of releasing one or more drugs in an amount that is able to provide a therapeutic effect.

The drug release device disclosed herein comprises a drug release component comprising one or more TPE polymers impregnated with one or more drugs, wherein at least one of the TPE polymers is polystyrene-*block*-poly(ethylene-*co*-butylene)-*block*-polystyrene (SEBS)

According to one aspect disclosed herein, there is provided a drug release device comprising a drug release component and a support, wherein the drug release component comprises one or more thermoplastic elastomer (TPE) polymers and is impregnated with one or more drugs, and at least one of the TPE polymers is polystyrene-*block*-poly(ethylene-*co*-butylene)-*block*-polystyrene (SEBS) and the drug release component is moulded to the support.

According to another aspect disclosed herein, there is provided a drug release device comprising a drug release component and a support, wherein: the drug release component comprises one or more thermoplastic elastomer (TPE) polymers and is impregnated with one or more drugs; at least one of the TPE polymers is polystyrene-*block*-poly(ethylene-*co*-butylene)-*block*-polystyrene (SEBS) the drug release component has a hardness in the range of 20-90 Shore A, as measured by a Type A Durometer in accordance with ASTM D2240, and has a melt point in the range of about 80 to 250°C; the support has a melt point in the range of about 70-200°C; and the drug release component is over-moulded on the support.

According to yet another aspect, there is provided a drug release device comprising a drug release component and a support, wherein, the drug release component comprises one or more thermoplastic elastomer (TPE) polymers and one or more polyolefins and is impregnated with one or more drugs; wherein, at least one of the TPE polymers is polystyrene-*block*-poly(ethylene-*co*-butylene)-*block*-polystyrene (SEBS); the drug release component has a melt point in the range of about 80 to 250°C and the support has a melt point in the range of about 70-200°C; and the drug release component is over-moulded on the support.

In one embodiment, the present disclosure provides a device according to the above aspect, wherein the device is configured for contacting a mucosal membrane of an animal. The device may be contacted with the mucosal membrane for a period of time to allow the drug to release from the drug release component to the mucosal membrane.

The device disclosed herein is suitable for retention at the vaginal, uterine endometrium, penile, rectal, nasal, olfactory, tracheal, bronchial, oesophageal, gastric, intestinal, ocular and oral mucosal membrane.

The device disclosed herein may be used as a subcutaneous, intramuscular or intra-vaginal implant.

In one embodiment, the device is configured for insertion into a vaginal cavity of an animal. In another embodiment, the device is configured for insertion into a vaginal cavity of an animal, retention therein for a period of time and removal from the vaginal cavity.

Described herein, there is provided a method of delivering one or more drugs to an animal, the method comprising the steps of contacting the device disclosed herein with a mucosal membrane of an animal for a period of time sufficient to allow the drug to be released from the device to the mucosal membrane.

Described herein is the use of a device disclosed herein, to deliver a drug to a mucosal membrane of an animal. The mucosal membrane can be the vaginal cavity of an animal.

According to yet another aspect, there is provided a device disclosed herein when used to deliver a drug to a mucosal membrane of an animal. In one embodiment, the mucosal membrane is the mucosal membrane of the vaginal cavity.

In another embodiment, progesterone is released to the mucosal in an amount effective to achieve a minimum progesterone plasma concentration of 2 ng/mL in the blood stream of the animal.

Described herein is a method of achieving in an animal a plasma progesterone concentration of greater than 2 ng/mL for a period of at least 7 days, said method comprising, contacting the device as disclosed herein with a mucosal membrane of the animal, for a period of time, typically about 5-7 days, sufficient to allow release of progesterone from the device to the mucosal membrane of the animal.

Also disclosed herein is a method of manufacturing the device disclosed herein.

There is disclosed herein a method comprising impregnating a drug into a polymer matrix comprising a block copolymer of butadiene or isoprene monomers and one or more ethylene and/or styrene monomers to form an impregnated polymer matrix, and moulding the impregnated polymer matrix to form a drug release component for a device disclosed herein.

Described herein is a method of manufacturing the device disclosed herein, the method comprising blending one or more drugs into a polymer matrix comprising one or more TPE-S block copolymers; wherein the TPE-S block copolymer is comprised of one or more elastomeric blocks selected from the group consisting of butadiene, isobutylene, propylene and isoprene monomers and one or more thermoplastic blocks selected from the group consisting of styrene and α-methylstyrene monomers, to form a drug impregnated polymer matrix, and moulding the drug impregnated polymer matrix onto a support to form the drug release device disclosed herein.

Described herein is a method of manufacturing the device disclosed herein, the method comprising blending one or more drugs into a polymer matrix comprising one or more TPE-S block copolymers and one or more polyolefin; wherein the TPE-S block copolymer is comprised of one or more elastomeric blocks selected from the group consisting of butadiene, isobutylene, propylene and isoprene monomers and one or more thermoplastic blocks selected from the group consisting of styrene and α-methylstyrene monomers, to form a drug impregnated polymer matrix, and moulding the drug impregnated polymer matrix onto a support to form the drug release device disclosed herein.

Also disclosed herein is a device for use in oestrus synchronisation of a herd.

In one embodiment, there is provided a device disclosed herein when used in herd oestrus synchrony.

The device disclosed herein is suitable for use in animals selected from the group consisting of: pigs, hind gut fermenters such as horses, ruminant animals including cows, goats, alpacas and smaller animals such as dogs and cats and humans.

The device disclosed herein is suitable for use in estrus synchronisation of animals. The device is suitable for use in estrus synchronisation of animals including ruminants including cows, buffalo, sheep and goats and post-gut fermenters including horses; pigs, dogs and cats.

**BRIEF DESCRIPTION OF DRAWINGS**

**Fig. 1A** shows the spine component of a device according to an embodiment disclosed herein. It shows a top view (I), front view (II), bottom view (III) and side view (IV) of the spine together with cross sections as indicated.

**Fig. 1B** shows the spine component of a device according to an embodiment disclosed herein. It shows a front view (I) and perspective view (II) together with cross sections as indicated.

**Fig. 1C** shows a device according to an embodiment disclosed herein. It specifically shows the spine of **Fig. 1B** over-moulded with the drug release component. It shows a cross-sectional side view (II), cross-sectional front view (III), schematic front view (IV), bottom view (V) of the device together with cross section D-D as shown.

**Fig. 2A** shows a schematic of an over-moulded drug delivery component of a device disclosed herein. Specifically it shows the spine of **Fig. 1A** over-moulded with the drug release component disclosed herein.

**Fig. 2B** shows a schematic of a device according to an embodiment disclosed herein with depiction of both the spine and over mould.

**Fig. 2C** shows a drug release device according to an embodiment disclosed herein.

**Fig. 3** shows a device retrieval line for a device according to an embodiment disclosed herein.

**Fig. 4** shows the release of P4 from a drug release formulation containing 6% P4 in $EVA_{40\%}$ compared to a representative sample of the CIDR® drug release material.

**Fig. 5** shows the comparison of P4 release from the drug release material of a CIDR® implant and a formulation containing 10%P4 in EVA;

**Fig. 6** shows the comparison of P4 release from formulations containing 8% P4 in $EVA_{40\%}$ and 8% P4 in Thermolast K;

**Fig. 7** shows the comparison of P4 release from a representative sample of the CIDR® to a formulation containing 12 % P4 in Mediprene®500452M with and without PEG;

**Fig. 8** shows the comparison of P4 release from a representative sample of the CIDR® device to a formulation containing10 % P4 in TPE (Mediprene®500452M);

**Fig. 9** shows Release of P4 from an entire CIDR® device to an equivalent amount of formulation containing 10% P4 in TPE (Mediprene®500452M) ;

**Fig. 10** shows P4 dissolution from a CIDR® device and an IVD prototype (device according to the present disclosure comprising Mediprene®500452M) into SVF monitored by HPLC;

**Fig. 11** shows plasma progesterone (ng/mL) content for each cow or heifer administered a commercially available CIDR® or IVD (a device according to the present disclosure comprising Mediprene®500452M) during part 2 period 1 of the study JX1302-K005 in Example 12. All data points were displayed on the chart as determined using a commercially available ELISA kit.

**Fig. 12** shows the average of baseline corrected Plasma progesterone (ng/mL) concentrations for each study group during the JX1302-K005 part 2 period 1 study . The error bars display 99% confidence intervals. Calculated plasma progesterone concentrations are displayed as determined using a commercially available ELISA kit.

**Fig. 13** shows plasma progesterone (ng/mL) content for each cow or heifer administered a commercially available CIDR® or IVD (a device according to the present disclosure comprising Mediprene®500452M) during part 2 period 2 of the JX1302-K005 study. All data points were displayed on the chart as recorded using a commercially available ELISA kit.

**Fig. 14** Shows the average Plasma progesterone (ng/mL) concentrations for each study group during the JX1302-K005 Part 2 period 2 study using a commercially available ELISA kit. The error bars display 99% confidence intervals.

**Fig. 15** (a) shows cross sections of the arm (left) and elongate body (right) of a device disclosed herein; and (b) shows cross sections of the arm (left) and elongate body (right) of a commercially available silicone based drug delivery device (CIDR®). It can be observed that the drug delivery material falls away from the H shaped spine in areas where the polymer is not held taut.

**Fig. 16** Shows the elongate body of a commercially available silicone based drug delivery device (CIDR®). Small slit like gaps can be seen in the silicone drug delivery coating, these are thought to be an artefact of the manufacturing process. Microorganisms may enter through these slits and form biofilms between the gap drug delivery material and spine of the CIDR® device (Fig. 15 (b)).

**Fig. 17** shows dissolution of progesterone from IVD prototypes (being devices disclosed herein comprising Mediprene®500452M) and commercially available CIDR® implants and of progesterone API (no matrix control) using 250 mL acetone at 40°C as a dissolution solution as described in Example 13 (Error bars represent 95% confidence intervals for data).

**Fig. 18** shows a Higuchi Model (Cumulative % of Progesterone released) for the Progesterone dissolution from Jurox IVD prototypes disclosed herein comprising Mediprene, the UK CIDR® and Australian CIDR® in Simulation Vaginal Fluid as described in Example 14.

**Fig. 19.** shows the amount of progesterone (mg/L) released from an IVD according to the present disclosure comprising Mediprene®500452M (AIVD1 and AIVD2) and the commercially available UK CIDR® (CIDR1.38_1 and CIDR1.38_2) and Australian CIDR® (CIDR1.9_1 and CIDR1.9_2) over 90 minutes. Specifically this relates to a study of Progesterone dissolution from Jurox IVD prototypes, the UK CIDR® and Australian CIDR® in an organic solution as described in Example 15.

**Fig 20.** shows initial amounts of progesterone (mg/L) released from the Jurox IVD (a device according to the present disclosure comprising Mediprene®500452M) and the commercially available CIDR® over 20 minutes. Specifically this relates to a study of Progesterone dissolution from Jurox IVD prototypes, the UK CIDR® and Australian CIDR® in an organic solution according to Example 15.

**Fig. 21** shows cumulative release of progesterone with increasing square root of time (minutes) for formulations disclosed herein with increasing progesterone dose as described in Example 16.

**Fig. 22** shows the flux plotted against the square root of mass/volume ratio for formulations disclosed herein with increasing progesterone dose per mock device. A linear relationship is seen between flux and square root of mass/volume ratio up to a maximum progesterone dose of value somewhere between 1.38 and 1.7 g/mock device according to Example 16

**Fig. 23** shows the average plasma progesterone (ng/mL) content for a cohort of 12 cows and heifers each administered a commercially available CIDR® (Treatment A) or IVD (Treatment B) according to the present disclosure comprising Mediprene®500452M in period 1 or period 2 of a two period cross over study JX1302-L011. Plasma progesterone concentrations were obtained using standard LCMS analytical techniques. The error bars represent 95% confidence intervals for treatment groups.

**Fig. 24** shows the JX1302-K005 Part 2 Period 1 plate 1 calibration curve.

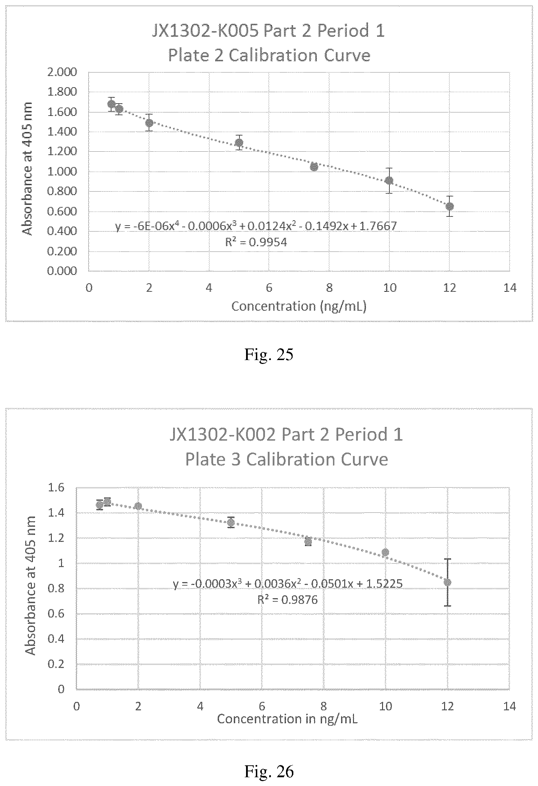

**Fig. 25** shows the JX1302-K005 Part 2 Period 1 plate 2 calibration curve

**Fig. 26** shows the JX1302-K005 Part 2 Period 1 plate 3 calibration curve.

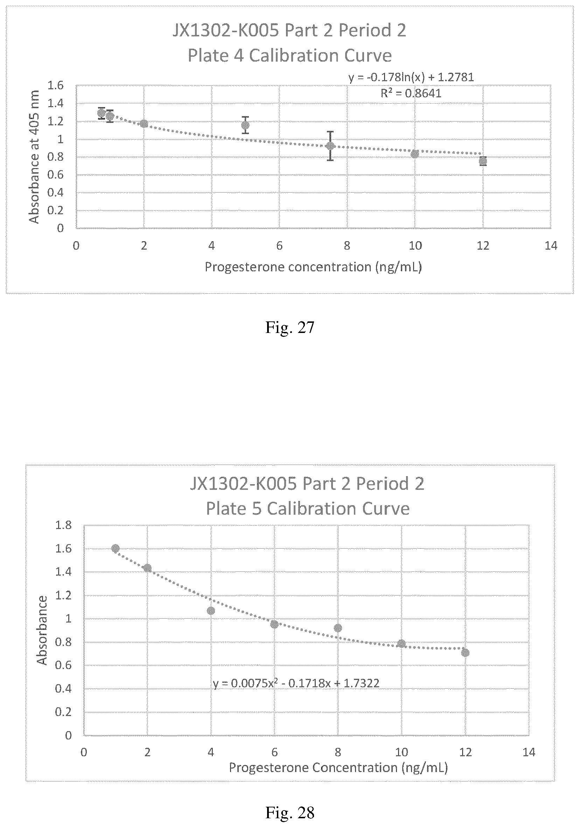

**Fig. 27** shows the JX1302-K005 Part 2 Period 1 plate 4 calibration curve.

**Fig. 28** shows the plate 5 calibration curve (for Day 0 and Day 1 data).

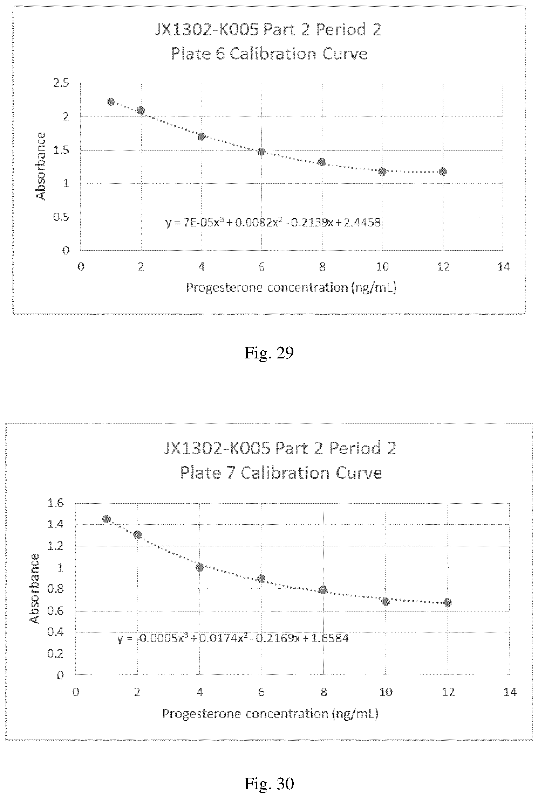

**Fig. 29** shows the plate 6 calibration curve (for Day 2 to Day 7 data).

**Fig. 30** plate 7 calibration curve (for Day 8 to Day 9 data).

## DETAILED DESCRIPTION

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

It will be understood that unless otherwise specified, generic polymer names are intended to encompass all possible structural isomers of these polymers including linear, branched and cross linked structures.

With regard to the definitions provided herein, unless stated otherwise, or implicit from context, the defined terms and phrases include the provided meanings. Unless explicitly stated otherwise, or apparent from context, the terms and phrases below do not exclude the meaning that the term or phrase has acquired by a person skilled in the relevant art. The definitions are provided to aid in describing particular embodiments, and are not intended to limit the claimed invention, because the scope of the invention is limited only by the claims. Furthermore, unless otherwise required by context, singular terms shall include pluralities and plural terms shall include the singular.

Throughout the present specification, various aspects and components of the invention can be presented in a range of formats. The range format is included for convenience and should not be interpreted as an inflexible limitation on the scope of the invention. Accordingly, the description of a range should be considered to have specifically disclosed all the possible sub-ranges as well as individual numerical values within that range, unless specifically indicated. For example, description of a range such as from 1 to 5 should be considered to have specifically disclosed sub-ranges such as from 1 to 3, from 1 to 4, from 1 to 5, from 2 to 4, from 2 to 5, from 3 to 5 etc., as well as individual and partial numbers within the recited range, for example, 1, 2, 3, 4, 5, 5.5 and 6. This applies regardless of the breadth of the disclosed range. Where specific values are required, these will be indicated in the specification.

Where a polymer may exist in a range of densities (e.g. polyethylene) it will be understood that all reasonable density preparations are encompassed unless otherwise specified; this includes very low density, low density, linear low density, medium density, high density and ultra-high density *etc.* All reasonable polymer molecular weights and polydispersities are also encompassed within the generic polymer name.

It will also be understood that all reasonable stereoisomers such as *cis-, trans-* and combinations of *cis-* and *trans-* isomers as well as syndiotactic, isotactic, atactic and polymers with all possible combinations of tacticity are referred to under the generic chemical name provided. Additionally it will be understood by a person skilled in the art that the degree of branching of a polymer can affect whether a polymer comprised of certain monomer are elastomers, thermoplastics or thermosetting plastics.

It is to be understood that the notation, *co*, within a polymer block, indicates the monomers in this block may exist in any arrangement *i.e.* random, statistical, alternating, repeating or block. For example, the A-B-A tri-block co-polymer: polystyrene-*block*-poly(ethylene-*co*-butylene)-*block*-polystyrene (SEBS), comprises of two polystyrene end blocks joined by a mid block of ethylene and butylene units in any random, statistical, alternating, repeating or block arrangement."

It will be understood that "therapeutically effective amount" as used herein, is the amount that is required to achieve the desired therapeutic effect of the drug.

The term "biocompatible" as used herein will be understood to mean not harmful or toxic to living tissue at the intended site of use.

The term "impregnated" or variations such as "impregnate", "impregnating" and "impregnation" as used herein, will be understood to mean blended or mixed throughout a medium. In relation to a specific medium being impregnated with a drug, it will be understood that the drug is blended or mixed throughout the medium, being the drug release component, TPE or a polymer matrix as disclosed herein. The blending or mixing of the drug in the medium is a result of the manufacturing process.

### Thermoplastic Elastomer (TPE) polymer

Thermoplastic elastomer (TPE) polymers (also referred to herein as thermoplastic elastomers or TPE's) are used in connection with the drug release device disclosed herein.

Polymeric materials can be elastomers or thermoplastics depending on their structure and physical properties. Without being bound to the following explanation, an elastomer or elastomeric polymer is a flexible polymeric material that is able to regain its shape after deformation. A thermoplastic or thermoplastic polymer is a polymeric material that is solid at room temperature and becomes pliable or mouldable at elevated temperature. Thermoplastics typically have favourable durability, strength, recyclability and ease of processing.

TPE's are heterogeneous materials comprised of regions of elastomeric polymeric material and regions of thermoplastic polymeric material. The term region, is to be understood to mean a three dimensional (3D) area of chemical space within the TPE polymer. The term elastomeric region will be used to describe a 3D area of the TPE that contains polymer chains and/or subunits of elastomeric nature. The term thermoplastic region will be used to describe a 3D area of a TPE material that contains polymer chains and/or subunits of a thermoplastic nature.

In TPE's the elastomeric and thermoplastic polymer chains and/or subunits may be associated with other like chains and/or subunits through intermolecular forces. They may also be joined covalently on the same polymer chain or part of another polymer chain. The elastic, flexible properties of the elastomeric regions and the durability, strength, recyclability and ease of processing of the thermoplastic regions contribute to the overall physical properties of the TPE.

Hereinafter, the term "subunits" may be used interchangeably with the term "blocks".

The one or more TPE polymers comprise subunits or blocks of monomers that are thermoplastic in nature and blocks of monomers that are elastomeric in nature.

Hereinafter, the collective term for the total of the "one or more regions" will be "component". Accordingly, the total of the one or more regions of elastomeric polymer will be referred to as the elastomeric component of the TPE and the total of the one or more regions of thermoplastic polymer will be referred to as the thermoplastic component of the TPE.

TPE polymers can generally be categorised as block copolymers or blends. A TPE that is in the form of a block copolymer (hereinafter referred to as a TPE block copolymer) comprises polymer chains of discrete blocks of monomers of a thermoplastic nature that are covalently linked to discrete blocks of monomers of an elastomeric nature. Accordingly, in block copolymers, the elastomeric regions of the TPE are made up of the one or more elastomeric subunit(s) and the thermoplastic regions of the TPE are made up of the one or more thermoplastic subunit(s).

A TPE that is in the form of a blend (hereinafter referred to as a "TPE blend" or "TPE polymer blend") comprises a thermoplastic dispersed with an elastomer or rubber. In TPE blends, the elastomer and thermoplastic polymer chains are blended and not covalently linked and may be bonded by intermolecular force. Accordingly, blends comprise regions of one or more elastomeric polymer(s) and regions of one or more thermoplastic polymer(s).

TPE block copolymers and TPE blends can be further categorised into classes according to their monomer content or structure. Block copolymer TPE classes include: Styrenic TPE polymers (TPE-S), poly(ester-co-ether) or poly(ester-co-amide) TPE polymers (TPE-E), polyurethane TPE polymers (TPE-U) and polyether block amide TPE polymers (PEBA). TPE blends may be further categorised as polyolefin blend TPE polymers (TPO) or vulcanized blend TPE polymers (TPV) (vulcanised thermoplastic elastomer).

Examples of specific thermoplastic elastomer (TPE) polymers disclosed herein include:

i) TPE block copolymers such as TPE-S block copolymers such as: polystyrene-*block*-polybutadiene-*block*-polystyrene) (SBS), polystyrene-*block*-polyisoprene-*block*-polystyrene (SIS), polystyrene-*block*-polyisobutylene-*block*-polystyrene (SIBS), polystyrene-*block*-poly(ethylene-*co*-propylene)-*block*-polystyrene (SEPS), polystyrene-*block*-poly(ethylene-*co*-propylene) (SEP) and polystyrene-*block*-poly(ethylene-*co*-butylene)-*block*-polystyrene (SEBS), polystyrene-*block*-poly(ethylene-*co*-poly(ethylene-*co*-propylene)-*block*-polystyrene (SEEPS), polyα-methylstyrene-*block*-poly(butadiene-*co*-isoprene)-*block*-α-methylstyrene, polystyrene-*block*-polydimethylsiloxane-*block*-styrene; polystyrene-*graft*-polybutadiene, polystyrene-*graft*-poly(ethylene oxide), poly[styrene-*per*-dimethylsiloxane], polyisobutylene-polystyrene (PIB-PS); TPUs and polyether-*block*-polyamide (PEBA) block copolymers of the commercially available Pebax® range, TPUs and PEBAs such as spandex or lycra, TPE-Us and PEBAs of the elastolan® range and TPE-E of the Hytrel® range; and

ii) TPE blends such as the TPOs including polypropylene blended with poly(ethylene-*co*-propylene) (EPM), polypropylene blended with poly(ethylene-*co*-propylene-*co*-diene) (EPDM), polypropylene blended with poly(ethylene-*co*-octene), polypropylene blended with poly(ethylene-*co*-α-olefin) (ECP).

Disclosed herein is a drug release component comprising one or more TPE polymers selected from the groups consisting of TPE-S, TPE-E, TPE-U, PEBA, TPO and TPV polymers.

In the drug release device disclosed herein, the one or more TPE polymers comprises an elastomeric component made up of one or more elastomeric regions and/or blocks and a thermoplastic component comprising one or more thermoplastic regions and/or blocks. The elastomeric region and/or blocks described herein may comprise one or more monomers selected from the group consisting of butadiene, isobutylene, propylene and isoprene and the thermoplastic region and/or blocks described herein may comprise one or more monomers selected from the group consisting of styrene, propylene, α-methylstyrene and ethylene.

In the drug release device disclosed herein, the drug release component comprises one or more styrenic TPE block co-polymers (TPE-S) comprising one or more elastomeric blocks which may be selected from the group consisting of butadiene, isobutylene, propylene and isoprene and one or more thermoplastic blocks which may be selected from the group consisting of styrene and α-methylstyrene.

Advantageously, when the drug release device of the present disclosure comprises a drug delivery component as described above, there is no need for a rate controlling barrier to control release of the drug from the device.

The TPE polymer is a block copolymer. The TPE polymer/s may be branched block copolymers. In another embodiment the TPE polymer/s are multiblock copolymers. In another embodiment the TPE polymer is a triblock copolymer or a diblock copolymer. In an additional embodiment the TPE polymer/s are starblock copolymers. In another embodiment the TPE polymer/s are an interpenetrating network within another polymer

The drug release component contains a TPE polymer in the form of an A-B-A triblock copolymer. Described herein, the A block may be polystyrene and the B block may be poly(butadiene-*co*-isoprene-*co*-isobutylene). Described herein, the A block may be polystyrene and the B block may be poly(butadiene-*co*-isoprene). Described herein, the A block may be polystyrene and the B block may be poly(isobutylene-*co*-isoprene). Described herein, the A block may be polystyrene and the B block may be poly(isobutylene-co-butadiene). Described herein, the A block may be polystyrene and the B block may be polybutadiene. Described herein, the A block may be polystyrene and the B block may be block is polyisoprene. Described herein, the A block may be polystyrene and the B block may be poly(isobutylene). In one embodiment the A block is polystyrene and the B block is poly(ethylene-*co*-butylene). Described herein, the A block may be polystyrene and the B block may be poly(3-methylbut-1-ene-*co*-ethylene-*co*-propylene. Described herein, A block may be polystyrene and the B block may be poly(3-methylbut-1-ene-*co*-ethylene-*co*-propylene-*co*-isoprene). Described herein, the A block may be polystyrene and the B block may be poly(3-methylbut-1-ene-*co*-ethylene-*co*-propylene-*co*-isoprene). Described herein, the A block may be polystyrene and the B block may be poly(ethylene-*co*-propylene).

A drug release components described herein contains a TPE polymer in the form of an A-B di-block copolymer where the A block is polystyrene and the B block is poly(ethylene-*co*-propylene).

The unsaturated hydrocarbon groups in the TPE polymer are fully reduced to saturated hydrocarbons For example the TPE polymer block comprised of polybutadiene is completely or partially hydrogenated to give a poly(ethylene-*co*-butylene) block. In another embodiment the polystyrene-*block*-polybutadiene-*block*-polystyrene is reduced to polystyrene-*block*-poly(ethylene-*co*-butylene)-*block*-polystyrene. Also described herein is a polyisoprene block can be partially or completely hydrogenated to give a poly(3-methylbut-1-ene-*co*-ethylene-*co*-propylene) block or a poly(isoprene-*co*-3-methylbut-1-ene-*co*-ethylene-*co*-propylene) block. This transformation can be performed by a person skilled in the art using hydrogen gas and an appropriate catalyst.

The TPE polymer may be fully or partially hydrogenated using methods known to the skilled person. Suitable methods are described, in US 3,364,549; US 3,670,633 and US 3,700,633.

As will be understood from the following, the TPE block copolymers disclosed herein may be mixed or blended with other polymers and/or additives to form blends or formulations containing TPE block copolymers. These may also be referred to herein as "TPE blends" or "TPE polymer blends" or 'TPE formulations".

In one embodiment, the TPE polymers as herein defined may be blended or mixed with one or more non-TPE polymers. In another embodiment, the TPE polymers as herein defined may be blended or mixed one or more non-polymer additives. In another embodiment the TPE polymers may be blended or mixed with one or more non-TPE polymers and one or more non-polymer additives.

Accordingly, the drug release component disclosed herein, in addition to the one or more TPE polymers disclosed above, may further comprise one or more non TPE polymers and/or one or more non-polymer additives.

The non-TPE polymers referred to herein include thermoplastic, elastomeric or thermosetting polymers. Examples of non-TPE polymers include polyolefins including polypropylene (PP), polyethylene (PE), polymethylpentene (PMP) and polybutene-1 (PB-1); polystyrene, polyesters, polylactic acid, poly(L-lactic acid), polycarbonate, poly(ethylene-*alt*-terephthalate), poly(butylene-*alt*-terephthalate), silicone oil, silicone resin, polydimethylsiloxane, phenyl vinyl methyl silicone, poly[1-(trimethylsilyl)-1-propyne], polyether aryl ketones, poly(ether ether ketone), poly(ether-*alt*-imide), poly(amide-*alt*-imide), poly(ethylene-*co*-vinyl acetate), polycaprolactone, poly(trimethylene terephthalate), polyhydroxyalkanoate, polyhydroxybutyrate, poly(3-hydroxybutyrate-*co*-3-hydroxyvalerate), poly(glycolic acid), poly(lactic-*co*-glycolic acid), poly(vinyl chloride), poly(ether sulfone), polyacrylate, polymethylmethacrylate, polysulfone, polyphenylene sulphide, poly(hydroxylethyl acrylate), polyhydroxylethylmethacrylate, poly-*p*-xylene, polytetrafluoroethylene, fluorinated ethylene propylene, perfluoroalkoxy, ethylene chlorotrifluoroethylene, ethylene tetrafluoroethylene, polyvinyl fluoride, poly(acrylonitrile-*co*-butadiene-co-styrene), poly(styrene-*co*-acrylonitrile), cyclic olefin copolymer, poly(methacylate-*co*-acrylonitrile-*co*-butadiene-*co*-styrene), poly(styrene-*co*-butadiene), acrylics, polyurethanes, acetals, Nylon 6, Nylon 66, Nylon 12, Nylon 6/12.

In one embodiment the non TPE polymer's are biocompatible.

In one embodiment TPE polymers are blended with 1-99% of one or more of the non TPE polymer/s.

In one embodiment, the effect of including non TPE polymers and/or non-polymer additives is to enhance the association between elastomeric and thermoplastic regions.

The non-polymer additives referred to herein include colouring agents, static dissipative compounds, antioxidants, UV stabilisers, salts, softeners, oils, biological compatible plasticizers, mould release agents and fillers.

In one embodiment the non-polymer additives are biocompatible.

The non-polymer additives may be blended with the TPE and non-TPE polymers disclosed herein in resin and/or granular form using methods of plastics compounding known to those skilled in the art.

In one embodiment the TPE polymer is blended with one or more oils including paraffin, isobutylene oil and silicone oil.

In another embodiment, the drug release component may include one or more of the following additives: calcium carbonate, 9,9-bis(methoxymethyl)fluorene, silica, one or more fatty acid salts selected from one or more of the following: magnesium, sodium, potassium and calcium salts of fatty acids; and povidone. In one embodiment, these additives are blended with the one or more TPE polymers and one or more drugs . In another embodiment, these additives are blended with the one or more TPE polymers, one or more drugs and polyolefin.

In one embodiment the TPE polymer is blended with one or more biocompatible plasticizers including : paraffin oil, silicone oil, isobutylene oil and phthalates.

In one embodiment the TPE polymer is blended with mould release agents to aid part removal from the mould during manufacture. These include to paraffin oil, polyvinyl alcohol, wax release agents, silicone agents and polymeric release systems.

In one embodiment the TPE polymer is blended with one or more additives to stabilise the polymer. These include: antioxidants; such as hindered phenols with thiodipropionate synergists; UV stabilisers such as benzotriazoles, titanium dioxide or carbon black and/or anti-ozonates such as ethylene vinyl acetate (EVA) and microcrystalline waxes.

In one embodiment inorganic fillers, such as calcium carbonate, amorphous silica or talc are mixed or blended with the one or more TPE polymers.

In one embodiment static dissipative agents are mixed or blended with the one or more TPE polymers. These include : carbon black, carbon nanotubes and carbon fibres.

In one embodiment commercially available colouring agents may be mixed or blended with the one or more TPE polymers.

In one embodiment the drug release component comprises a TPE block copolymer blended with a thermoplastic and oil. The drug release component comprises a TPE-S block copolymer comprising one or more elastomeric blocks which may be selected from the group consisting of butadiene, isobutylene, propylene and isoprene and one or more thermoplastic blocks which may be selected from the group consisting of styrene and $\alpha$-methylstyrene blended with a thermoplastic and oil. In one embodiment, the oil may be any biocompatible oil including paraffin oil, iosbutylene oil and silicone oil. In another embodiment, the thermoplastic may be a polyolefin including polyethylene (PE), polypropylene (PP), polymethylpentene (PMP) and polybutene-1 (PB-1)).

In one embodiment the drug release component comprises an A-B-A tri-block copolymer blended with a thermoplastic and an oil. Described herein, the A block may be poly(styrene), and the B block may be poly(butadiene-*co*-isoprene-*co*-isobutylene), the oil can be paraffin and the thermoplastic can be polypropylene. Described herein, the A block may be poly(styrene), and the B block may be poly(ethylene-*co*-propylene) and the thermoplastic can be polypropylene and the oil can be paraffin oil or silicone oil. In another embodiment the A block is poly(styrene) and B block is poly(ethylene-*co*-butylene) and the thermoplastic is polypropylene and the oil is paraffin oil or silicone oil. In a further embodiment the A block is poly(styrene) and B block is poly(ethylene-*co*-butylene) and the thermoplastic is polypropylene and the oil is paraffin oil, the filler is calcium carbonate or amorphous silica.

In one embodiment the blocks in the A-B-A triblock copolymer have a polydispersity index of less than 3 (preferably less than 2, more preferably less than 1.5 and most preferably less than 1.3).

Many of the above described block copolymers are commercially available individually as granules or resins or as a blend or formulation as discussed herein. They are also able to be prepared by a person skilled in the art using known procedures. Examples of suitable procedures include living anion polymerisation techniques, such as using alkyl lithium catalysts as described in US 4,764,572. Other suitable techniques are described in Jiri George Drobny, Handbook of Thermoplastic Elastomers. Chapter 5 Styrenic Block Co-polymers, Elsivier Inc., 2014.

The TPE polymers as herein described may be blended or mixed with non-TPE polymers. For example SEBS may be blended with polypropylene. The TPE polymers as herein defined may be blended or mixed with one or more non-TPE polymers and one or more non-polymer additives. For example, SEBS may be blended with polypropylene and a biocompatible oil. In one embodiment, the TPE polymers are in the form of a blend or a formulation, such as commercially available Mediprene® (available from Elasto, Hexpol) or Thermolast® (available from Kraiburg TPE GmbH & Co.) products. Examples of commercially available products that may be used in the drug release component disclosed herein include Mediprene® 500452M, Thermolast® K, Thermolast® 35 and Thermolast®45.

In one embodiment, the block copolymer TPE-S can be blended with a thermoplastic polymer. For example, a TPE-S can be blended with one or more polyolefins such as polyethylene (PE), polypropylene (PP), polymethylpentene (PMP), polybutene-1 (PB-1)

It will be understood by a person skilled in the art that polyolefins, such as polypropylene, may be constructed in different tacticities. Covalently joined blocks of atactic and isotactic monomer may form a thermoplastic elastomer where the atactic blocks remain flexible giving rise to elastomeric regions and the isotactic blocks crystallise at ambient temperature to give thermoplastic regions.

TPE polymers are a two phase material most commonly comprising of elastomeric polymer regions dispersed within a network of thermoplastic polymer regions. Unlike homogenous random co-polymers, that will display one glass transition temperature ($T_g$), heterogeneous TPE polymers will display two glass transitions at temperatures corresponding to both the glass transition temperature ($T_g$) of the elastomer block and the $T_g$ of the thermoplastic block.

In one embodiment the TPE polymers have a glass transition temperature ($T_g$) in the range of about -110 to 5°C, for the elastomeric component.

In another embodiment, the TPE polymers have a $T_g$ in the range of about 70°C to 180°C for the thermoplastic component.

In one embodiment the TPE polymers have a melting temperature ($T_m$) in the range of about 80-250°C or 120-250°C for the thermoplastic component.

In yet another embodiment the TPE polymers have a melting temperature ($T_m$) in the range of about 80-250°C or 120-250°C for an added polyolefin thermoplastic component e.g. a polyolefin such as polypropylene.

In another embodiment the TPE polymer blends have a $T_g$ in the range of about -110 to 5°C for the elastomeric component, a $T_g$ in the range of about 70-180°C for the thermoplastic component and a $T_m$ in the range of about 80-250°C. In another embodiment the TPE polymers have a $T_g$ in the range of about -110 to 5°C for the elastomeric component, a $T_g$ in the range of about 70-180°C for the thermoplastic component and a $T_m$ in the range of about 80-250°C or 150-250°C, for an added polyolefin component.

It will be appreciated that the drug release component of the device disclosed herein includes TPE polymers and additionally other ingredients. In one embodiment, the drug release component has a melt point in the range of about 80-250°C, 100-220°C, 150-200°C or 180-200°C.

These non TPE polymers may be purchased as a resin and blended with the TPE resin prior to injection moulding or compounded with a TPE resin using techniques known by those skilled in the art of injection moulding and plastics compounding such as melt mixing, dry blending or solution mixing. Suitable methodology is described in US 3,299,174 and Jiri George Drobny, Handbook of Thermoplastic Elastomers. Chapter 5 Styrenic Block Co-polymers, Elsivier Inc., 2014.

The inventors have found that a device according to the above aspect, wherein the drug release component comprises one or more TPE polymers comprising an elastomeric component and a thermoplastic component as herein before described, blended with a polyolefin and optionally with an oil; when impregnated with a drug, is capable of releasing the drug in a dosage suitable to provide a therapeutic effect. Other ingredients which do not affect the drug delivery characteristics of the device may be present including pigments, stabilisers, surfactants, waxes, flow promoters and fillers for ease of processing or to modify the final appearance. The device may be manufactured in a manner such that it meets all of the expectations and requirements of a medicinal product, (for example such requirements as uniformity of content and dose).

Accordingly, in one embodiment disclosed herein, the device comprises a drug release component that includes a TPE polymer comprising an elastomeric component and a thermoplastic component as herein before wherein the impregnated with one or more drugs, wherein a therapeutically effective amount of the one or more drugs is released from the device from the time that the device is inserted until the time the device is removed at a time period of about 7-14 days after insertion.

Devices containing drug release components that are formulated with a TPE and a polyolefin, have been found to adhere to a chemically compatible internal support of substantially equal or lesser melting point than the drug release component using an injection moulding process. Accordingly, in one embodiment disclosed herein, the device does not include a rate control barrier. In another embodiment, the drug release component does not include a rate control barrier.

Advantageously, the inventors have found that when the drug release component of the device disclosed herein is formulated with a polyolefin, the drug delivery component of the device may be adhered to a chemically compatible internal support of lesser melting point than the polyolefin component using an injection moulding process.

Accordingly, in one embodiment disclosed herein, the drug release component adheres to a central support in an over moulding process that allows the device to be constructed in a diverse variety of geometries without a gap between the layer of the drug delivery component and the support. This is advantageous for biological applications at a mucosal site. The close fit between the drug release component and the support means that establishment of microbial biofilms and infection is less likely in comparison with prior art devices. This results in a device that has improved sterility and biocompatibility.

The TPE polymer has a hardness in the range of 20-90 Shore A.

Unless stated otherwise, it will be understood that any reference to hardness or Shore hardness in relation to the device disclosed herein will mean a measurement of hardness as measured on a Type A or D Durometer in accordance with ASTM D2240). In relation to commercially available products, hardness may be referred to using an alternate standard.

Also disclosed herein are TPE's with a shore hardness of 20-80 Shore D (type D Durometer).

In yet another embodiment the TPE polymer is blended with other polymers and additives as described above the resulting blend may have a shore hardness less than 20 shore A. When the TPE polymer is blended with other polymers and additives as described above the resulting blend may have a hardness in the range of 20-90 shore A.

Also disclosed herein is a TPE polymer blended with other polymers, and additives with a shore hardness in the range of 20-90 shore D.

In one embodiment, the drug release component disclosed herein comprising the one or more TPE-S polymers has a hardness of : 20-90 Shore A, 20-55 Shore A, 20-50 Shore A, 25-45 Shore A, 35-45, Shore A, 30-50 Shore A, 30-45 Shore A or 40-45 Shore A, about 45 Shore A and about 35 Shore A.

The required hardness of the drug release component may be obtained by varying the amount of additive or the monomer blocks of the TPE polymer. For example, the hardness of a drug release component comprising TPE-S as herein described and one or more polyolefins (eg polypropylene) can be manipulated by varying the ratio of polyolefin to TPE-S and/or by varying the ratio of monomers in the polymer to modify the ratio of thermoplastic to elastomeric regions as discussed further below. Additionally the required hardness of the drug release component may be modified by the addition of a suitable oil.

The drug release component may comprise one or more of the TPE polymers described above. Suitable blends are commercially available, for example, TPE's from the Mediprene® (available from Elasto, Hexpo), Hytrel® (available from Dupont), TPS-HA series (Ho Hsiang Ching Co., Ltd.), SEPTONÂ®(available from KURARAY CO., LTD),, Krayton®(available from Krayton Polymer), and Thermolast® (available from Kraiburg), ranges may be used. Examples of commercially available products include Mediprene® 500452M, Thermolast® K, Thermolast® 35 and Thermolast®45.

The TPE polymer includes or consists of a polystyrene-*block*-poly(ethylene-*co*-butylene)-*block*-styrene polymer. Polystyrene-*block*-poly(ethylene-*co*-butylene)-*block*-styrene is available as a blend under the trade name Mediprene®, e.g. Mediprene 500452M. For Example, the commercially available Mediprene 500452M product includes SEBS, polypropylene and paraffin oil with the resulting Mediprene blend having a hardness of 45 Shore A. The thermoplastic elastomer, polystyrene-*block*-poly(ethylene-*co*-butylene)-*block*-styrene, is also available as a blend under the trade name Thermolast® K, e.g. Thermolast® K TFU4FTand has a hardness of 35 Shore A.

The TPE-S may be present in the formulations disclosed herein in an amount of 30-97 % w/w, 30-90 % w/w, 30-80% w/w, 30-70% w/w, 30-60% w/w, 30-50% w/w, 30-40% w/w, 40-45% w/w, 40-65%w/w, 45-55% w/w, 45-50% w/w, 48-52 % w/w about 50% w/w, 50-55% w/w or 55-60% w/w. The one or more polyolefins, such as polypropylene, may be present in such formulations in an amount of 3-50% w/w , 3-40% w/w, 3-30% w/w, 3-20% w/w, 3-10% w/w, 3-5% w/w, 10-20% w/w or 20-30% w/w.

In another embodiment, the block copolymer TPE can be blended with one or more thermoplastic polymers and one or more oils as hereinbefore defined. The oil may be present in such a formulation in an amount of about 5-60% w/w, 10-50% w/w, 20-50% w/w, 30-50% w/w, 40-50% w/w, 30-40% w/w, 20-30 % w/w. The quantities of the TPE polymer and thermoplastic polymer are as described above.

In one embodiment the block copolymer TPE-S in an amount of 30-90 % w/w, 30-60% w/w or 45-65% is blended with one or more thermoplastic polymers selected from the group consisting of polyethylene (PE), polypropylene (PP), polymethylpentene (PMP), polybutene-1 (PB-1) in an amount of 3-40%w/w, 3-20% w/w or 10-20% w/w; and an oil selected from the group consisting of paraffin oil, iosbutylene oil and silicone oil in an amount of 5-60% w/w, 10-50% w/w or 30-50% w/w.

The amount of TPE-S and polyolefin may be manipulated to achieve an overall TPE formulation with the desired harness. In one embodiment, the TPE formulation comprises TPE-S and polyolefin and has a hardness in the range of 20-50 Shore A, 30-50 Shore, or 35-45 Shore A (Durometer A).

In another embodiment, the TPE formulation comprises TPE-S, polyolefin and oil and has a hardness in the range of 20-50 Shore A, 30-50 Shore, or 35-45 Shore A (Durometer A).

In one embodiment, the drug release component comprises the above TPE formulation hereinbefore described. In one embodiment the drug release component has a hardness in the range of 20-50 Shore A, 30-50 Shore, or 35-45 Shore A (Durometer A).

It will be appreciated that the polystyrene-*block*-poly(ethylene-*co*-butylene)-*block*-styrene thermoplastic elastomer polymer products of the Thermolast® K and Mediprene® range vary in their relative ratio of each of the styrene, ethylene and butylene monomers in the polymer resin (and potentially also the chain length of polymer blocks). Resins in this range will also vary in the relative ratio in blends of other polymers such as polypropylene and their oil content and additive content. The composition of the resin formulation affects the mechanical properties of the resulting drug release component. For instance, it would be appreciated by those skilled in the art, that polymer resins that contain a block copolymer thermoplastic elastomer with longer chain styrene blocks are harder than those with shorter chain styrene blocks and longer chain butadiene blocks. TPEs blended with more polypropylene or polyethylene will be harder than TPE blended with a lesser amount. TPE blends containing a low amount of oil will be harder than those with a larger amount of oil. TPE blends with increased amounts of filler will be harder than those with a lesser amount.

In one embodiment, the device includes one or more TPE polymers and one or more non-TPE polymers. In one embodiment, the drug release component comprises one or more TPE polymers impregnated with one or more drugs and one or more non-TPE polymers impregnated with one or more drugs. In one embodiment, the drug release component comprises one or more TPE polymers impregnated with one or more drugs and one or more non-TPE polymers that is not impregnated with one or more drugs. In another embodiment the drug release component does not comprise one or more non-TPE polymers. In this embodiment any non-TPE polymers present in the device are not impregnated with one or more drugs for release in the vaginal cavity of an animal.

In one embodiment, the device disclosed herein is biocompatible.

In one embodiment, the device does not comprise ethylene vinyl acetate copolymers.

In another embodiment, the device does not include a biodegradable polymer.

**Shape of the device**

The device disclosed herein contains one or more drugs and is adapted to contact the mucosal membrane of an animal and is capable of releasing the one or more drugs to the animal. The device may be configured to a size and geometry that enables positioning at the site of a mucosal membrane of an animal such as retention in a body cavity of an animal, or implantation parentally in an animal.

In one embodiment, the device comprises an elongate body extending from a first end to a second end and two arms attached to and extending from the first end of the elongate body, wherein the two arms have arm tips and are moveable relative to the elongate body. It will be understood that when a force is applied to the arm tips the arms move relative to the elongate body by pivoting or bending about or around the point of attachment of the arms to the elongate body.

In one embodiment the two arms substantially mirror one another along the axis of the elongate body.

The arms have sufficient flexibility to allow them to be moved when external pressure or force is applied to allow the device to assume a number of configurations where the arms vary in their position relative to the elongate body. In assuming different positions, the angle between each arm and the elongate body changes. External pressure or force may be applied to the arms in a number of ways including pressure from: the hands of the operator of the device, the walls of the applicator into which the device has been positioned and the walls of the cavity of the animal in which the device has been inserted, such as the walls of the vagina of the animal. The arms may be moved away from or towards the elongate body. A force that moves the arms away from the elongate body may be referred to hereinafter as an "abducting force". A force that moves the arms towards the elongate bode may be referred to hereinafter as an "adducting force".

In one embodiment, each arm extends away from the elongate body so that the angle between the axis of the elongate body and the axis of each arm is in the range of approximately 5-180°. Examples of the angle between each arm and the elongate body include approximately: 5-180°, 10-180°, 20-180°, 30-180°, 40-180°, 50-180°, 60-180°, 70-180°, 80-180°, 90-180°, 100-180°, 110-180°, 120-180°, 130-180°, 140-180°, 150-180°, 160-180°, 170-180°, 45-120°, 50-115°, 55-110°, 60-105°, 65-100°, 70-95°, 75-90°, 80-100, 80-130, 85-95, 70, 80, 90°, 100, 110, 120, 130°, 140, 150, 160, 170, 180°. It will be understood that the angle is a measure of the angle between the arm and the elongate body with reference to the axis that extends in the direction of the overall length of the arm and the direction of the overall length of elongate body.

In one embodiment, external pressure is applied to the arms. In another embodiment, no external pressure is applied to the arms.

In one embodiment, the arms of the device extend from the elongate body such that the angle between the elongate body and each arm is about 80-100°. In one specific embodiment, the angle is about 85-95°. In another embodiment the angle is 85-90 or about 90° wherein the arms are approximately perpendicular to the elongate body. In such an embodiment, the device has a configuration that may be described as substantially "T" shaped. In another embodiment the angle is about 90-100° or 95-100° or about 95°. In such an embodiment, the device has a configuration that may be described as substantially "Y" shaped.

In another specific embodiment there is no external pressure on the arms. In such an embodiment, the device is said to be in an "unrestrained" configuration.

In one embodiment, the arms move independently of each other,

In one embodiment, a force or pressure may be applied to the arms such as to the arm tips, to cause movement of the arms away from the elongate body to increase the angle between each arm and the elongate body. The maximum angle able to be attained is up to approximately 180°. When the arms are in this position, the configuration of the device is such that the two arms are forced to lie substantially parallel to one another in the axis of the elongate body, to effectively provide a substantially elongate device. In such an embodiment, the device has a configuration that is substantially "I" shaped. In this position the device in in a "fully restrained" configuration. A fully restrained device is able to be positioned inside an insertion device in its restrained configuration.

In another embodiment, the angle between each arm and the elongate body is about 90-180. In one embodiment, when the angle between each arm and the elongate body is about 90-180, the device may be in a restrained configuration.

It is to be understood that the phrase "abduction of the arms" may be used to describe the movement of the arms to achieve an angle of up to 180° with the elongate body. Accordingly, in one embodiment the arms may be abducted at an angle of up to about 180° relative to the length of the elongate body.

When the arms experience no external pressure or force, the arms of the device resume an angle in the range of approximately 80-100° with the elongate body, i.e. resume a "T" shape where the arms are approximately perpendicular to the elongate body or resume a "Y" shape . In one embodiment, when the device is in an "unrestrained" position it is not in use.

The phrase "adduction of the arms" is to be understood to describe a movement of the arms to create a smaller angle between the arms and elongate body. The force that causes such a movement may be the elastic recoil of the arms back to their original "uninserted" position on removal of an abducting force, such as operator hands or applicator walls. Adduction of the arms is also possible by the application of a compressing force on the arms in the direction of the elongate body.

In one embodiment the arms are sufficiently inflexible so as to resist significant deformation, abduction or adduction by the forces or pressure exerted by muscles in the vaginal wall. In one embodiment, this force is in the range of about 1-20N, In one embodiment, the arms are moved by a force of about >3N, about >6N, about >9N, about >12N, about >15N, or about >20N. In a specific embodiment, the force to pivot the arms without deformation is approximately 12N.

It will be understood that "deformation " of the arms occurs if due to lack of resilience the arms bend or break.

In another embodiment, complete abduction of the arms to form a 180° angle with axis of the elongate body is possible. The device may assume this position when it is in use in an applicator ready to be inserted in the vaginal cavity of an animal or when it is being withdrawn from the vaginal cavity of an animal.

In another embodiment the drug release device is substantially "T" or "Y" shaped in its unrestrained configuration such that the arms are moveable relative to the elongate body in the plane of the "T" or Y" shape.

In one embodiment the device adopts a substantially "T" shaped configuration when it is in an unrestrained form. In another embodiment the device adopts a substantially "Y" shaped configuration when it is in a partially restrained form, such as inside the cavity of an animal including a vaginal cavity.

When the device is not in use, it will be understood that the configuration of the device is in an unrestrained form wherein the two arms have no force or pressure applied to them. When the device is in use, it will be understood that the two arms have been moved to assume an abducted or restrained state. This device is in a restrained form in an applicator, in a vaginal cavity of an animal or when being withdrawn/removed from the vaginal cavity.

The phrase "substantially "T" or "Y" or "I" shaped" is intended to mean that the device, when viewed in one plane has the appearance of the letter "T" or "Y" or "I" or variations thereof.

The length of the spine and the device disclosed herein may be in a range of 10 - 19cm, 10-18cm, 10-16cm, 12-18cm, 12-16cm, 12-15cm, 13-15 cm, 14-15cm or about 14, 14.5 or 15 cm. In one embodiment the length of the spine and device is about 14-15 cm.

It will be understood that the length of the spine (and device) is the distance from the end of the elongate body from which the arms do not extend, to the axis that extends from one arm tip to the other arm tip, such that the axis of the distance measured is substantially perpendicular to the axis between the two arm tips.

In one embodiment, the elongate body of the spine and device is in a range of 9 - 15 cm. In another embodiment, the elongate body of the device in a range of about 10- 14 cm. In another embodiment, the length of the elongate body of the spine and device is about 11-13 cm or about 12-12.5cm and is suitable for use in heifers or Brahman cattle.

The length of the elongate body will be understood to extend from the first end where the two arms are attached to the second opposite end, such that it does not incorporate any bending of the elongate body from the flexure of the arms. The span of the arms of the spine or device disclosed herein from one outer end to the other outer end, may be in the range of 12-18cm, 14-16cm or about 14, about 14, or about 15cm, or about 16cm.

The surface area of the spine, device and drug release component may be $\geq$120 cm$^2$. In another embodiment, the surface area of the spine, device and drug release component is less than 150 cm$^2$. In one embodiment, the surface area of the drug release component and the device is the same. In one embodiment, the surface area of the spine, device and drug release component is in the range of about: 120-150 cm$^2$, 120-150 cm$^2$, or about 128cm$^2$. In other embodiments it may be in the range of about 75-150 cm$^2$, 100-150 cm$^2$ or 124-125 cm$^2$.

In one embodiment, the surface area is the available release surface of the drug from the drug release component or the polymer matrix which includes the drug release component.

A device suitable for use in heifers and smaller cows such as *Bos taurus indicus* cattle breeds (e.g.Brahman) has a spine or device length of about 13-16cm, an elongate body length of about 11-13cm, an arm span of about 14-16cm and a spine or device surface area of about 122-126cm$^2$. In another embodiment suitable for use in heifers or Brahman cattle, the spine or device has a length of about 14-15cm, an elongate body length of about 11-12 cm, an arm span of about 14-15cm and a spine or device surface area of about 124-125 cm$^2$.

In one embodiment, the device is flexible, in that the two arms are able to be moved to different positions. In the "non-inserted position" the devices are substantially "T or Y shaped" wherein the arms extend away from the axis of the elongate body. To obtain an "insertion position", the arms move so that they are substantially in the same axis as the elongated body and substantially parallel to one another, thereby extending the length of each device to a length greater than the elongated body itself. The device assumes this position in the applicator. The device is inserted into the body cavity (e.g. vagina) of the animal using an applicator. The arms are positioned so that the device, in its "retention position", is in a Y shape and that the device is removed from the vagina by pulling a retrieval means, such as a cord, attached to an end of the elongate body opposite to the end the two arms are attached, such that the device adopts a narrower Y shape - being in between the "insertion" and "retention" positions.

In one embodiment, the body is substantially circular in cross-section, such that the device has curved surfaces. In this embodiment, the elongate body has no flat, planar surfaces. In another embodiment, the body is substantially circular in cross-section, but additionally includes multiple concave indentations along its length. This advantageously increases the surface area of the elongate body. In another embodiment, the arms are substantially semi-circular in cross-section such that when they are in a fully restrained position such that the angle between the arm and the elongate body is approximately 180°, the two arms lie parallel to one another to form a two part cylinder with a substantially circular cross section. Such a design has been found to facilitate efficient application, retention and removal.

**Support**

In one embodiment the device includes a support for the one or more thermoplastic elastomer polymers.

In another embodiment the one or more thermoplastic elastomer polymers is in contact with a support. The support may include, either partially or completely, a non-brittle plastic with high elastic memory and substantially high flexural modulus to resist substantial extension of the arms on exposure to the compression forces of the vaginal wall of for example a cow. Suitable plastics will only enable complete extension of the arms when the compression force applied to each arm tip in the direction perpendicular to the device elongate body exceeds about 3N, about 6N, about 9N, about 12N, about 15N, or about 20N.

Examples of suitable plastics for the support include one of the following or blends of two or more of the following polymers; polypropylene (PP), polyethylene, low density polyethylenes (LDPE), High density polyethylenes (HDPE), poly vinyl chloride (PVC), polystyrene, polyesters, polylactic acid, poly(L-lactic acid), polycarbonate, poly(ethylene-*alt*-terephthalate), poly(butylene-*alt*-terephthalate), silicone oil, silicone resin, polydimethylsiloxane, phenyl vinyl methyl silicone, poly[1-(trimethylsilyl)-1-propyne], polyether aryl ketones, poly(ether ether ketone), poly(ether-*alt*-imide), poly(amide-*alt*-imide), poly(ethylene-*co*-vinyl acetate), polycaprolactone, poly(trimethylene terephthalate), polyhydroxyalkanoate, polyhydroxybutyrate, poly(3-hydroxybutyrate-*co*-3-hydroxyvalerate), poly(glycolic acid), poly(lactic-*co*-glycolic acid), poly(vinyl chloride), poly(ether sulfone), polyacrylate, polymethylmethacrylate, polysulfone, polyphenylene sulphide, poly(hydroxylethyl acrylate), polyhydroxylethylmethacrylate, poly-*p*-xylene, polytetrafluoroethylene, fluorinated ethylene propylene, perfluoroalkoxy, ethylene chlorotrifluoroethylene, ethylene tetrafluoroethylene, polyvinyl fluoride, poly(acrylonitrile-*co*-butadiene-*co*-styrene), poly(styrene-*co*-acrylonitrile), cyclo olefin copolymer, poly(methacylate-*co*-acrylonitrile-*co*-butadiene-*co*-styrene), poly(styrene-*co*-butadiene), acrylics, polyurethanes, acetals, Nylon 6, Nylon 66, Nylon 12, Nylon 6/12.

According to one embodiment of the present disclosure, the support includes low density polyethylenes (LDPE), high density polyethylenes (HDPE), polypropylene (PP), polyvinyl chloride (PVC), Nylon 6, Nylon 66, Nylon 12 and Nylon 6/12.

According to one embodiment of the present disclosure, the support includes low density polyethylenes (LDPE), high density polyethylenes (HDPE), polypropylene (PP), polyvinyl chloride (PVC).

The support may be in the form of a spine. It is to be understood that the term spine refers to an internal or partially internal supporting apparatus for the drug release component. According to the present disclosure, the drug release component comprising the one or more thermoplastic elastomer polymers may be moulded over the spine. In one embodiment, the drug release component is chemically compatible with the support material with which it is in contact.

The support is made of a material that has a melt point in the range of 70-200°C. The inventors have found that a device having advantageous properties results when the drug release component comprising the one or more thermoplastic elastomer polymers has substantially similar or a higher melt point than that of the support material. Without being bound by theory, the inventors believe that enhanced adhesion of the TPE polymer blend to the support material results when the molten TPE polymer is able to melt more than 1 nm of the support material on contact in an over moulding process. This partial melting of the support material enables chemical and physical interactions between both polymers resulting in optimisation of adhesion of the two materials. Additionally, this partial melting of the support ensures that no cavities between the two materials exists. This is believed to result in the device of the invention being less vulnerable to shielding bacterial contamination from the innate antimicrobial defences of a mucous membrane cavity such as the vagina. Enclosed cavities such as gaps between a support and an over moulded material are shielded from the natural attrition of cervical and vaginal fluids and biofilms may quickly form at these sites.

In one embodiment, the drug release component adheres to the support. In another embodiment, the drug release component is melted onto the support.

The technique of over moulding will be understood by those skilled in the art and can be achieved using known techniques, such as those described in Douglas M. Bryce, 1996, Plastic Injection Moulding: Manufacturing Process Fundamentals volume 1, Society of Manufacturing Engineers.

The term "mould" and variations such as "moulded" and "moulding" that result from an over moulding process may be referred to herein as "mould" or "over mould" or variations thereof.

In one embodiment, the drug release component of the device has a higher melting point than the support material with which it is in contact. Advantageously, this results in the drug release component being adhered to the internal support in an over moulding manufacturing step. In one embodiment, the drug release component has long term adherence to the internal support following the over moulding manufacturing step.

In one embodiment, the one or more thermoplastic elastomer polymer is adhered to the support by over moulding of a TPE having a melt point in the range of 80 to 250°C onto a support having a melt point in the range of 70-200°C.

In another embodiment, the drug release component comprising the one or more thermoplastic elastomer polymer is adhered to the support by over moulding of a drug release component having a melt point in the range of 80 to 250°C onto a support having a melt point in the range of 70-200°C.

In yet another embodiment, the drug release component comprising the one or more thermoplastic elastomer polymer is adhered to the support by over moulding of a drug release component having a melt point in the range of 80 to 250°C onto a support having a melt point in the range of 70-200°C, wherein the melt point of the drug release component is higher than the melt point of the support.

In yet another embodiment, the drug release component comprising the one or more thermoplastic elastomer polymer is adhered to the support by over moulding of a drug release component having a melt point in the range of 80 to 250°C onto a support having a melt point in the range of 70-200°C, wherein the melt point of the drug release component is higher than the melt point of the support such the drug release component when molten is able to melt more than 1 nm of the support material on contact in an over moulding process.

Coating methods include, over moulding and dip coating.

In one embodiment, the drug release component comprising the one or more thermoplastic elastomer polymers is over-moulded onto the pre-formed support using an insert moulding process. In another embodiment, the one or more thermoplastic elastomer polymers is over-moulded onto the support using a two shot injection moulding process.

In yet another embodiment, the drug release component comprising the one or more thermoplastic elastomer polymer is adhered to the support by over moulding of the drug release component onto polypropylene or low density polyethylene or a polymer composition with a melt point lower than the drug release component which is in the range of 70-200°C.

In another embodiment adhesion of the TPE to the spine is aided by the addition of raised nodules on the spine. These nodules provide stability to the over moulded TPE and act like small keys to eliminate the possibility of movement of the TPE across the spine. Additionally these nodules aid in the central positioning of the spine in the mould cavity during the insert type over moulding process. In a further embodiment, holes are included in the TPE layer to ensure no air entrapment between the TPE layer and support material to aid adhesion between components. In another embodiment, positioning nodules are included at appropriate points on the mould cavity to centrally position of the spine during the insert type over moulding process. In another embodiment, recesses instead of, or in conjunction with, nodules are present on the spine to aid adhesion between the spine and the TPE. In yet another embodiment, longitudinal indentations are present on the spine to aid adhesion.

In another embodiment, the spine may be made more resistant to abduction of the arms by increasing the polymer resin thickness at the intersection of the arms with the elongate body.

In another embodiment, the spine may be made more resistant to abduction of the arms by increasing the elastic modulus of the polymer resin used to manufacture the spine.

In one embodiment the support material is substantially Y-shaped or substantially T-shaped. In one embodiment, the support is a substantially Y-shaped spine over-moulded with a thermoplastic elastomer as described above. In one embodiment, the resulting Y-shaped device has arms with a flexibility as described above.

Also disclosed herein is a device which does not include a support for the thermoplastic elastomer. The thermoplastic elastomer can have appropriate flexural modulus and elastic memory so as to enable it to be inserted into the vaginal cavity, retained for a period of time to release the drug and then removed from the vaginal cavity without the need for a support to provide the required degree of flexural modulus elastic memory. The device can be substantially Y-shaped or substantially T-shaped and the arms are flexible as described above.

In one embodiment, the device disclosed herein has sufficient resilience and memory to have only a small amount of permanent deformation after the arms are extended for insertion of the device. In one embodiment, the arms are substantially resistant to deformation so that they are only extended to a parallel position by a force of greater than about 3N, about 6N, about 9N, about 12N, about 15N, or about 20N.

In another embodiment, the device has a resilient memory less than nylon. Accordingly, the device disclosed herein in its non-inserted form is able to retain its structural integrity in the absence of any external force, especially in the absence of a force of greater than about 3N, about 6N, about 9N, about 12N, about 15N, or about 20N. Accordingly, the device disclosed herein is not limp in nature.

According to one aspect, the device comprises a drug release component which comprises a thermoplastic elastomer impregnated with a drug. The device may be used for the parenteral delivery of heat stable drugs or the delivery of heat stable drugs to a mucosal membrane. Suitable embodiments of this device for parenteral administration of this device are subcutaneous, intramuscular or intra-vaginal implants. The device may serve as an implantable structural support such as a medical scaffold, surgical sutures, barrier, etc. in addition to functioning as a drug delivery device. Suitable mucosal surfaces include vaginal, uterine endometrium, penile, rectal, nasal, olfactory, tracheal, bronchial, oesophageal, gastric, intestinal, ocular and oral mucosa. Suitable drugs include steroids, antibiotics, antifungals and analgesics. In one embodiment, the drug is progesterone. The device may also be of an appropriate geometry to be retained in a body cavity or at a mucosal membrane site.

In one embodiment, the drug release component consists only of one or more TPE's as described above.

The drug release component may be impregnated with a drug selected from the group consisting of steroidal hormones including: progesterone, megesterol acetate, progestogens, progestins, norethandrolone, delmadinone, etc.; antimicrobials including: penicillins, sulfonamides, tetracyclines, lincosamides, neomycin, etc.; anthelmintics including: pyrantel, piperizine, praziquantal, seditives such as medetomidine, acepromazine, chlorpromazine, diazepam, thiopentone sodium, etc.; analgesics including: fentanyl, etc.; and corticosteroids including: flumethasone, Triamcinolone acetonide, prednisolone, etc.

In one embodiment, the drug is progesterone.

In one embodiment, the surface area of the device is the available release surface for the drug. In one embodiment, the surface area of the device is in the range of 50-150 cm$^2$ and provides a total progesterone release surface of 50-150 cm$^2$. In another embodiment, only the one or more TPE's as described above provide the release surface.

In another embodiment, the drug release component is impregnated with progesterone so as to have greater than 5% by weight progesterone to the weight of the drug release component or polymer matrix.

In another embodiment, the drug release component of the device has a total drug load of at least 0.7 gram. In one particular embodiment the drug is progesterone.

In another embodiment, the progesterone in the drug release component or polymer matrix is less than 5 mm, preferably less than 2 mm and most preferably less than 1 mm away from said release surface.

In one embodiment, the drug release device disclosed herein comprises a drug release component comprising one or more thermoplastic elastomer (TPE) impregnated with progesterone (P4) over-moulded onto a support comprising one or more selected from the group comprising low density polyethylenes (LDPE), polypropylene and polyvinyl chloride (PVC) such that the resulting shape of the device is substantially Y- or T- shaped.

In one embodiment, the drug release device disclosed herein comprises a progesterone (P4) loaded thermoplastic elastomer (TPE) drug delivery component over-moulded onto a flexible polypropylene (PP) or polyvinyl chloride (PVC) spine such that the resulting shape of the device is substantially Y-shaped.

## Uses

The device disclosed herein is suitable for pigs, hind gut fermenters such as horses, ruminant animals including cows, goats, alpacas and smaller animals such as dogs and cats and humans. It will be appreciated by a person skilled in the art that the geometry of the device will need to be modified for different types of animals to accommodate the different shapes and sizes of the body cavity, mucosal membrane or implantation site.

In one embodiment, the device is suitable for retention at a mucosal membrane of pigs, hind gut fermenters such as mares, ruminant animals including cows, goats, alpacas and smaller animals such as dogs and cats and humans.

In one embodiment the device is suitable for retention at the vaginal, uterine endometrium, penile, rectal, nasal, olfactory, tracheal, bronchial, oesophageal, gastric, intestinal, ocular and oral mucosal membrane.

In one embodiment the device is suitable as a vaginal implant for female pigs, hind gut fermenters such as mares, ruminant animals including cows, goats, alpacas and smaller animals such as dogs and cats and humans. It will be appreciated by a person skilled in the art that the geometry of the device will need to be modified for different types of animals to be compatible with the different shapes and sizes of vaginas.

In one embodiment, the device is suitable for use in cows of the *Bos taurus* species including the subspecies: *Bos taurus taurus, Bos taurus indicus* and *Bos taurus primigenius.*

In one embodiment, the device is suitable for use in heifers and smaller cows.

In another embodiment, the device is suitable for use in *Bos taurus indicus* cows such as brahman cows.

In another embodiment, the device is suitable for use in *Bos taurus taurus* cows.

In one embodiment the device is suitable for use in lactating dairy and beef cows.

In one embodiment the device is suitable for use in ruminants such as cows, buffalo, sheep and goats and post-gut fermenters such as horses for estrus synchronisation. In another embodiment, the device is suitable for estrus synchronisation in pigs. In another embodiment, a smaller version of the device is suitable for use in small animals for estrus synchronisation such as for the breeding of show animals including dogs and cats using donor semen. An embodiment of this invention is a device with appropriate geometry for vaginal drug release in humans.

In one embodiment, there is disclosed a low dose device comprising progesterone in an amount of less than about 0.6g or in the range of about 0.4-0.6g. Such a device is envisaged to be useful for use in heifers, cows of low body weight or progesterone sensitive breeds such as Brahman cows and Brahman cross breeds.

In another embodiment, there is disclosed a high dose device comprising progesterone in an amount of greater than about 0.8g, or in the range of about 0.8- 2g . Such a device is envisaged to be useful for lactating dairy and beef cows.

In one embodiment disclosed herein, there is provided a durable and well tolerated drug release device that may be retained in the vagina of an animal such as a cow. This device consists of a drug delivery component comprised of a thermoplastic elastomer that imparts better elasticity, durability and flexibility than that of existing silicone devices. It has been found that a copolymer of styrene, butadiene and/or ethylene material gave ideal release of progesterone without the need for a rate controlling barrier.

In a preferred embodiment, the device disclosed herein uses a drug release component comprising a thermoplastic elastomeric with a balance between durability and flexibility and effective drug release. In a preferred embodiment a drug release component may be adhered to a support material without the aid of an adhesive as part of an insert moulding or two shot moulding process. This enables the manufacture of a diverse range of device geometries, sizes and surface features while maintaining contact between a drug delivery layer and the support material. Without this adhesion, a drug delivery material such as silicone will fall away from concave areas of the support material. This is undesirable as the unsupported drug delivery material is at risk of damage and creates a space sheltered from the innate defences of the mucus membrane where pathogenic microbes may replicate and form undesired biofilms placing the treated animal or human at greater risk of infection. In one embodiment, the TPE of the drug release component is flexible and durable enough to be moulded into 1mm layers requiring less drug delivery material than existing products. In another embodiment, the drug release component as described herein is flexible and durable enough to be moulded into 1mm layers requiring less drug delivery material than existing products.

Also described herein is a method of delivering one or more drugs to an animal comprising contacting the animal device described herein with a mucosal membrane of the animal for a period of time sufficient to allow the drug to be released from the device to the mucosal membrane.

The device can be removed from contact with the mucosal membrane.

The present disclosure provides a method of delivering a drug to a vaginal cavity of an animal comprising inserting into the vaginal cavity a device disclosed herein and retaining the device in the vaginal cavity for a period of time sufficient to allow the drug to be released from the device into the vaginal cavity.

The device can be removed from the vaginal cavity.

The device can be removed by a retrieval means attached to an end of the device.

There is disclosed use of a device disclosed herein to deliver a drug to the mucosal membrane of an animal. In one embodiment the mucosal membrane is the vaginal cavity of an animal.

According another aspect, there is provided a device as disclosed herein when used to deliver a drug to a mucosal membrane of an animal. In one embodiment, the mucosal membrane is the mucosal membrane of the vaginal cavity.

A therapeutically effective amount of the drug can be released to the mucosal membrane such as the mucosal membrane of the vagina and passed into the blood stream.

Progesterone can be released to the mucosal membrane in an amount effective to achieve a minimum progesterone plasma concentration of 2 ng/mL in the blood stream of the animal.

Also described herein is a method of achieving in an animal a plasma progesterone concentration of 2 ng/mL or greater for a period of at least 7 days, said method comprising contacting with mucosal membrane of an animal a device as disclosed herein, for a period of time, typically 5-7 days, sufficient to allow release of progesterone from the device to the animal.

The device may be inserted and retained in the vaginal cavity of the animal.

The device may be withdrawn from the vagina by a pull withdrawal system such as a cord attached to the end of the device.

The device may release progesterone in the vagina for a period of 2-20 days.

The device may have a residual load after 7 days of less than 65% by weight of its progesterone load at insertion.

Also disclosed herein is a method of manufacturing a device disclosed herein, comprising impregnating a drug into a polymer matrix comprising a block copolymer of butadiene or isoprene monomers and one or more ethylene and/or styrene monomers to form a drug impregnated polymer matrix, and moulding the impregnated polymer matrix to form a drug release component for a device disclosed herein.

The one or more drugs may be blended with the one or more TPE-S block copolymers to form the drug impregnated polymer matrix. The one or more drugs may be blended into a polymer matrix comprising one or more TPE-S block copolymers and one or more polyolefins to form the drug impregnated polymer matrix. When in molten form, the drug impregnated matrix can be moulded onto a support to form the drug release device. The molten form of the drug release component may be referred to herein as the molten drug impregnated polymer matrix or mixture.

In one embodiment, the drug release component is a block copolymer of butadiene monomers and one or more ethylene monomers and the drug is progesterone.

The impregnated polymer matrix may be formed by injecting an uncured mixture of reactive monomers and drug into a mould, then polymerising these monomers with an appropriate photo, thermal or chemical initiation process to form a solid plastic. The mould may contain a spine.

The impregnated polymer drug delivery component may be formed by mixing progesterone with molten thermoplastic elastomer, injecting this molten mixture into a mould, then allowing sufficient time for cooling of this mixture and removal of the implant from the mould. The mould may contain a spine.

The impregnated polymer drug delivery component may be formed by mixing progesterone with thermoplastic elastomer resin, feeding this mixture into the barrel of an injection moulding machine and moulding devices using a method known to those skilled in the art of injection moulding. In one embodiment the mould contains a spine.

Also described herein is a method of manufacturing a device disclosed herein comprising providing tablets or pellets of the one or more drugs together with one or more additives; mixing the tablets or pellets with granules of a polymer matrix, comprising the one or more TPE polymers disclosed herein, to form a drug impregnated polymer matrix wherein the one or more drugs is dispersed in the polymer matrix; melting the drug impregnated polymer matrix by loading into a hopper of an injection moulding machine to form a molten drug impregnated polymer matrix; injecting the molten drug impregnated polymer matrix into a mould cavity containing the support and cooling to solidify the drug release device described herein.

The one or more drugs may be dispersed evenly in the polymer matrix.

A drug masterbatch of the drug and excipients may be prepared, the drug masterbatch may be compressed into tablets or pellets of 2-50 mg; drug masterbatch pellets may be mixed with granules of mediprene 500452M such that one material is evenly dispersed within the other to form a solid mixture; the solid mixture may be loaded into a hopper of an injection moulding machine; the drug and polymer become molten in the heated barrel of the injection moulding machine the polymer drug mixture may be injected into a mould cavity and solidifies on cooling to form the drug release component.

The impregnated polymer drug delivery component may be formed by mixing progesterone with thermoplastic elastomer resin, feeding this mixture and a separate mixture of polymer resin suitable for the support material into the two separate barrels of an injection moulding machine capable of two shot sequential injection moulding. Devices are moulded using a method known to those skilled in the art of injection moulding by first injecting the polymer for a support material then altering the mould and injecting the thermoplastic elastomer and progesterone resin. In one embodiment the mould contains a spine.

In the method disclosed herein, the molten drug release component on contact with the support during the moulding process may partially melt the support the molten drug release component on contact with the support during the moulding process may melt more than about 1nm of the support.

Described herein is the disclosed device disclosed herein for use in oestrus synchronisation of a herd.

Described herein is the disclosed a device disclosed herein when used in herd oestrus synchrony.

The device can be impregnated with progesterone and upon insertion of the device into the vaginal cavity of each heifer and/or cow in a herd allows for synchronised insemination of heifers and/or cows as well as predictable and uniform calving times within the herd.

**EXAMPLES**

A list of abbreviations used herein are shown in Table 1.

*Table 1 - List of abbreviations*

| Name | Abbreviation |
|---|---|
| AI | Artificial Insemination |
| CIDR® | The innovator product (Controlled Internal Drug Release Device) available from Zoetis Inc |
| EVA | poly (ethylene-co-vinyl acetate) |
| $EVA_{x\%}$ | poly (ethylene-co-vinyl acetate) containing x% w/w vinyl acetate content |
| IVD | Intra-Vaginal Device |
| P4 | Progesterone |
| SBS | poly (Styrene-Butadiene-Styrene) block copolymer |

(continued)

| Name | Abbreviation |
|------|-------------|
| SVF | Simulation Vaginal Fluid |
| TPE | Thermoplastic Elastomer |
| USP | United States Pharmacopeia |
| VA | Vinyl Acetate |

Mediprene® contains the A-B-A triblock copolymer polystyrene-block-poly(ethylene-co-butadiene)-block-polystyrene (SEBS). Blends such as Mediprene®500452M further include polypropylene and paraffin oil. Mediprene® (Elasto)and Thermolast® formulations (Kraiburg) having a hardness in the range of 30-50 Shore (A type Durometer) were used in the following Examples.

The components of Mediprene®500452M (45 Shore A), as provided by by Elasto (Hexpol), are shown below in Table 2.

*Table 2: Components of Mediprene® 500452M*

| Proprietary Name | # | Component Redulated Name | Common Name |
|------------------|---|--------------------------|-------------|
| Mediprene 500452M | 1 | Polypropylene | PP |
| | 2 | Liquid paraffin | Medicinal white oil, Mineral oil |
| | 3 | Polystyrene-*block*-poly(ethylene-*co*-butadiene) - *block*-polystyrene rubber | SEBS, SEBS rubber, Styrene butadiene rubber, styrenic block copolymer |

## Detailed description of Figures

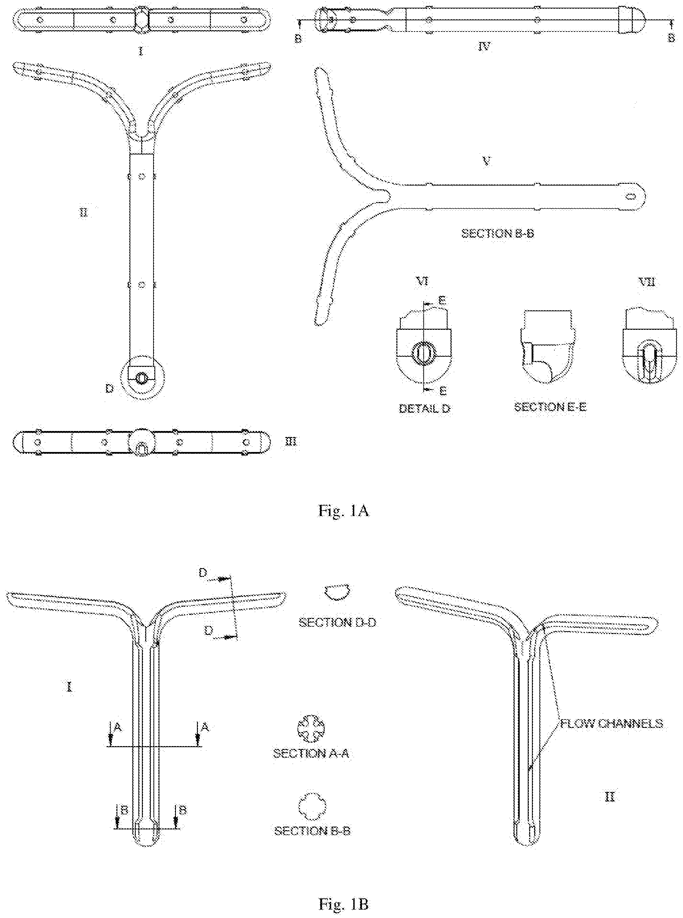

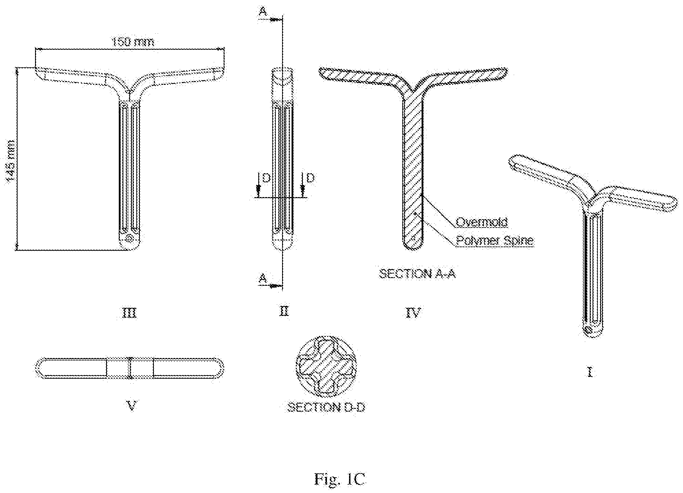

Fig. 1A-1C shows the spine of a device according to an embodiment of the present disclosure according to the first aspect prior to the TPE being over-moulded on to the spine. The spine in Fig 1A may be described as substantially "Y-shaped" while the spine/device in Fig 1B and IC may be described as substantially "T-shaped" In Fig 1B and 1C, the angle between the elongate body and the arms is about 95°. The spine consists of an elongate body and two arms that extend from one end of the elongate body. The spine can be made of low density polyethylene (LDPE) of a grade that allows manual extension of the wing component and elastic flexure of the arms to their original position at temperatures between 0 and 40°C. The spine of the device disclosed herein can be made from other suitable plastics as disclosed herein including one or more of polypropylene (PP) and polyvinyl chloride (PVC).

The device arms can be designed for optimal flexure and retraction during insertion and removal in the vagina.

Figure 1A, shows the spine in schematic front view (V) and cross-sectional front view (II). The full length of the spine, in its relaxed or unrestrained form, as shown in side view (IV) is approximately 18.31 cm and the span of the arms from one outer end to the other outer end, as shown in view (I), is approximately 14.26 cm. The length of the elongate body from the junction of the two arms to the end of the elongate body is about 14.17 cm. One end of the elongate body, opposite to where the arms are attached, has an orifice to which a retrieval means can be attached. The section D showing the orifice is enlarged with front (VI) and back (VII) views and cross section E-E of view (VI). The spine has nodules spaced along the length of the body and arms. In an area of the elongate body where there are no nodules, the width of the elongate body is about 1.3cm. In an area of the elongate body where there are nodules, the width is about 1.5cm. The diameter of each nodule is about 0.3cm. These nodules can be used to position the spine, for example hold the device centrally in the cavity of the mould during the over moulding process. The nodules may also assist in providing improved adhesion of the TPE to the spine.

The surface area of this device is approximately 126 cm$^2$.

In another embodiment, the length of the device is about 14.7cm, the length of the elongate body is about 12cm and the length of the arms from one end to another is about 15cm. The surface area of this device is approximately 124 cm$^2$.

**Fig. 1B** shows the spine of a device according to one embodiment disclosed herein. Flow channels, which may assist in the over moulding process, are also shown in the drawings. A front view (I), perspective view (II) and cross sectional view of D-D is shown. The full length of the device spine, in its relaxed form, in **Fig. 1B** is approximately 14.5 cm and the span of the arms from one outer end to the other outer end is approximately 15 cm. The spine may have indentations spaced along the length of the body and across the arms as shown in **Fig 1B.** The flow channels are in the form of indentations. The cross section D-D of the arm shows a substantially semi-circular shape.

**Fig. 1C** shows a slight variation of the spine of **Fig. 1B** over-moulded with the drug release component. The spine of **Fig. 1C** differs from the spine of Figure 1B in that at one end of the elongate body, opposite to where the arms are attached, there is an orifice to which a retrieval means can be attached.

**Fig. 1C** shows a view of the spine being overmoulded (I), a cross-sectional side view of the overmoulded device (II), a cross-sectional front view of the overmoulded device (III) and a cross-sectional bottom view of the overmoulded device (V). A schematic front view (IV) shows the device as viewed in its overmoulded form. Cross section D-D of the elongate body shows the spine with indentations overmoulded with the TPE containing drug release component. In these embodiments, the drug release component comprises blends of TPE-S and polypropylene in the form of commercially available Mediprene® or Thermolast® products, impregnated with progesterone, as described in Examples 1-4 and 11. The drug release component is adhered to the spine by virtue of the melt point of the LDPE and the drug release component through the over-moulding process.

Through changes in geometry of the spine the surface area of the devices of **Fig. 1B** and **1C** can be maintained even with significant changes to the length of the elongate body compared to the device of **Fig. 1A.** The surface area of the device of **Fig. 1B** and **1C** is approximately 124 cm$^2$

One end of the elongate body, opposite to where the arms are attached, has an orifice to which a retrieval means can be attached, as shown in **Fig. 1A** and **1C.**

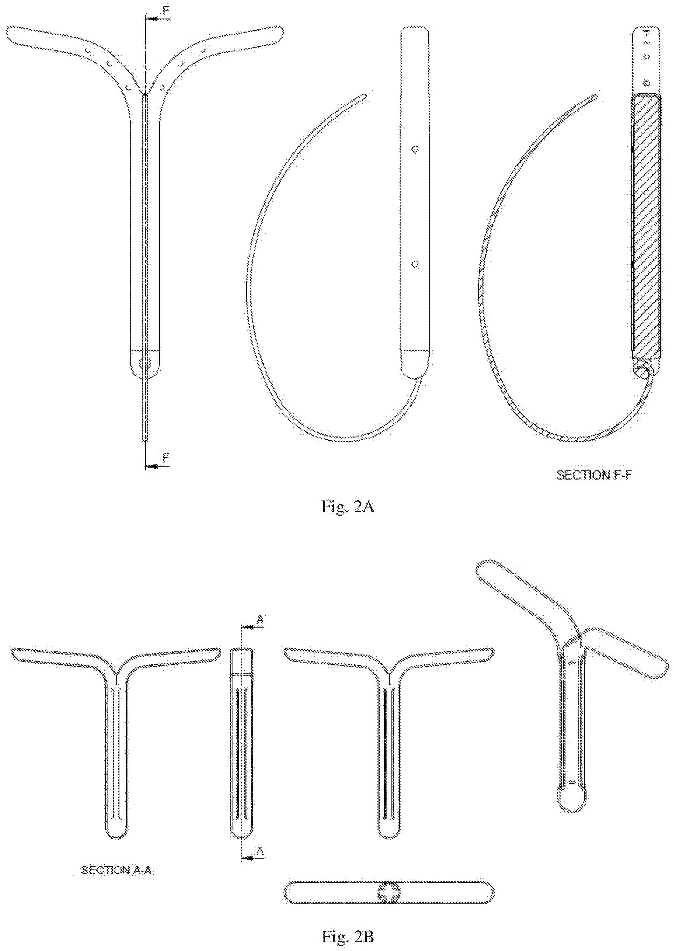

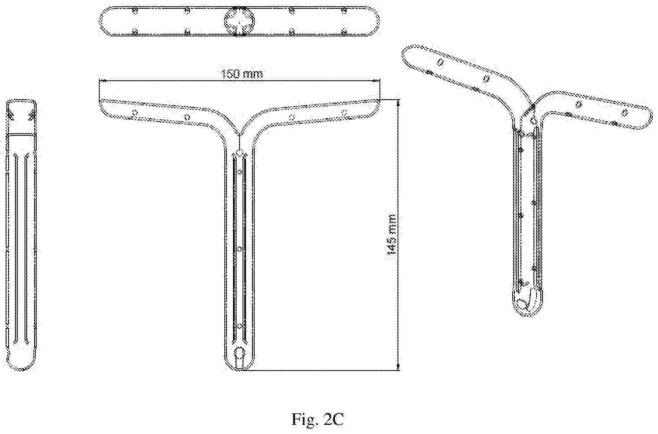

**Fig. 2A** shows the spine of **Fig. 1A** with a thermoplastic elastomer overlay as disclosed herein. The overall appearance of the device is smooth and rounded. The drug release component is adhered to the spine by virtue of the melt point of the LDPE and the drug release component through the over-moulding process. Similarly, **Fig. 2B** shows the spine of **Fig. 1B,** and **Fig. 2C** shows the spine of **Fig. 1C** with a thermoplastic elastomer overlay as disclosed herein. Specifically, according to one embodiment, the device in **Fig. 2A** shows the LDPE spines of **Fig. 1A, 1B** and **1C** over-moulded with the drug release component as disclosed herein comprising a blend of TPE-S, polypropylene, oil and progesterone. Specific embodiments are disclosed in Examples 1-4 and 11-12.

The spine as disclosed herein and as shown in **Fig. 1A-C** can be coated with the drug release component comprising TPE-S, polypropylene and oil using known techniques of over-moulding. The preferred method of coating is by pre-alignment of the spine in the cavity of a two part mould of the required size and injection of the molten drug release component into the space surrounding the centrally position spine. The preferred temperature for this process is 180-210°C. The heat of the molten drug delivery component, will melt the LDPE spine to the required degree to achieve adhesion between the two layers. The device is allowed to cool so that both plastics are solidified prior to removal of the coated device after separation of the mould halves. Example 12 below describes the process of injection moulding to manufacture the spine disclosed herein and the over-moulding process to coat the spine.

**Examples**

In-vitro data has been obtained comparing progesterone release of the device according to the present disclosure to a commercially available device (CIDR®). The device according to the present disclosure (specifically according to Example 12) releases the progesterone more slowly than the CIDR® product *in vitro.* The CIDR product exhibits dump release properties while the device disclosed herein according to Example 12 exhibits slow release properties.

Additionally, the *in vivo* performance of a device disclosed herein was compared to the performance of the CIDR® device. The release rates of progesterone from the device according to the present disclosure *in vivo* was found to be comparable to that of the CIDR device.

In Examples 1-11, progesterone impregnated TPE-S coatings were prepared and were set as sheets (approx. 1-2mm). In Example 12 the drug release component (comprising Mediprene and progesterone) was applied to an LDPE spine according to the shape of Fig. 1A-B with the dimensions as follows: length of spine is about 14.7cm, length of the elongate body about 12cm, span of arms from one to another about 15cm; surface area approximately 124 cm$^2$.

The Examples include commercially available polymers (EVA and Silicone) and Mediprene® or Thermolast®.

**Example 1**

Table 3: Components of example 1.

| Component | Quantity (g) | Supplier | Function |
|---|---|---|---|
| Progesterone | 2.0 g | Sigma Aldrich | Active |
| Mediprene® 500452M, (45 Shore A) | 20 g | Elasto (Hexpol) | Drug delivery |

Approximately two test drug release polymer sheets can be prepared following the steps outlined below:

1. Heat Mediprene® until molten, approx. 190 °C

2. Add Progesterone to step 1 and blend molten mixture for 2 minutes

3. Manually filling 1, 1.5 or 2 mm deep rectangular moulds to form polymer sheets

## Example 2

Variations on the formulation in Example 1 were prepared using 1.6 g of progesterone instead of 2.0 g.

*Table 4: Components of example 2.*

| Component | Quantity (g) | Supplier | Function |
|---|---|---|---|
| Progesterone | 1.6 g | Sigma Aldrich | Active |
| Mediprene® 500452M, (45 Shore A) | 20 g | Elastotec | Drug delivery |

Approximately two test drug release polymer sheets can be prepared following the steps outlined below:

1. Heat Mediprene® until molten, approx. 190 °C

2. Add Progesterone to step 1 and blend molten mixture for 2 minutes

3. Mould as for example 1.

## Example 3

Variations on the formulation in example 1 were prepared using an alternate thermoplastic elastomer to Mediprene®, being Thermolast®M 35 shore A.

*Table 5: Components of example 3.*

| Component | Quantity (g) | Supplier | Function |
|---|---|---|---|
| Progesterone | 2.0 g | Sigma Aldrich | Active |
| Thermolast ®M 35 shore A | 20 g | Kraiburg | Drug delivery |

Approximately two test drug release polymer sheets can be prepared following the steps outlined below:

1. Heat Thermolast® at 190 °C until molten

2. Add Progesterone to step 1 and blend molten mixture until well mixed for 1-5 min.

3. Mould as for example 1.

## Example 4

Variations on the formulation in example 1 were prepared using alternate thermoplastic elastomer to Mediprene®.

*Table 6: Components of example 4.*

| Component | Quantity (g) | Supplier | Function |
|---|---|---|---|
| Progesterone | 2.0 g | Sigma Aldrich | Active |
| Thermolast® M 45 shore A | 20 g | Kraiburg | Drug delivery |

Approximately two test drug release polymer sheets can be prepared following the steps outlined below:

1. Heat Thermolast® at 190 °C until molten

2. Add Progesterone to step 1 and blend molten mixture for 2 minutes

3. Mould as for example 1.

**Example 5 (Comparative)**

Variations on the formulation in example 1 were prepared using ethyl vinyl acetate with a 40% w/w vinyl acetate co-monomer content ($EVA_{40\%}$) instead of Mediprene®.

*Table 7: Components of example 5.*

| Component | Quantity (g) | Supplier | Function |
|---|---|---|---|
| Progesterone | 2.0 g | Sigma Aldrich | Active |
| $EVA_{40\%}$ | 20 g | Sigma Aldrich | Drug delivery |

Approximately two test drug release polymer sheets can be prepared following the steps outlined below:

1. Heat EV $A_{40\%}$ at 190 °C until molten

2. Add Progesterone to step 1 and blend molten mixture for 2 minutes

3. Mould as for example 1.

**Example 6 (Comparative)**

Variations on the formulation in example 4 were prepared using ethyl vinyl acetate with a 25% w/w vinyl acetate co-monomer ($EVA_{25\%}$) content instead of Mediprene®500452M, (45 Shore A).

*Table 8: Components of example 6*

| Component | Quantity (g) | Supplier | Function |
|---|---|---|---|
| Progesterone | 2.0 g | Sigma Aldrich | Active |
| $EVA_{25\%}$ | 20 g | Sigma Aldrich | Drug delivery |

Approximately two test drug release polymer sheets can be prepared following the steps outlined below:

1. Heat $EVA_{25\%}$ at 190 °C until molten

2. Add Progesterone to step 1 and blend molten mixture for 2 minutes

3. Mould as for example 1.

**Example 7 (Comparative)**

Variations on the formulation in example 4 were prepared using ethyl vinyl acetate with a 12% w/w vinyl acetate co-monomer content ($EVA_{12\%}$) instead of Mediprene®

*Table 9: Components of example 7*

| Component | Quantity (g) | Supplier | Function |
|---|---|---|---|
| Progesterone | 1.6 g | Sigma Aldrich | Active |
| $EVA_{12\%}$ | 20 g | Sigma Aldrich | Drug delivery |

Approximately two test drug release polymer sheets can be prepared following the steps outlined below:

1. Heat $EVA_{12\%}$ at 190 °C until molten

2. Add Progesterone to step 1 and blend molten mixture for 2 minutes

3. Mould as for example 1.

**Example 8 (Comparative)**

Variations on the formulation in example 4 were prepared using 10 g of EV $A_{40\%}$ and 1.5 g of progesterone.

*Table 10: Components of example 8*

| Component | Quantity (g) | Supplier | Function |
|---|---|---|---|
| Progesterone | 1.5 g | Sigma Aldrich | Active |
| $EVA_{40\%}$ | 10 g | Sigma Aldrich | Drug delivery |

Approximately one test drug release polymer sheets can be prepared following the steps outlined below:

1. Heat EV $A_{40\%}$ at 190 °C until molten

2. Add Progesterone to step 1 and blend molten mixture for 2 minutes

3. Mould as for example 1.

**Example 9 (Comparative)**

Variations on the formulation in example 4 were prepared using 10 g $EVA_{40\%}$ and 0.5 g of progesterone

*Table 11: Components of example 9*

| Component | Quantity (g) | Supplier | Function |
|---|---|---|---|
| Progesterone | 0.5 g | Sigma Aldrich | Active |
| $EVA_{40\%}$ | 10 g | Sigma Aldrich | Drug delivery |

Approximately one test drug release polymer sheets can be prepared following the steps outlined below:

1. Heat EV $A_{40\%}$ at 190 °C until molten

2. Add Progesterone to step 1 and blend molten mixture for 2 minutes

3. Mould as for example 1.

**Example 10 (Comparative)**

Variations on the formulation in example 1 were prepared using 20 g of one part acetoxy room temperature vulcanisation (RTV) silicone and 3 g of progesterone at room temperature.

*Table 12: Components of example 10*

| Component | Quantity (g) | Supplier | Function |
|---|---|---|---|
| Progesterone | 3 g | Sigma Aldrich | Active |
| One part acetoxy RTV Silicone | 20 g | Viking | Drug delivery |

Approximately one test drug release polymer sheets can be prepared following the steps outlined below:

1. Combine silicone and progesterone

2. Mould as for example 1.

3. Allow to cure in a well-ventilated area overnight

**Example 11**

*Table 13: Components of example 11*

| Component | Quantity (g) | Supplier | Function |
|---|---|---|---|
| Progesterone | 1.0 g | Sigma Aldrich | Active |
| Thermolast K® (35 Shore A) | 10 g | Elastotec | Drug delivery |
| Sorbitol | 0.1 | Sigma Aldrich | |

Approximately two test drug release polymer sheets can be prepared following the steps outlined below:

1. Heat Thermolast K® until molten, approx. 190 °C

2. Add Progesterone and sorbitol to step 1 and blend molten mixture for 2 minutes

3. Mould as for example 1.

**<u>Manufacturing method</u>**

**Example 12**

*Production of the Low Density Polyethylene (LDPE) spine*

Production of the spine using suitable plastics as herein disclosed by injection moulding can be achieved using techniques known to those skilled in the art (for example as described in Douglas M. Bryce, 1996, Plastic Injection Moulding: Manufacturing Process Fundamentals volume 1, Society of Manufacturing Engineers.
To produce a LDPE spine, medical grade beads of LDPE resin are dispensed into the hopper of the injection unit of an injection moulding machine with a barrel temperature greater than the melting point of the selected resin, and then injected into a mould or dye of the required shape. After cooling the solidified spine is released.

*Coating of the spine with drug release material*

*Table 14: Components of example 12*

| Component | Quantity (g) | Supplier | Function |
|---|---|---|---|
| Progesterone | 1.2 Kg | Sigma Aldrich | Active |
| Mediprene® | 12 Kg | Elastotec | Drug delivery |

Coating of the spine by over-moulding can be achieved using techniques known to those skilled in the art (for example as described in Douglas M. Bryce, 1996, Plastic Injection Moulding: Manufacturing Process Fundamentals volume 1, Society of Manufacturing Engineers).
Those familiar with the art are aware that a masterbatch of a drug such as progesterone can be prepared to enhance the distribution of drug throughout the final product. This can be manufactured in an analogous manner to preparation of materbatches of coloured resin. According to the present disclosure, masterbatches of varying concentrations of progesterone may be prepared and combined with the drug release component as disclosed herein comprising blends of one or more of TPE polymers, polyolefins and oil (eg Mediprene®) and used in the manufacturing process. Other excipient such as, , other polymers, waxes or organic binding agents may also be used.

**Method:**

Approximately 1.25 kg of a 95% progesterone masterbatch in pellet form is produced. Povidone and antistatic agents may be added in an amount of 0.1-5 %w/w to aid pellet formation.
Devices can be coated following the steps outlined below:

1. The 1.25 kg of the progesterone masterbatch is combined with 6.75 kg of Mediprene®500452M granules.

2.Previously manufactured device support inserts or spines as described in accordance with the present disclosure (see Example 12) are loaded into the central cavity of a two part dye or mould in a manner typical for insert moulding processes.

3.The Mediprene® and progesterone mixture is fed into the hopper of an injection moulding machine with a barrel temperature of at least 190°C, preferably 190-240 °C.

4.The material is mixed suitably to enable adequate uniformity.

5. 10-20 g of molten mixture is charged into the injector.

6. Molten material is injected into a two part dye pre-loaded with a spine.

7. The device is allowed to cool in the mould for 5-60 seconds

8.The dye is separated and the moulded device is ejected.

9.Overage waste is removed from injection ports known as gates to those familiar with the process.

Advantageously, the overage waste can be reprocessed in the making of the another device.
It will be appreciated that the devices disclosed herein may be manufactured in other ways. For example, with reference to step 2, it is anticipated that the manufacturing process may involve moulding the device support insert or spine immediately prior to adding the drug delivery layer in a two shot injection moulding machine.

**Dissolution Experiments**

To enable rapid comparison between formulations, Accelerated dissolution (AD) media was prepared by mixing 1 part ethanol and 2 parts water. The solution was degassed by filtration through a 0.45 $\mu$M Nylon filter.
Simulated Vaginal Fluid (SVF) media was prepared using the reagents and quantities below that were based on the formulation published by H.O. Owen and D. F. Katz, Contraception 59, 91, 1999. The reagents specified in the table below were added to 1 L of water for injection. The pH of the solution was adjusted to 7.4, the pH typical for the vagina of healthy cows, using 5% NaOH$_{(aq)}$ the solution was degassed by filtration through a 0.45 $\mu$M Nylon filter.

Table 15: Components of SVF

| Component | Quantity added in g/L |
|---|---|
| NaCl | 3.5 |
| KOH | 1.4 |
| CaOH | 0.2 |
| Lactic acid | 2.0 |
| acetic acid | 1.0 |
| glycerol | 0.2 |
| urea | 0.4 |

(continued)

| Component | Quantity added in g/L |
|-----------|-----------------------|
| glucose | 5.0 |

The dissolution tests methods used are described below.

**Dissolution procedure 1**

A comparison of P4 release from polymer samples were monitored in a Sotax dissolution apparatus equipped with an Agilent 8453 UV/vis and autosampler. Each of the seven wells was charged with accelerated dissolution media (ADM) (500 mL, deggassed by filtration through a 0.45 $\mu$M Nylon filter). The system was allowed to equilibrate to 39.5°C. Up to six test polymers were placed as simultaneously as practical, one per basket in the Sotax dissolution apparatus type 1 configuration. The seventh well was left as a blank. The commencement time was noted and the autosampling program on the dissolution apparatus was commenced. Cells were stirred at 50 rpm and heated at 39.5 °C for the duration of the experiment. Readings of the absorbance at 248 nm were taken on the solution at least every 5 minutes for the first hour and every hour for the time therafter. The dissolution baths were monitored for at least 24 hours.

**Dissolution procedure 2**

A comparison of P4 release from polymers samples were monitored in a Sotax dissolution apparatus equipped with an Agilent 8453 UV/vis and autosampler as described in procedure 1 above but with the following modifications; each of the seven wells was charged with SVF (500 mL, deggassed by filtration through a 0.45 $\mu$M Nylon filter). The seventh well was left as a blank and cells were stirred at 50 rpm. Readings of the absorbance at 248 nm were taken on the solution at least every 5 minutes for the first hour and every hour for the time therafter. The dissolution baths were monitored for at least 7 days.

**Dissolution procedure 3**

A comparison of P4 release from polymers samples were monitored in a Sotax dissolution apparatus equipped with an Agilent 8453 UV/vis and autosampler as described in procedure 1 above but with the following modifications; each of the seven wells was charged with SVF (1000 mL, deggassed by filtration through a 0.45 $\mu$M Nylon filter). Test devices were placed one per well with no basket. The seventh well was left as a blank. No cells were stirred. Readings of the absorbance at 248 nm were taken on the solution at least every 5 minutes for the first hour and every hour for the time therafter. The dissolution baths were monitored for at least 7 days.

***In vitro* experiment**

*In vitro* experiments were run to compare the test formulations to a commercially available and currently registered controlled internal drug release (CIDR®) device labelled for use in cattle. Initially experiments were performed over one day in AD media to generate rapid comparisons. Lead formulations were then compared to the CIDR® over one week in SVF.

**Results**

**Experiment 1:**

The dissolution of progesterone from samples of 6 and 8% P4 in EVA formulations (variations on example 9 with 0.6 and 0.8 g of progesterone respectively) with a cross sectional area of 0.5 cm$^2$ was compared to the dissolution of P4 from the outer surface of a 1 cm$^2$ section of a representative progesterone release device, the CIDR®.

Preparation of CIDR® sections

Two 1 cm$^2$ cross sections were cut from the wing top section of the CIDR® to obtain 1.5 mm thick samples of progesterone impregnated silicon. As the inner surface of the CIDR® section would not usually be exposed to the vaginal environment, for comparitive purposes, it was adhered to aluminium foil using water proof silicone sealant to disable P4 from dissoluting from this surface. Sample information is summarised in table 16 below.

Preparation of test polymer sections

Samples with a cross sectional area of 0.5 cm$^2$ were cut from 1 mm thick sheets of progesterone in EVA sheets. Both sides were left exposed to the dissolution solvent giving an effective surface area of 1 cm$^2$. Sample information is summarised in the table below.

Dissolution procedure 1 was used to measure the rate of P4 dissolution.

*Table 16: sample information for Experiment 1*

| Test sample | n | CSA cm$^2$ of item | Item SA cm$^2$ exposed to AD media | Sheet Thickness cm | Volume of polymer cm | Notes |
|---|---|---|---|---|---|---|
| CIDR® section | 2 | 1 | 1 | 0.15 | 0.15 | One side covered |
| EVA$_{40\%}$ sheet 6% P4 | 2 | 0.5 | 1 | 0.1 | 0.05 | Both sides exposed |
| EVA$_{40\%}$ sheet 8% P4 | 2 | 0.5 | 1 | 0.1 | 0.05 | Both sides exposed |
| n=number of samples; CSA= cross sectional area; SA = surfac area; AD=accelerated dissolution | | | | | | |

Dissolution procedure 1 was used to measure the rate of P4 dissolution.

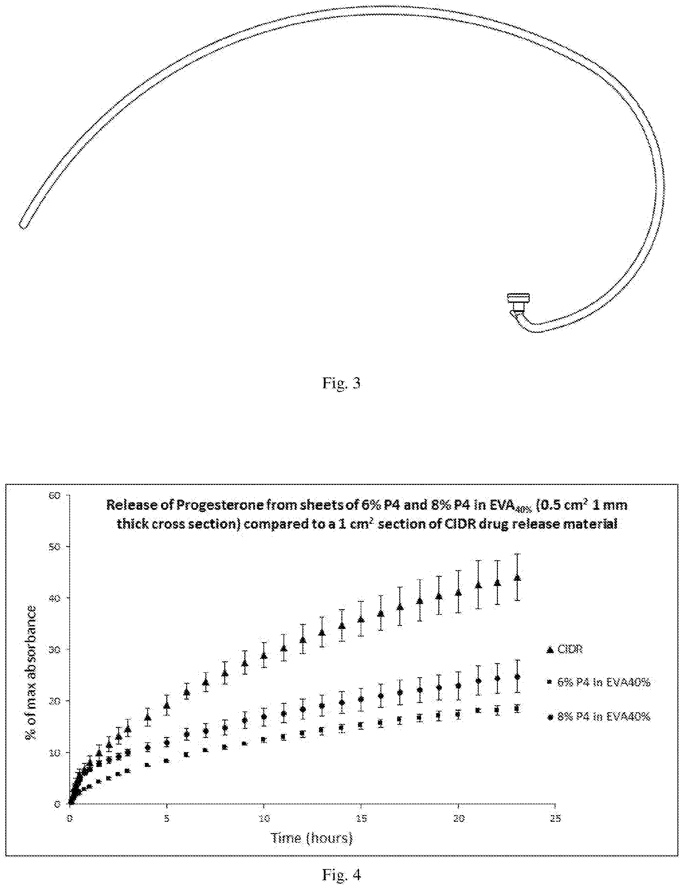

The dissolution rate of P4 from each section of polymer into 500 mL of ADM is shown in Fig.4. While both 6% and 8% levels of P4 in EVA sheets gave a lower overall release of P4 into the ADM over 24 hours, the rate of P4 release from the 8% P4 in EVA sheets was nearly identical to the P4 release of the CIDR® in the first one hour of this experiment. The rate and amount of progesterone released into the dissolution medium was higher for the 8% P4 sheets than the 4% P4 sheets. Error bars represent 95% confidence intervals (mean $\pm$ (2 $\times$ standard deviation/√n).

**Experiment 2:**

The dissolution of progesterone from both surfaces of 0.5 cm$^2$ samples of 10% P4 in EVA samples (variation on Example 9 using 1.0 g of progesterone) was compared to the dissolution of P4 from the outer surface of a 1 cm$^2$ section of the CIDR® device.

CIDR® and EVA sheet samples were prepared in an analogous manner to the preparation of samples described above and the sample size parameters are descibed in the table below. Dissolution procedure 1 was used to assess P4 dissolution rates.

*Table 17: Sample information for Experiment 2.*

| Test sample | n | CSA cm$^2$ of item | Item SA cm$^2$ exposed to AD media | Sheet Thickness cm | Volume of polymer cm$^3$ | Notes |
|---|---|---|---|---|---|---|
| CIDR® section | 3 | 1 | 1 | 0.15 | 0.15 | One side covered |
| EVA$_{40\%}$ sheet | 3 | 0.5 | 1 | 0.1 | 0.05 | Both sides exposed |

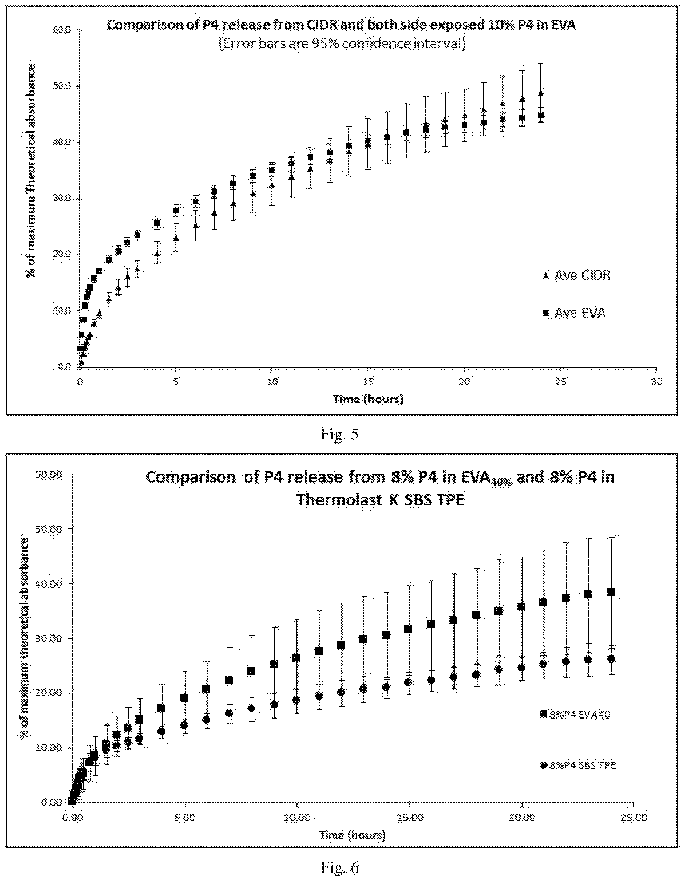

The results in Fig.5. show that after 24 hours, the rate of P4 released from the both the CIDR® and EVA sheet sections are highly similar. One small difference is in the initial P4 release rate, which is more rapid for the double sided 10% P4 in EVA samples than for the CIDR® samples. Error bars represent 95% confidence intervals (mean $\pm$ 2 $\times$ (standard deviation/√n).

**Experiment 3:**

The dissolution of P4 from 0.5 cm$^2$ samples of 8% P4 in EVA$_{40\%}$ sheets (example 9) and 8% P4 in Thermolast K® sheets (variation on example 3 using 1.6 g of progesterone) was compared. Samples of both test formulations were prepared in an anologous manner to the EVA sheet samples in example 1. Sample details are summarised in the table below. Dissolution procedure 1 was used to assess P4 dissolution rates.

Table 18: Sample information for Experiment 3.

| Test sample | n | CSA cm$^2$ of item | Item SA cm$^2$ exposed to AD media | Sheet Thickness cm | Volume of polymer cm$^3$ | Notes |
|---|---|---|---|---|---|---|
| 8% P4 Thermolast K® (35 Shore A) | 2 | 0.5 | 1 | 0.1 | 0.05 | Both sides exposed |
| 8% P4 EVA$_{40\%}$ sheet | 2 | 0.5 | 1 | 0.1 | 0.05 | Both sides exposed |

The results in **Fig.6.** show a large variablity in P4 dissolution between 8% P4 in EVA samples, but that these samples showed a greater P4 dissolution than 8% P4 in Thermolast K® for all time points after one hour.

**Experiment 4:**

The effects of adding a mild solubilising agent to a P4 in SEBS polymer preparation was assessed. Sheets of 12% P4 in Mediprene (variation on example 1) and 12% P4 in Mediprene with PEG (variation on example using 0.1 g of PEG and no Sorbitol) were prepared and cut into 0.5 cm$^2$ sections as described above. The samples detailed in the table below were compared using disolution procedure 2.

Table 19: Sample information for experiment 4.

| Test item | n | CSA cm$^2$ of item | Item SA cm$^2$ exposed to SVF media | Sheet Thickness cm$^2$ | Volume of polymer cm$^3$ | Notes |
|---|---|---|---|---|---|---|
| CIDR® | 2 | 1 | 1 | 0.2-0.5 | 0.3 | One side covered |
| 12% P4 in Mediprene®500452M (45 Shore A) with PEG | 2 | 0.5 | 1 | 0.2 | 0.1 | Both sides exposed |
| 12% P4 in Mediprene®500452M (45 Shore A) | 2 | 0.5 | 1 | 0.2 | 0.1 | Both sides exposed |

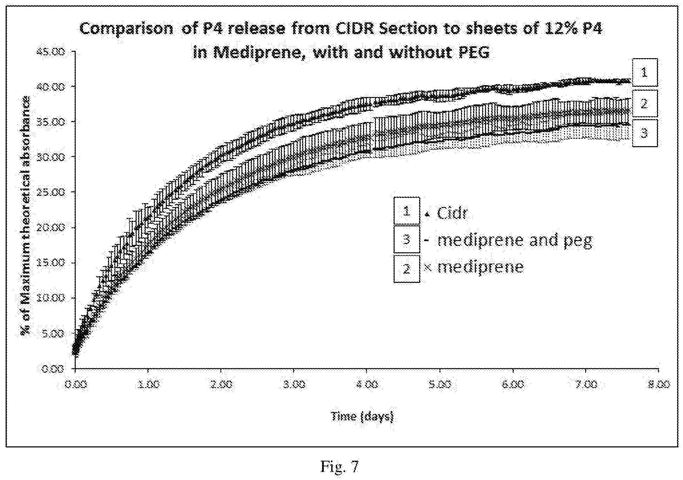

The results in **Fig.7** show that the inclusion of PEG had little effect on the rate of P4 dissolution into the aqueous dissolution media

**Experiment 5:**

A comparison was made between the P4 dissolution rate of one entire CIDR implant to the P4 dissolution from a 2 mm thick sheet of 10% P4 in Mediprene (prepared according to exampe 1) with a cross sectional area of 63 cm$^2$ and total surface area of 126 cm$^2$. This latter preparation is a suitable representation of the drug release device disclosed herein. Specifically, an appropriate drug delivery device according to one embodiment of the present disclosure comprises at least a 1 mm coating of a drug release component as disclosed herein comprising Mediprene and progesterone and an appropriate surface area (approx. 124 cm$^2$) to enable P4 diffusion from the outer exposed surface.
Disolution procedure 3 was used for this comparison.

*Table 20: Sample information for Experiment 5.*

| Test item | n | CSA cm$^2$ of item | Item SA cm$^2$ exposed to SVF media | Sheet Thickness cm$^2$ | mass of polymer | Notes |
|---|---|---|---|---|---|---|
| CIDR® | 3 | 120 | 120 | 0.2-0.5 | 19.5 | One side internal facing |
| 10% P4 in Mediprene® 500452M (45 Shore A) | 3 | 63 | 126 | 0.2 | 11.3 | Both sides exposed |

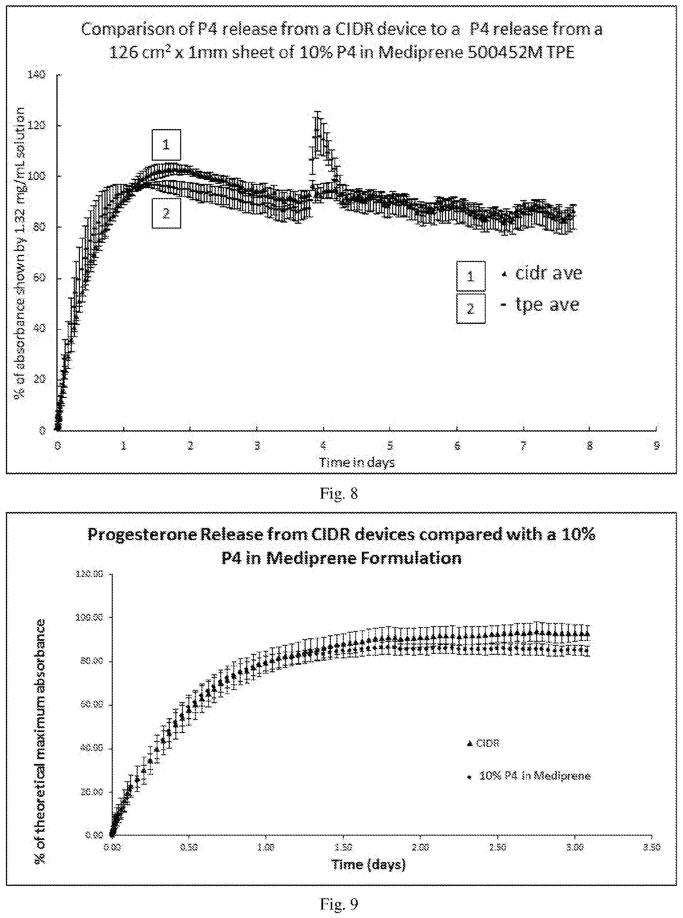

The results of the dissolution experiment monitored by UV/vis spectrophotometry are shown in Fig.8. The total amount of P4 released was very similar for both the CIDR® implant. The mediprene sheet of this surface area appears to have a slighly more rapid initial release rate but a slightly lower total release of P4. The rapid spike in absorbance of the TPE sampes between 4-5 days was likely due to solid material or air bubbles obstructing the autosampler during this time and is not attributable to this material.

**Experiment 7:**

Experiment 6 was repeated with monitoring of P4 into SVF media using three replicate HPLC chromatograms at each time point. The area under the curve of the progesterone peak enabled comparison of changes in progesterone concentration only. Samples were prepared as for experiment 6 and as detailed in table 21 below.

*Table 21: Sample information for Experiment 7.*

| Test item | n | CSA cm$^2$ of item | Item SA cm$^2$ exposed to SVF media | Sheet Thickness cm | mass of polymer | Notes |
|---|---|---|---|---|---|---|
| CIDR® | 3 | 120 | 120 | 0.2-0.5 | 19.5 | One side internal facing |
| 10% P4 in Mediprene® 500452M (45 Shore A) | 3 | 63 | 126 | 0.2 | 11.3 | Both sides exposed |

Fig. 9. shows the change in area under the absorbance peak for progesterone for the CIDR® and Mediprene for progressive dissolution time. Comparable P4 peak areas were obtained for both sample groups at all time points except for day 6. Using this method, variation between samples within both test groups was large (see error bars in Fig.9 representing 95% confidence intervals). Logarithmic curves were fitted to the data and showed very similar initial P4 release rates, the fitted trend lines could suggest a slightly lower final concentration of P4 dissolutes from the 10% P4 in Mediprene® test group, however there is not a significant difference in the final or penultimate concentrations measured.

***In vivo* pharmacokinetic assessment of example IVD device according to the present disclosure**

**Summary**

The PK performance of a drug release device as disclosed herein (which may be referred to hereinafter as IVD or Jurox IVD) was assessed in comparison to a commercially available product, the CIDR®.

**Procedures**

A two period cross over study was performed to compare the performance of the IVD (≈1540 mg dose progesterone, prepared as previously described herein) to a product currently registered for estrus synchronisation in cattle, the CIDR® (1340 mg dose progesterone). Six healthy, ovariectomised cows and six healthy, ovariectomised heifers were selected for the study and acclimated at the investigator site. Test and reference items were randomly assigned to Treatment Group A or B. Cows were randomly assigned a number from 1 to 6 and heifers were randomly assigned a number from 7 to 12. The cows and heifers were treated based on number assigned in the schedule in table 22 below.

*Table 22. The treatment groups assigned for cows (numbered 1 to 6) and heifers (numbered 7 to 12) for period 1 and 2 of the two group cross over study.*

| Cow or heifer number | Treatment Group Period 1 | Treatment Group Period 2 |
|---|---|---|
| 1, 2, 3, 7, 8, 9 | **A (CIDR)** | **B (IVD)** |
| 4, 5, 6, 10, 11, 12 | **B (IVD)** | **A (CIDR)** |

During this study, cows were assessed for changes in plasma progesterone concentration attributed to each treatment, the retention of devices for the study period and the quantity of residual progesterone in each device. The methods employed to assess these parameters are detailed below.

*Plasma progesterone analysis*

Blood samples were collected into labelled lithium heparin tubes at the following time points within 24 hours before IVD insertion and after dosing at 2, 4, 6, 8 hours (all $\pm 5$ minutes) and 24 hours ($\pm$ 15 minutes) and on Day 2, Day 3, Day 4.5, Day 6, Day 7 just prior to removal of the IVD (Day 7-Pre), Day 7 approximately 12 hour post IVD removal (Day 7-12 h), Day 8 and Day 9. Plasma was separated from the blood by centrifugation at 3000 rotations per minute (rpm) for 10 minutes using a Hettich 32R Centrifuge and 1613 rotor (relative centrifugal force approximately equivalent to $10^3 \times g$). Plasma was decanted to plain labelled polypropylene tubes within 30 minutes of collection.

Bovine plasma was analysed for progesterone concentration using an Ovucheck® plasma enzyme-linked immune-sorbent assay (ELISA) kit. The Immulite CLEIA method has been previously validated against a RIA assay kit and progesterone output values show 95% correlation with the RIA output values (see LeBlanc, S.J. and Broes, A., Can. Vet. J. 2014, 55, 582-584). Progesterone concentration analysis was performed in accordance with the kit instruction leaflet albeit with the followings exception; plasma progesterone concentration was calculated from the sigmoidal polynomial equation fitted to an Abs vs. concentration graph of standards of known progesterone concentration prepared in blank matrix. The standard solutions provided in the kit were not used. This kit was used as a method for rapid approximation of progesterone concentration in both test groups.

All cows and heifers were overectomised prior to the study however it should be noted that progesterone may be produced in other tissues. Additionally, interference from the steroids 11$\alpha$-hydroxyprogesterone (66%), 5-Pregnan-3$\beta$-ol-20-one (16%), 5$\beta$-Pregnan-3, 20-dione (4.5%), 5$\alpha$-Pregnan-3, 20-dione (3.3%) and Deoxy-corticosterone acetate (3%) is noted by the manufacturers in the ovucheck plasma kit insert. The manufacturer indicates that the assay range is between 1 and 10 ng/mL. Quality control (QC) samples at 1, 2, 5 and 10 ng/mL were prepared by spiking 90 $\mu$L of blank bovine plasma with 10 $\mu$L of standard solutions of appropriate concentration. These QC samples were analysed on each assay plate as an indication of the accuracy of results generated from regression of the calibration curve. The QC standards were prepared separately to calibration samples and the accuracy data for these samples in appendix 1.

*Device retention*

Presence or absence of the device was noted at each scheduled blood collection point after visual or manual examination.

*Residual progesterone in each device*

Each explanted device was cut into 7-10 pieces and the fragments were placed in a round bottom flask. Fragments were extracted with acetone at 40 °C, the extracts were combined, the flask was then made to the mark and a 20 uL aliquot of each combined extracts solution was diluted with 980 uL of acetonitrile. These solutions were analysed by HPLC and their concentration was determined by regression of a calibration curve prepared from standards of known progesterone concentration. Sigmoidal curves were fitted to calibration curves.

**Results**

***Device retention.***

All devices of all test groups were retained for the intended duration of treatment during both period 1 and 2 of this study.

*Period 1 of JX1302-K005 part 2*

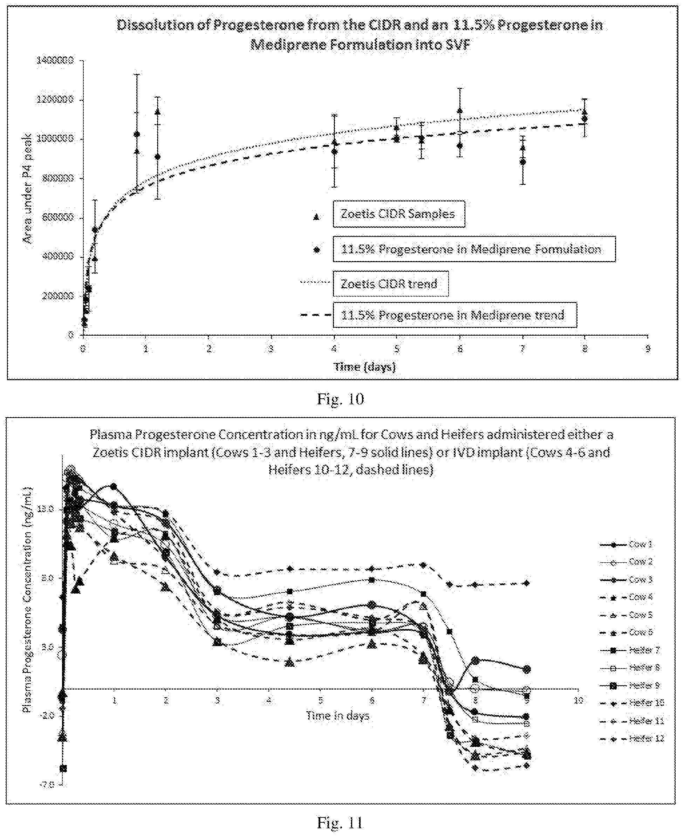

The calculated plasma progesterone concentration for all time points in period 1 are summarised in table 23 and plotted in **Fig. 11.**

The calculated concentration was reported as obtained even where calculated concentration is lower than the Lower Limit of Qualification (1 ng/mL according to kit instructions) or higher than the Upper Limit of Qualification (ULOQ, 10 ng/mL according to kit instructions). Calculated concentrations above 10 ng/mL were interpreted with caution as above the ULOQ. These samples were not diluted and reanalysed. The progesterone concentration calculated from regression of the calibration curve was negative for heifer 9 prior to treatment and for many sample points post removal. Negative values and calculated concentrations less than 1 ng/mL have been interpreted as being of a value less than the lower limit of quantification (LLOQ).

*Table 23: Plasma progesterone (ng/mL) content for each cow or heifer administered a CIDR® or Jurox IVD during study Part II period 1 of JX1302-K005. All data points are reported as obtained (ng/mL). Note: shaded cells indicated that a progesterone releasing device was present in the animal at this time point.*

**JX1302-K005 Part 2 Period 1, Analyst 1**

|  | Cow # | | | | | | Heifer # | | | | | |
|---|---|---|---|---|---|---|---|---|---|---|---|---|
| | Treatment A | | | | Treatment B | | Treatment A | | | | Treatment B | |
| time | 1 | 2 | 3 | 4 | 5 | 6 | 7 | 8 | 9 | 10 | 11 | 12 |
| 0.0 | 0.6 | 1.8 | 3.8 | 0.8 | 0.2 | 0.2 | 3.9 | 0.6 | -0.2 | 5.6 | 0.1 | 0.5 |
| 2 h | 12.8 | 12.9 | 12.5 | 12.2 | 12.4 | 12.3 | 12.8 | 12.9 | 12.6 | 12.7 | 12.7 | 12.8 |
| 4 h | 12.8 | 12.9 | 12.6 | 12.2 | 12.5 | 12.5 | 12.7 | 12.9 | 12.7 | 12.9 | 12.9 | 12.8 |
| 6 h | 12.7 | 12.8 | 12.6 | 6.1 | 12.6 | 12.6 | 12.9 | 12.9 | 12.5 | 12.8 | 12.9 | 12.8 |
| 8 h | 12.6 | 12.7 | 12.7 | 12.6 | 12.5 | 12.4 | 12.8 | 12.7 | 12.5 | 12.8 | 12.9 | 12.8 |
| 1 d | 13.6 | 12.1 | 12.9 | 11.4 | 10.0 | 10.3 | 12.9 | 11.3 | 11.7 | 12.9 | 12.7 | 12.6 |
| 2 d | 10.2 | 11.0 | 12.2 | 11.5 | 9.3 | 7.9 | 12.9 | 11.6 | 10.5 | 12.6 | 12.0 | 10.1 |
| 3 d | 11.9 | 11.9 | 13.8 | 11.6 | 11.1 | 9.1 | 13.7 | 10.9 | 9.1 | 14.9 | 12.3 | 12.1 |
| 4.5 d | 10.0 | 11.8 | 11.8 | 9.4 | 10.0 | 5.8 | 13.7 | 11.9 | 10.9 | 15.1 | 13.0 | 12.6 |
| 6 d | 10.3 | 10.4 | 12.8 | 10.7 | 10.3 | 8.8 | 14.5 | 10.2 | 11.2 | 15.0 | 11.5 | 11.7 |
| 7 d | 10.0 | 10.8 | 10.4 | 6.9 | 12.7 | 6.2 | 13.6 | 10.9 | 10.0 | 15.3 | 12.7 | 10.3 |
| 7.5 d | -0.5 | 1.2 | -0.2 | -2.6 | -4.2 | -4.2 | 10.3 | 0.3 | -4.9 | 14.2 | -2.3 | -4.2 |
| 8 d | -2.9 | 0.2 | 5.9 | -5.4 | -6.1 | -6.2 | 1.8 | -3.6 | -5.3 | 14.2 | -5.1 | -7.0 |
| 9 d | 0.1 | 0.2 | 0.4 | 0.0 | 0.0 | 0.0 | 0.1 | 0.0 | 0.0 | 9.7 | 0.0 | 0.0 |

Prior to administration of any devices, the endogenous progesterone concentration for most animals measured below 2 ng/mL. Plasma taken from Cow 3, heifer 7 and heifer 10 were calculated contain 3.8, 3.9 and 5.6 ng/mL of progesterone respectively. Within 2 hours of administration of devices, plasma progesterone concentrations increased to greater than 10 ng/mL for all animals. It should be noted that the precision of QC samples on plate 1 used to generate Day 0 data was reasonable for 10 and 1 ng/mL concentration samples, but poor for 5ng/mL and 2 ng/mL concentration samples (appendix 1. Table III). It could be reasonably assumed that calculated concentrations of nominal value higher than 10 ng/ml and less than 1 ng/mL are most likely indeed greater than 10ng/ mL and less than 1 ng/mL respecively;

however, variable concentrations greater than the nominal concentration were calculated for the 2 and 5 ng/mL QC samples.

Throughout day 1 and day 2, the measured plasma progesteone concentration for each cow continued to be calculated as higher than 10 ng/mL for the majority of test animals. Calculated values of 9.3 and 7.9 ng/mL were obtained on day 2 for Cows 5 and 6. Literature opinion suggests that the large increase of blood progesterone concentration caused by insertion of a progesterone releasing device may induces enzymatic pathways for progesterone down regulation causing a drop in progesterone concentration after 48 hours (see Rathbone, M. J. et al., J. Control. Release, 1994, 54, 117-148). The drop in plasma progesterone concentration in these cows may be attributable to this enzymatic down regulation, be from lesser progesterone release or may be simply an anomaly of the analytical method. Day 1 and 2 samples were analysed on plate 2 (QC accuracy in appendix 1, table VI). Accuracy for the 10 ng/mL QC samples were acceptable; however, the 5 and 2 ng/mL QC samples returned calculated values up to 200% higher than their nominal concentration. As a result of this, the calculated progesterone concentration for cow 5 and 6 on day 2 should be interpreted with caution. If the QC samples are an adequate prediction of sample accuracy, the actual sample concnetration at these points may actually be of a concentration as low as half their nominal value (ie. 4.6 and 3.9 ng/mL respectively). It is accepted that an estrus synchronisation device should mediate bovine plasma progesterone concentrations greater than 2 ng/mL for a 5-7 day treatment period with co-administration of other suitable reproductive hormones (i.e. GNRH, an oestradiol derivative and/or prostaglandin) for effective estrus synchrony (see Rathbone, M. J. et al., J. Control. Release, 1994, 54, 117-148). Given this information, these progesterone concentrations are not likely to be detrimental to efficacy of estrus synchronisation.

From day 3 until the initial day 7 blood collection, plasma progesterone concentration for the majority of animals continued to be calculated as greater than 10 ng/mL. The two exceptions to this is were cows 4 and 6, with cow 6 recording the lowest plasma progesterone concentration (5.8 ng/mL, day 4.5) for this treatment period. Despite this, generally plasma progesterone concentrations between test groups A and B are comparable to each other during the treatment period (to day 7) and fall rapidly to low concentrations after removal of the devices. It is also known to those skilled in the art that a rapid fall in plasma progesterone concentration is required on removal of the device for the commencement of pro-estrus and effective estrus synchrony. Unusually, the measured plasma progesterone concentration for heifer 10 remained high until the completion of schedualled plasma testing. This continuation of high plasma progesterone concentrations were not observed in heifer 10 during the second phase of this study. Although this heifer was overectomised, some ovarian tissue may potentially have been retained and allowed normal reproductive cycles to continue. Alternatively, interfering steroids (abovementioned in the procedure section) may have been present. These samples were analysed on plate 3 alongside QC samples described in appendix 1 table IX. QC samples analysed along side this sample group returned calculated progesterone concentrations much higher than their nominal conentration i.e Calculated concentrations of greater than 10 ng/mL were obtained for the 5ng/mL QC sample and an average calculated value of 5 ng/mL was returned for the 2 ng/mL QC sample. If QC accuracy is an adequate predictor of sample accuracy, the true concentration of the cow 6 day 4.5 sample may be only marginally higher than 2 ng/mL. Although low, this progesterone concentration would not likely hinder estrus synchronisation efficacy.

Day 9 plasma progesterone concentration was calculated to be less than 1 for all animals other than heifer 10 (QC data can be found in appendix 1 table XII). Blood progesterone concentration (9.7 ng/mL) remained high in this heifer.

On consideration of the plasma progesterone concentrations calculated by the ovucheck kit and the accuracy of QC samples that were analysed alongside test samples, it is quite likely that all animals, except cow 10 (device B), received an appropriate doseage of progesterone in period 1 of this study for efficacious estrus synchronisation.

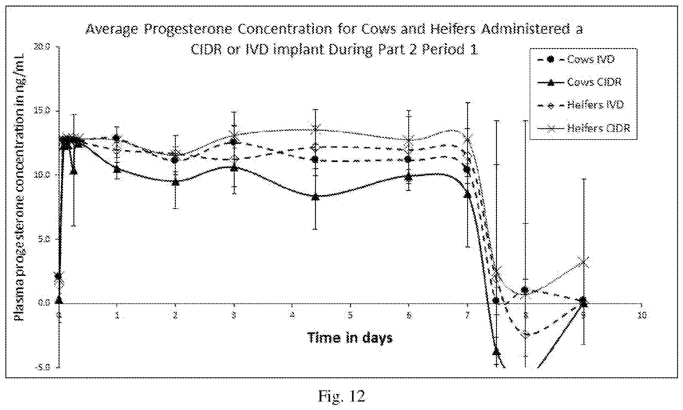

The average progesterone concentration for each test group was also calculated to model plasma progesterone concentration changes typical of each test group and allow comparison between test groups. This data is shown in table 24 and Fig. 12 .

*Table 24: The average change in Plasma progesterone concentrations (ng/mL) treatment A and B groups during part 2 period 1 of study JX1302-K005. 99.7% confidence intervals are shown. Note: shaded cells indicated that a progesterone releasing device was present in the animal at this time point.*

**JX1302-K005 Part 2 Period 1, baseline corrected averages, Analyst 1**

| Time | Cows | | | | Heifers | | | |
| --- | --- | --- | --- | --- | --- | --- | --- | --- |
| | A | | B | | A | | B | |
| | Ave | 99% CE | Ave | 99% CE | Ave | 99% CE | Ave | 99% CE |
| 0 | 2.1 | 1.83 | 0.4 | 0.38 | 1.5 | 2.48 | 2.1 | 3.54 |
| 2 h | 12.7 | 0.23 | 12.3 | 0.11 | 12.8 | 0.18 | 12.7 | 0.05 |
| 4 h | 12.8 | 0.20 | 12.4 | 0.18 | 12.7 | 0.13 | 12.9 | 0.05 |
| 6 h | 12.7 | 0.15 | 10.4 | 4.36 | 12.7 | 0.23 | 12.8 | 0.07 |
| 8 h | 12.6 | 0.04 | 12.5 | 0.12 | 12.7 | 0.16 | 12.8 | 0.02 |
| 1 d | 12.9 | 0.88 | 10.5 | 0.84 | 12.0 | 0.96 | 12.7 | 0.16 |
| 2 d | 11.1 | 1.12 | 9.5 | 2.13 | 11.7 | 1.39 | 11.6 | 1.53 |
| 3 d | 12.5 | 1.28 | 10.6 | 1.51 | 11.2 | 2.66 | 13.1 | 1.84 |
| 4.4 d | 11.2 | 1.23 | 8.4 | 2.62 | 12.2 | 1.65 | 13.6 | 1.55 |
| 6 d | 11.2 | 1.60 | 9.9 | 1.14 | 12.0 | 2.59 | 12.8 | 2.27 |
| 7 d | 10.4 | 0.49 | 8.6 | 4.17 | 11.5 | 2.12 | 12.8 | 2.87 |
| 7.5 d | 0.2 | 1.07 | -3.7 | 1.04 | 1.9 | 8.93 | 2.6 | 11.68 |
| 8 d | 1.0 | 5.18 | -5.9 | 0.55 | -2.4 | 4.29 | 0.7 | 13.51 |
| 9 d | 0.2 | 0.19 | 0.0 | 0.00 | 0.1 | 0.07 | 3.3 | 6.48 |

The trends in Fig. 12 imply similarity between plasma progesterone concentration changes attributable to each treatment. All test groups display the rapid rise in plasma progesterone concentration on Day 0, maintenance of greater than 2 ng/mL plasma progesterone until day 7 then rapid drop in plasma progesterone after removal of the device (on day 7) required for use in a hormonal treatment procedure for estrus synchronisation. Cows and heifers that were administered treatment B appear to show greater variation in progesterone concentration than those in the treatment A group; however, this apparent trend should be interpreted with caution as body mass is not accounted for in these graphs and the sample sizes (n=3) for each group is small.

### Period 2 of JX1302-K005 part 2

Part 2 period 2 of JX1302-K001 was conducted in an analogous manner to part 2 period 1 of this study with the exception that cows and heifers were given the treatment that they did not receive in the previous study period.

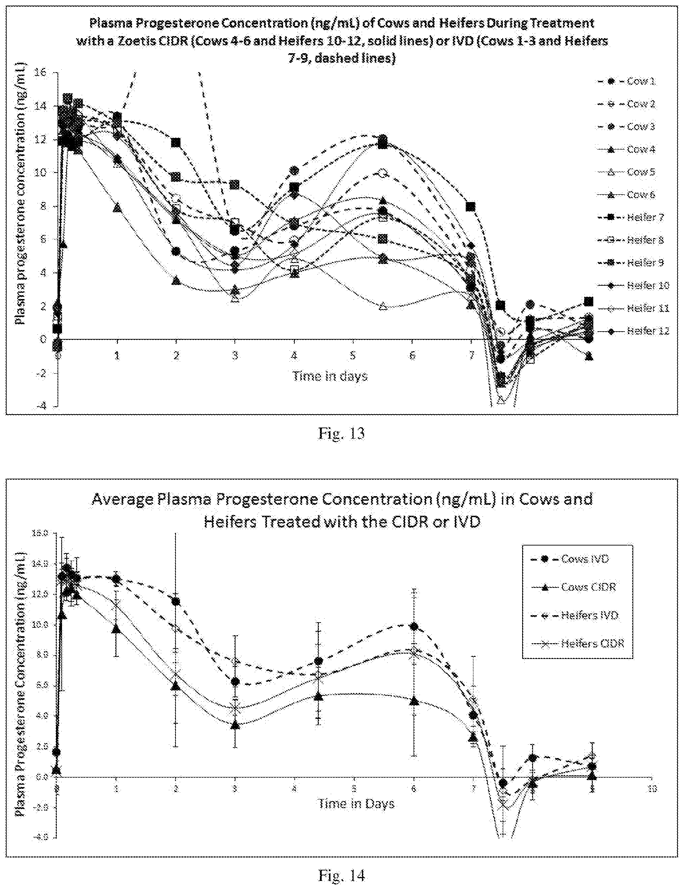

The measured plasma progesterone concentration for individual cows and heifers as recorded at each time point during part 2 period 2 are shown in table 25 and **Fig. 13.** Samples collected in this study period were analysed across 3 ELISA plates (numbered 5-7, data included in Appendix 1).

*Table 25: Plasma progesterone (ng/mL) content for each cow or heifer administered a CIDR® or Jurox IVD during study Part II period 2 of JX1302-K005. All data points are reported as obtained. Note: shaded cells indicated that a progesterone releasing device was present in the animal at this time point.*

**JX1302-K005 Part 2 Period 2, Analyst 2**

| Time | Cow # 1 | 2 | 3 | 4 | 5 | 6 | Heifer # 7 | 8 | 9 | 10 | 11 | 12 |
|---|---|---|---|---|---|---|---|---|---|---|---|---|
| 0.0 | 1.9 | 1.1 | 1.9 | 2.3 | -0.3 | -0.2 | 0.6 | 1.4 | -0.4 | 1.6 | -0.1 | -0.1 |
|  |  | B |  |  | A |  |  | B |  |  | A |  |
| 2 h | 13.1 | 13.7 | 12.9 | 5.7 | 13.8 | 12.6 | 11.9 | 13.2 | 13.7 | 12.7 | 12.9 | 13.1 |
| 4 h | 14.3 | 13.7 | 13.3 | 11.8 | 12.9 | 12.1 | 12.0 | 13.3 | 14.5 | 12.3 | 13.0 | 13.1 |
| 6 h | 13.3 | 13.7 | 12.9 | 11.7 | 13.6 | 12.0 | 11.6 | 13.5 | 13.6 | 12.6 | 12.8 | 12.8 |
| 8 h | 12.7 | 13.5 | 13.0 | 12.0 | 12.6 | 11.4 | 11.9 | 13.2 | 14.2 | 12.3 | 13.4 | 12.2 |
| 1 d | 13.4 | 12.6 | 13.2 | 10.9 | 10.6 | 8.0 | 13.1 | 12.6 | 12.9 | 10.9 | 10.7 | 12.2 |
| 2 d | 5.3 | 8.5 | 20.9 | 7.2 | 7.3 | 3.6 | 11.8 | 7.9 | 9.7 | 5.4 | 7.3 | 7.7 |
| 3 d | 5.3 | 7.0 | 6.5 | 5.1 | 2.5 | 3.0 | 6.6 | 7.0 | 9.3 | 4.2 | 5.0 | 4.5 |
| 4.5 d | 6.8 | 5.9 | 10.1 | 7.1 | 4.9 | 4.0 | 9.1 | 4.2 | 6.9 | 5.7 | 5.2 | 8.7 |
| 6 d | 7.7 | 9.9 | 12.0 | 8.4 | 2.0 | 4.9 | 11.7 | 7.3 | 6.0 | 11.8 | 7.5 | 4.9 |
| 7 d | 3.1 | 4.2 | 5.0 | 3.3 | 2.6 | 2.1 | 8.0 | 3.6 | 3.6 | 5.7 | 3.0 | 4.6 |
| 7.5 d | -1.1 | 0.4 | -0.4 | -7.5 | -3.6 | -2.6 | 2.1 | -2.4 | -2.2 | -0.7 | -2.2 | -2.5 |
| 8 d | 0.7 | 1.2 | 2.1 | 0.3 | -0.8 | -0.6 | 1.1 | -1.2 | -0.4 | -0.1 | -0.3 | -0.3 |
| 9 d | 0.1 | 1.3 | 0.8 | -1.0 | 0.8 | 0.6 | 2.3 | 1.2 | 0.9 | 1.3 | 0.5 | 0.4 |

Prior to treatment, progesterone concentration was calculated to be less than 2 ng/mL for all cows and heifers. Within 2 hours of administration of treatment A or treatment B all cows except cow 4 (5.7 ng/mL) reached plasma progesterone concentrations above the assay ULOQ (10 ng/mL), this level was reached by cow 4 at the 4 h blood test point. Plasma progesterone concentrations for all cows and heifers remained above the assay ULOQ for longer than 24 hours after administration. Blood samples collected on Day 0 and 1 were analysed on plate 5. On this plate, the QC sample with a concentration of 2 ng/mL returned acceptable accuracy for calculated values, however the 5 and 10 ng/mL QC standards returned calculated concentrations 20-30% lower than their nominal value (appendix 1, table XV). It is highly likely that the plasma progesterone conentration measured in cows and heifers from 4h to 24 hour is indeed higher than 10 ng/mL

From 2 days post administration, variation in results was observed. Despite this variability, measured plasma progesterone concentration remained adequately higher than the required plasma concentration (2 ng/mL) in all animals. Cow 5 (2.0 ng/mL) and cow 6 (2.1 ng/mL) were calculated to have marginal plasma progesterone concentration at the day 6 and 7 blood collection points repectively. Cow 6 also showed the lowest progeterone concentration in period 1 of this study. Most cows and heifers showed a general trend of decreasing plasma progesterone from day 2-3 and an increase in plasma progesterone from day 3-7. This may possibly be attributable to endogenous enzymatic regulation of plasma progesterone concentration. After removal of all devices on day 7, plasma progesterone concentration for all animals fell rapidly to below the LLOQ by the Day 7-12h timepoint. Blood samples taken on days 2 to 7 were analysed on plate 6. On this plate the accuracy of the 1 ng/mL QC standard was acceptable however the 2, 5 and 10 ng/mL samples all returned calculated concentrations 30-50% lower than their nominal value (appendix 1, table XVIII). It is possible that actual progesterone concentrations may higher than estimated by this assay if QC accuracy is a suitable predictor of sample accuracy. By the 7.5 day blood collection all cows and heifers with the exception of cow 7 (2.1 ng/mL) were estimated to have plasma progesterone concentrations of less than 1 ng/mL. Many of the calculated concentrations are negative values. These values are not possible and are most likely obtained due to poor predictive ability of the ovucheck kit at very low concentrations and/or the actual sample concentration being less the low levels of progesterone remaining blank bovine plasma used for the preparation of calibration standards.

Analysis of day 8 and 9 plasma progesterone concentration was performed on plate 7. By day 8, the plasma progesteone concentration for cow 7 had dropped to below 2 ng/mL but then rose to 2.1 ng/mL on day 9. The calculated plasma progesterone concentration for all other animals remained at a suitably low concentration for the remainder of the study. The 2 and 5 ng/mL QC samples on this plate returned calculated progesterone concentrations with suitable accuracy (C=2 ng/mL, 1.7 and 1.9 ng/mL; C=5 ng/mL, 4.6 and 4.8 ng/mL); however, the 1 ng/mL QC sample returned calculated concentrations 180-190% higher than expected (1.8 and 1.9 ng/mL, see Appendix 1 table XXI).

The plasma progesterone concentration changes for each individual animal in this phase of the study considered with the QC samples that were analysed alongside test samples support the likely hood of both test items delivered an appropriate doseage of progesterone over 7 days for estrus synchronisation. Additionally the average progesterone concentration and 99% confidence intervals were calculated for each test group and are displayed in table 26 and **Fig. 14** to enable comparisons between test groups and visualisation of trends.

The trends in **Fig. 14** imply that test groups display the rapid rise in plasma progesterone concentration on Day 0, maintenance of greater than 2 ng/mL plasma progesterone until day 7 then rapid drop in plasma progesterone after removal of the device (on day 7) required as part of a hormonal program for estrus synchronisation. Cows and heifers that were administered treatment A appear to show a lower concentration of progesterone during the period of day 1 to 4 than those in the treatment B group; however, this apparent trend should be interpreted with caution as body mass is

*Table 26: The average Plasma progesterone concentrations in ng/mL are shown in the table below for each test group in part 2 period 2 of study JX1302-K005. 99% confidence intervals are shown. Note: shaded cells indicated that a progesterone releasing device was present in the animal at this time point.*

| JX1302-K005 Part 2 Period 1, Analyst 2 | | | | | | | | |
| --- | --- | --- | --- | --- | --- | --- | --- | --- |
| | Cows | | | | Heifers | | | |
| Time | Ave | 99% CE | Ave | 99% CE | Ave | 99% CE | Ave | 99% CE |
| 0 | 1.6 | 0.56 | 0.6 | 1.70 | 0.5 | 1.08 | 0.4 | 1.13 |
| Treatment | B | | A | | B | | A | |
| 2 h | 13.2 | 0.48 | 10.7 | 5.04 | 13.0 | 1.10 | 12.9 | 0.19 |
| 4 h | 13.8 | 0.58 | 12.3 | 0.67 | 13.3 | 1.40 | 12.8 | 0.47 |
| 6 h | 13.3 | 0.44 | 12.4 | 1.16 | 12.9 | 1.32 | 12.7 | 0.11 |
| 8 h | 13.1 | 0.45 | 12.0 | 0.66 | 13.1 | 1.33 | 12.6 | 0.80 |
| 1 d | 13.1 | 0.46 | 9.8 | 1.85 | 12.9 | 0.34 | 11.3 | 0.93 |
| 2 d | 11.6 | 9.55 | 6.0 | 2.44 | 9.8 | 2.28 | 6.8 | 1.42 |
| 3 d | 6.3 | 1.00 | 3.5 | 1.56 | 7.6 | 1.68 | 4.6 | 0.49 |
| 4.4 d | 7.6 | 2.52 | 5.3 | 1.86 | 6.7 | 2.85 | 6.5 | 2.19 |
| 6 d | 9.9 | 2.49 | 5.1 | 3.66 | 8.3 | 3.44 | 8.1 | 4.01 |
| 7 d | 4.1 | 1.08 | 2.7 | 0.66 | 5.1 | 2.88 | 4.4 | 1.56 |
| 7.5 d | -0.4 | 0.91 | -4.6 | 3.03 | -0.9 | 2.91 | -1.8 | 1.08 |
| 8 d | 1.3 | 0.83 | -0.4 | 0.65 | -0.2 | 1.33 | -0.2 | 0.13 |
| 9 d | 0.7 | 0.73 | 0.2 | 1.12 | 1.4 | 0.85 | 0.7 | 0.57 |

not accounted for in these graphs and the sample sizes (n=3) for each group is small.

### *Progesterone remaining in devices*

Devices were collected after period 1 of this study and analysed for residual progesterone. The calculated amount of residual progesterone for implants used in this study is summarised in table 27. On average a slightly higher amount of residual progesterone remained in the IVD implants used in both test groups than the CIDR® implants. This was to be expected, given the higher dose of progesterone incorporated in the IVD (1540 mg vs. 1340 mg).

*Table 27: The amount of residual progesterone calculated for each implant and average residual progesterone remaining in devices of each test group.*

| Cow/heifer | Device | mass remaining (mg) | Average mass remaining (mg) | Standard deviation |
|---|---|---|---|---|
| 1 | CIDR | 726 | 709 | 17 |
| 2 | CIDR | 707 | | |
| 3 | CIDR | 693 | | |
| 4 | IVD | 964 | 822 | 167 |
| 5 | IVD | 865 | | |
| 6 | IVD | 638 | | |
| 7 | CIDR | 675 | 737 | 58 |
| 8 | CIDR | 746 | | |
| 9 | CIDR | 790 | | |
| 10 | IVD | 953 | 929 | 39 |
| 11 | IVD | 884 | | |
| 12 | IVD | 949 | | |

On dismantling CIDR® devices it was noted that yellow, pink and brown bacterial films had growth in the space between the silicone drug delivery material and the device spine. On segmentation of the IVD device after use no gap between the drug delivery material and spine was observed and no bacterial films were present. The drug delivery material of the IVD device cannot be removed from the spine with manual force.

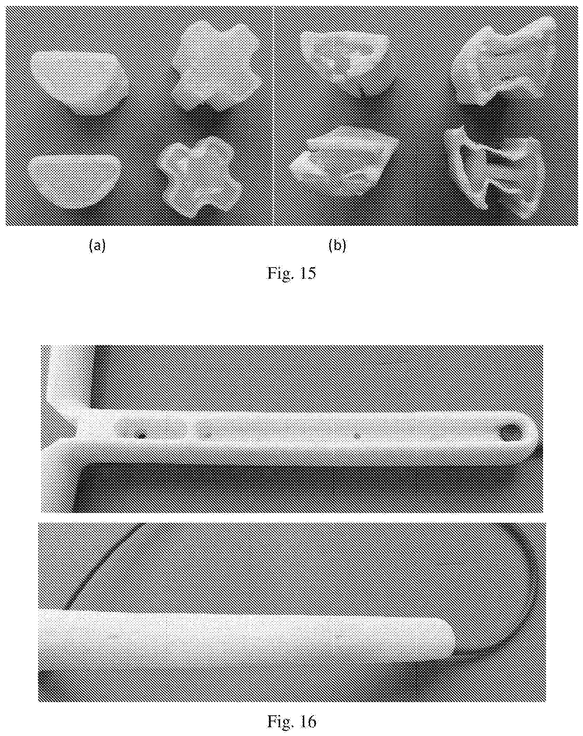

**Fig. 15** shows cross sections of the elongate body and wing of an example device of this invention (a) and cross sections of the elongate body and wing of a competitor's silicone device (b). It is thought that microbes may enter this space through small slits and holes in the drug delivery material **(fig. 16).**

The devices of the present disclosure have clear advantages over known devices in that due to their design, they do not create an environment for bacterial growth, despite the fact that they contain residual progesterone following use. More specifically, in preferred embodiments, the disclosed device comprises a TPE polymer impregnated with a drug that is over-moulded onto a support medium which is in the form of a spine wherein the spine and the TPE have melt points in approximately the same range. Without being bound by theory, the inventors believe that such a device allows the two materials to fuse to an extend that there are no gaps between the two materials to allow bacteria to enter and multiply. The device disclosed herein is advantageously sterile.

### *Pharmacokinetic study JX1302-L011 using LCMS for the determination of plasma progesterone concentration*

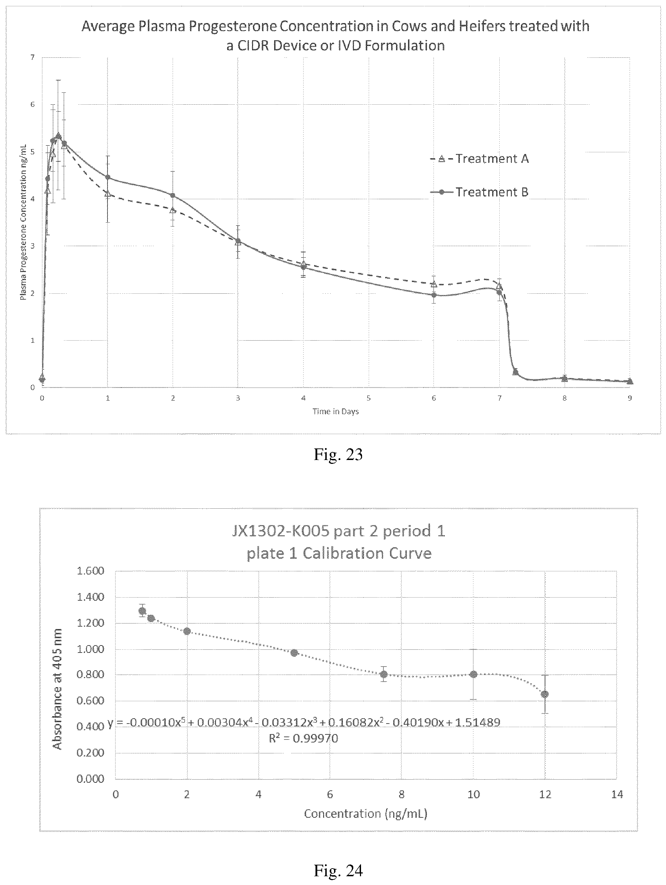

An additional comparison of the progesterone pharmokinetics exhibited by the CIDR® device and a IVD device described herin in example 12 was a conducted under study number JX1302-L011. This study was a two period cross over study conducted in an analogous manner to part 2 period 1 and 2 of JX1302-K005 described with the exception that standard LCMS analytical techniques were used to determine plasma progesterone concentration. A higher degree of acuracy and precision was achieved using LCMS methods. The calculated concentration of quality control (QC) samples was within 85-115% of the known concentration and precision of repeated analysis was less than 15% over the concentration range for samples tested. The plasma progesterone concentration for individual cows and heifers as recorded at each time point during JX1302-L011 period 1 and 2 are shown in table 28 for treatment A and table 29 for treatment B. The average plasma progesterone concentrations during both periods of the study are shown in **Fig. 23.**

*Table* 28: *Plasma progesterone concentration for cows and heifers administered treatment A (UK CIDR®) during the two period cross over study JX1302-L011. Treatment was administered at t=0 and removed at t=7 days. StDev = Standard Deviation, SE = Standard Error.*

| Time (days) | Cow numbers | | | | | | Heifer numbers | | | | | | Ave | St Dev | SE |
|---|---|---|---|---|---|---|---|---|---|---|---|---|---|---|---|
| | 1 | 2 | 3 | 4 | 5 | 6 | 7 | 8 | 9 | 10 | 11 | 12 | | | |
| 0 | 0.12 | 0.59 | 0.16 | 0.55 | 0.07 | 0.75 | 0.01 | 0.03 | 0.00 | 0.09 | 0.30 | 0.16 | 0.24 | 0.25 | 0.15 |
| 0.08 | 3.06 | 3.43 | 1.49 | 4.04 | 6.44 | 4.51 | 5.33 | 1.33 | 4.45 | 6.19 | 5.57 | 4.40 | 4.19 | 1.65 | 0.95 |
| 0.17 | 3.19 | 4.72 | 1.95 | 5.03 | 6.79 | 6.18 | 6.74 | 1.91 | 4.18 | 6.89 | 6.00 | 5.92 | 4.96 | 1.81 | 1.04 |
| 0.25 | 3.18 | 5.94 | 1.52 | 6.05 | 6.68 | 6.06 | 7.07 | 2.16 | 4.59 | 6.91 | 7.23 | 6.89 | 5.36 | 2.01 | 1.16 |
| 0.33 | 3.72 | 6.98 | 1.43 | 5.09 | 6.71 | 6.69 | 6.10 | 1.57 | 4.64 | 6.60 | 6.53 | 5.52 | 5.13 | 1.96 | 1.13 |
| 1 | 3.02 | 4.60 | 2.88 | 4.75 | 5.90 | 5.37 | 5.15 | 2.42 | 3.32 | 4.09 | 4.03 | 3.94 | 4.12 | 1.08 | 0.62 |
| 2 | 3.36 | 4.33 | 3.20 | 3.29 | 4.70 | 4.26 | 4.46 | 3.18 | 3.37 | 3.58 | 3.10 | 4.38 | 3.77 | 0.60 | 0.35 |
| 3 | 2.90 | 2.88 | 2.97 | 3.38 | 4.22 | 3.78 | 3.14 | 2.39 | 2.10 | 3.66 | 3.12 | 2.51 | 3.09 | 0.61 | 0.35 |
| 4 | 2.87 | 3.14 | 2.95 | 2.42 | 3.21 | 2.95 | 2.87 | 1.96 | 2.06 | 2.59 | 2.32 | 2.19 | 2.63 | 0.43 | 0.25 |
| 6 | 2.11 | 2.37 | 2.52 | 2.08 | 2.44 | 2.29 | 2.71 | 2.19 | 2.21 | 1.93 | 1.78 | 1.75 | 2.20 | 0.29 | 0.17 |
| 7 | 2.22 | 2.18 | 2.16 | 2.02 | 2.57 | 2.58 | 2.43 | 2.05 | 1.96 | 1.86 | 2.06 | 1.89 | 2.16 | 0.25 | 0.14 |
| 7.25 | 0.39 | 0.49 | 0.49 | 0.20 | 0.37 | 0.47 | 0.24 | 0.20 | 0.13 | 0.29 | 0.41 | 0.37 | 0.34 | 0.12 | 0.07 |
| 8 | 0.23 | 0.39 | 0.26 | 0.19 | 0.23 | 0.30 | 0.12 | 0.02 | 0.03 | 0.17 | 0.22 | 0.25 | 0.20 | 0.10 | 0.06 |
| 9 | 0.17 | 0.22 | 0.18 | 0.12 | 0.16 | 0.27 | 0.03 | 0.00 | 0.00 | 0.14 | 0.17 | 0.22 | 0.14 | 0.09 | 0.05 |
| Period | 1 | 1 | 1 | 2 | 2 | 2 | 1 | 1 | 1 | 2 | 2 | 2 | N/A | N/A | N/A |

EP 3 400 050 B1

*Table* 29: Plasma progesterone concentration for cows and heifers administered treatment B (IVD device disclosed herein) during the two period cross over study JX1302-L011. Treatment was administered at t=0 and removed at t=7 days. StDev = Standard Deviation, SE = Standard Error.

| Time (days) | Cow numbers | | | | | | Heifer numbers | | | | | | Ave | St Dev | SE |
|---|---|---|---|---|---|---|---|---|---|---|---|---|---|---|---|
| | 1 | 2 | 3 | 4 | 5 | 6 | 7 | 8 | 9 | 10 | 11 | 12 | | | |
| 0 | 0.03 | 0.42 | 0.08 | 0.11 | 0.08 | 0.63 | 0.12 | 0.14 | 0.03 | 0.01 | 0.20 | 0.04 | 0.16 | 0.18 | 0.11 |
| 0.08 | 4.36 | 3.26 | 3.01 | 5.03 | 5.62 | 4.25 | 4.38 | 3.78 | 4.14 | 5.27 | 6.23 | 3.87 | 4.43 | 0.95 | 0.55 |
| 0.17 | 3.84 | 3.72 | 4.32 | 6.19 | 6.83 | 5.15 | 5.14 | 5.06 | 4.39 | 6.52 | 6.98 | 4.76 | 5.24 | 1.14 | 0.66 |
| 0.25 | 4.09 | 4.40 | 4.24 | 5.51 | 6.61 | 5.28 | 5.60 | 4.87 | 4.72 | 6.56 | 6.68 | 5.38 | 5.33 | 0.92 | 0.53 |
| 0.33 | 4.25 | 5.23 | 4.48 | 5.11 | 6.53 | 6.07 | 5.58 | 4.29 | 4.79 | 5.63 | 6.31 | 3.99 | 5.19 | 0.85 | 0.49 |
| 1 | 4.64 | 4.28 | 3.44 | 3.24 | 4.73 | 4.56 | 4.81 | 3.46 | 4.19 | 5.13 | 5.55 | 5.55 | 4.46 | 0.78 | 0.45 |
| 2 | 4.39 | 4.04 | 2.97 | 2.94 | 4.20 | 4.08 | 4.11 | 4.46 | 3.37 | 4.10 | 3.79 | 6.43 | 4.07 | 0.90 | 0.52 |
| 3 | 3.65 | 3.04 | 2.80 | 2.65 | 2.70 | 2.57 | 3.50 | 3.38 | 2.99 | 3.37 | 3.75 | 3.03 | 3.12 | 0.40 | 0.23 |
| 4 | 2.62 | 2.37 | 2.19 | 2.80 | 2.64 | 2.74 | 2.29 | 2.06 | 2.07 | 2.83 | 3.27 | 2.74 | 2.55 | 0.36 | 0.21 |
| 6 | 1.86 | 1.89 | 1.63 | 1.99 | 2.30 | 2.21 | 2.06 | 1.53 | 1.64 | 2.06 | 2.56 | 1.85 | 1.97 | 0.30 | 0.17 |
| 7 | 1.85 | 1.80 | 1.89 | 1.83 | 2.29 | 2.34 | 2.14 | 1.62 | 1.81 | 2.23 | 2.63 | 1.81 | 2.02 | 0.30 | 0.17 |
| 7.25 | 0.32 | 0.30 | 0.24 | 0.33 | 0.48 | 0.57 | 0.28 | 0.22 | 0.19 | 0.40 | 0.36 | 0.34 | 0.34 | 0.11 | 0.06 |
| 8 | 0.17 | 0.19 | 0.14 | 0.24 | 0.32 | 0.29 | 0.19 | 0.14 | 0.11 | 0.18 | 0.17 | 0.19 | 0.19 | 0.06 | 0.04 |
| 9 | 0.17 | 0.17 | 0.10 | 0.23 | 0.18 | 0.12 | 0.17 | 0.12 | 0.12 | 0.09 | 0.00 | 0.07 | 0.13 | 0.06 | 0.03 |
| Period | 1 | 1 | 1 | 2 | 2 | 2 | 1 | 1 | 1 | 2 | 2 | 2 | N/A | N/A | N/A |

Both the registered CIDR® product (treatment A) and IVD device disclosed within (treatment B) brought about a rapid increase of plasma progesterone concentration on administration and ensured that plasma progesterone concentrations were sustained at around 2 ng/mL up to their removal 7 days after administration. As there is very little difference in the plasma progesterone concentration of cows and heifers when administered the CIDR® or IVD it is highly likely that the device disclosed herein is also effective for estrous synchronisation.

**Discussion of results**

In summary, several formulations of P4 in commercial elastomers and thermoplastic elastomers were prepared and the P4 release profile of these materials was compared to sections of or entire units of the CIDR® silicone based P4 releasing vaginal implant. These test P4 releasing compounds included varied grades of the elastomer EVA as well as commercially available SEBS block copolymers such as Mediprene® and Thermolast K®.

By alteration of the percentage active material in the formulation and selection of the appropriate grade of SEBS block copolymer, a preferred formulation of P4 in a SEBS copolymer Mediprene® was able to be tuned to release P4 into dissolution media with a highly similar rate as that of the CIDR®. No other drug release mechanism or barrier was required.

Additionally, one early prototype (spine prototype 1 shown in **fig. 1A** with progesterone and TPE drug release component and an elongate body length of about 141.70mm) was retained in a heifer for 7 days and mediated similar changes in plasma progesterone levels to cows and heifers treated with the CIDR® implant for this time. On modification of the prototype spine design to an elongate body length of about 124 mm (as depicted in **fig. 1C** and **2C**), implants spine prototype 2 could be retained for 7 days in all cows and heifers tested. It is anticipated that implants encompassing spine prototype 1 would also be retained if moulded in more polymer resins that are more inflexible than LDPE such as HDPE, polypropylene or other thermoplastics or TPEs.

The CIDR® has been shown to be efficacious for synchronisation of Bovine estrus. It has also been shown that such a device, as that pictured in **Fig.1-3**. is efficacious for synchronising bovine estrus *in vivo.*

While it is also possible that the appropriate grade of EVA with an appropriate level of active material could also match the *in vitro* P4 dissolution profile of the CIDR, SEBS polymers were chosen for advanced development work as their durability and mechanical properties are superior to those of EVA and Silicon.

**Comparison of Progesterone *in vitro* dissolution from the Jurox IVD to a competitor product (CIDR®)**

**Background**

Progesterone is a Class II, high membrane permeability, low aqueous solubility drug according to the Biopharmaceutical Classification System. As a result, the equilibrium driven diffusion of progesterone from any drug delivery device into the cow vaginal environment is primarily limited by:

- The low solubility of progesterone (< 0.1 g/L aqueous pH 7 buffer)

- Ability of the dissolution solvent to penetrate the drug delivery matrix

- Interactions between progesterone and the drug delivery matrix

- Ability of the solvent to surround the progesterone molecule (dissolution)

- Ability of progesterone to diffuse out of the drug delivery matrix

The dissolution of progesterone from the device disclosed herein (which may hereinafter be referred to as the Jurox IVD or IVD) has been compared to competitor commercially available device, the CIDR®, in an organic solvent to investigate the interactions between progesterone and the drug delivery matrix and the ability of progesterone to diffuse out of the drug delivery matrix under sink conditions without the limitation of poor progesterone solubility. The solvent acetone was used as it provides excellent progesterone solubility and penetrates polymers well.

The Jurox IVD used in this experiment is as described in Example 12. It comprises a drug release component that consists of Mediprene 500452M impregnated with progesterone and overmoulded onto a spine made of LDPE according to the method disclosed in Example 12.

**Example 13. Progesterone dissolution from IVD prototypes (n=6) to the UK CIDR® (n=6) in an organic solvent**

**Experimental.**

Six Jurox IVD devices as described above, and six UK CIDR devices were cut into 7 pieces and placed into individual reagent bottles already equipped with a magnetic elliptical stirrer bead 1.38 g of progesterone (BP) was weighed into a reagent bottle prepared in the same way. The combined mass of each reagent bottle and solid contents was recorded ($M_i$) and each bottle was then charged with 250 mL of acetone. Reagent bottles were heated to 35°C in a temperature controlled water bath and stirred using a submersible multipoint stirrer plate.

Reagent bottles were removed from the water bath at times 2, 4.5 and 47.5 hours after the commencement of stirring. All residual water from the temperature controlled water bath was removed from the outside of the container. The total mass of the dissolution vessel, drug product and drug product solution was then recorded ($M_f$) and the reagent bottles were left at ambient temperature. The initial mass (of components prior to solvent addition) was subtracted from the final mass to give the solvent mass at each time point.

The temperature of each solution of device extracts was recorded at 2.5, 5 and 48 hours after stirring commenced and a 2 mL aliquot of solution was sampled immediately following the temperature reading. The aliquot of extracts were diluted in 100 mL of 50:50 acetonitrile/water and a sample of the diluted solution was filtered into HPLC vials

The mass of solvent was converted to its corresponding volume using the literature density of acetone and equation below.

$$Final\ Volume\ (mL) = \frac{M_f(g) - M_i(g)}{\rho\left(\frac{g}{mL}\right)}$$

**Density ($\rho$)=** literature value for acetone at the solution temperature

**Final mass ($M_f$)=** mass recorded immediately prior to sampling

= m(dissolution vessel) + m(drug product) + m(drug product solution)

**Initial mass ($M_i$)=** mass of all components prior to solvent addition

= m(dissolution vessel) + m(drug product)

A progesterone dissolution profile and final content for each implant was be obtained using a HPLC/UV method for progesterone quantification in IVD and CIDR

**Results**

Table 30 summarises the dissolution of progesterone from Jurox IVD and CIDR devices at these time points (2.5, 5 and 48 hours). Results are expressed as a percentage of the dose concentration (1.38 g) and are displayed graphically in Figure 2.

Table 30. Dissolution of progesterone from n=6 IVD prototypes and n=6 CIDR® implants and 1.38 g of progesterone API.

| Time in Hours | 2.5 | 5 | 48 |
|---|---|---|---|
| 1.38 g P4 standard | 100.8 | 104. 1 | 103. 2 |
| Jurox IVD 1 | 47.0 | 65.1 | 103. 5 |
| Jurox IVD 2 | 53.5 | 73.6 | 108. 7 |
| Jurox IVD 3 | 50.2 | 69.5 | 102. 8 |
| Jurox IVD 4 | 48.6 | 64.5 | 98.8 |
| Jurox IVD 5 | 51.6 | 69.3 | 100. 7 |
| Jurox IVD 6 | 47.6 | 65.0 | 101. 0 |

(continued)

| Time in Hours | 2.5 | 5 | 48 |
|---|---|---|---|
| ave IVD | 49.7 | 67.8 | 102. 6 |
| RSD | 4.6 | 4.8 | 3.0 |
| *1.38 g P4 standard* | 100.8 | 104. 1 | 103.2 |
| CIDR 1 | 100.8 | 101. 3 | 98.8 |
| CIDR 2 | 99.3 | 101. 3 | 100.7 |
| CIDR 3 | 99.3 | 100. 3 | 101.0 |
| CIDR 4 | 99.0 | 99.0 | 100.9 |
| CIDR 5 | 100.6 | 101. 1 | 101.8 |
| CIDR 6 | 102.0 | 102. 4 | 101.3 |
| ave CIDR | 100.2 | 100. 9 | 100.7 |
| RSD | 1.1 | 1.1 | 0.9 |

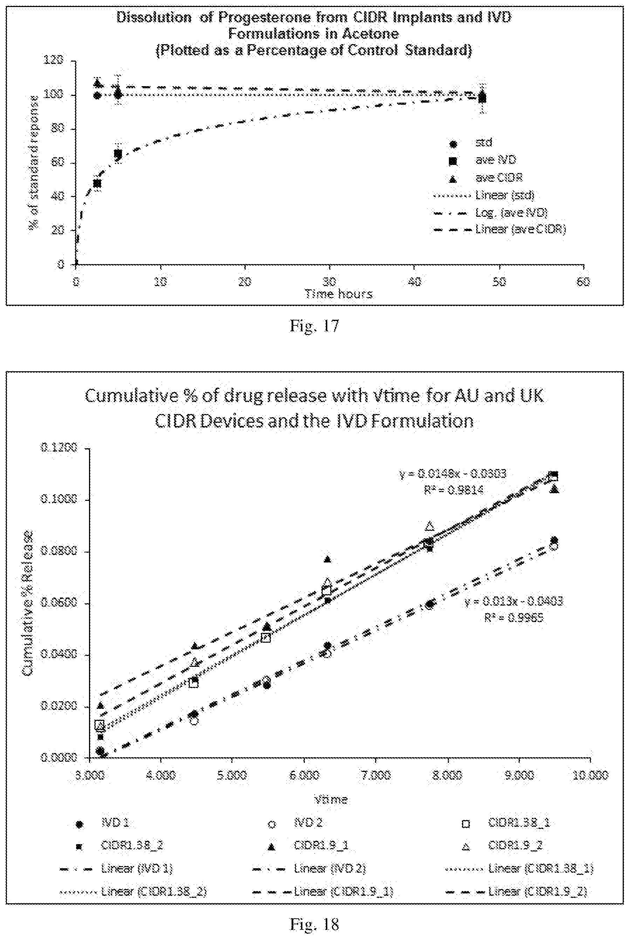

**Fig. 17** shows dissolution of progesterone from the CIDR occurs entirely before the 2.5 hour sample point, while the Jurox IVD had only released on average 48% and 65% of its total dose at the 2.5 and 5 hour time points respectively. It is clear that the competitor silicone product has a significantly different progesterone release mechanism to the Jurox IVD device when the limitation of progesterone solubility are removed.

**Example 14. Progesterone dissolution from Jurox IVD prototypes, the UK CIDR® and Australian CIDR in Simulation Vaginal Fluid.**

**Background**

The rate of extraction of progesterone from the Jurox IVD drug delivery component or layer depends on solvent entry into the polymer matrix, solubilisation of the progesterone and diffusion of progesterone through and out of the polymer matrix. To provide an estimate of the rate of extraction of progesterone from the IVD into the cow vaginal fluid, the initial release rate for progesterone into SVF was investigated. Note: It is difficult to maintain sink conditions for 1.38 g of progesterone in SVF due to the limited solubility of progesterone in aqueous solvents. For this reason only the initial rate of progesterone release was monitored. Identification of the initial progesterone release profile in SVF will provide information to suggest a mechanism by which the drug substance is released *in vitro* and *in vivo*. The initial rate of progesterone dissolution from the Jurox IVD (1.38 g dose) was compared to a UK and AU registered silicone drug delivery product, the CIDR (1.38 and 1.9 g dose) in simulation vaginal fluid (SVF) was compared.

**Experimental**

The mass of two Jurox IVD devices (1.38 g progesterone dose), two UK CIDR devices (1.38g progesterone dose) and two AUS CIDR devices (1.9g progesterone dose) were taken before placing the devices in 1000 mL of SVF equilibrated to 37°C in a temperature controlled water bath. A 1 mL aliquot of the extracts solution was taken from each preparation at 10, 20, 30, 40, 60 and 90 minutes, then again at 24, 45 and 67 hours. Aliquots were analysed by HPLC without further preparation.

In SVF the initial velocity of progesterone release from the IVD follows zero-order kinetics as shown by the Higuchi model **(Fig. 18)** This rate is concentration independent and is directly due to the diffusivity of progesterone through the matrix of the drug embedded polymer. Due to the small amount of drug released (12 mg, 0.9%), these results may or may not reflect diffusion *in vivo*.

**Example 15. Progesterone dissolution from Jurox IVD prototypes, the UK CIDR® and Australian CIDR in an organic solution.**

The rate of progesterone dissolution from the Jurox IVD (1.38 g progesterone dose) was compared to a UK and AU registered competitor product with a silicon elastomer drug delivery matrix, the CIDR (1.38 and 1.9 g progesterone dose) in an organic solvent (Acetone) was compared.

**Experimental**

The mass of two Jurox IVD devices, two UK CIDR devices (1.38g progesterone dose) and two AUS CIDR devices (1.9g progesterone dose) were taken before placing the devices in 1000 mL of acetone equilibrated to 37°C in a temperature controlled water bath. A 1 mL aliquot was taken at 2, 4, 6, 8, 10, 15, 20, 30, 45, 60, 75 and 90 minutes. Aliquots were analysed by HPLC without further preparation.

**Results**

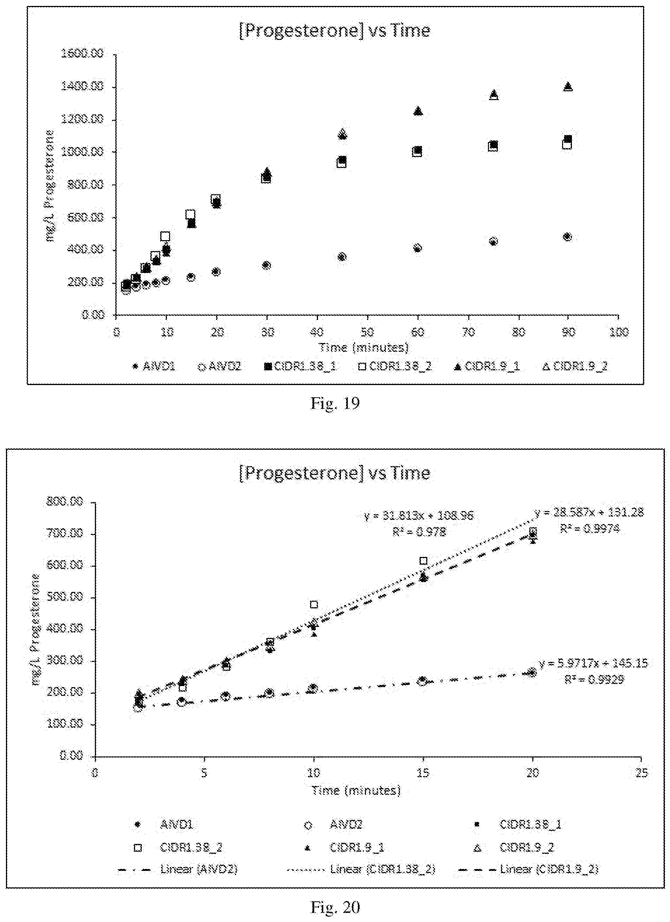

An organic solvent (acetone) was used to extract progesterone due to its high solubility of the drug, and the rate at which the device is depleted of progesterone. Under these conditions it is clear that the IVD has a much slower release profile to the CIDR. CIDR is a silicone based drug containing polymer that appears to release quickly **(Fig. 19)** under these conditions with approximately 70-80% of the dose released in 90 mins. Additionally the initial velocity of a 1.38g and 1.9g progesterone content CIDR were identical **(Fig. 20)**. The IVD releases approximately 35% of the dose in this 90 minutes period indicating a different mechanism for the progesterone to be extracted from the device. These factors include the ability of the polymer to swell (due to different combinations of domains and segments of the TPE-S), and tortuosity of progesterone to escape/diffuse out of the polymer matrix. This would consequently alter the ability of the media to penetrate to the inner most part of the polymer and vary the fluctuating void size within the matrix to accommodate the free volume theory.

**Example 16. Progesterone dissolution from in-house manufactured drug delivery polymers at 0.5, 1, 1.38 and 1.7 g loading of progesterone in Mediprene®500452M.**

**Experimental**

In-house manufactured drug delivery polymers were prepared at progesterone in mediprene 500452M concentrations equivalent to those of the Juorx IVD products with a final dose of 0.5, 1.0, 1.38 and 1.7 g of progesterone per device. Polymers were made by heating 11.1 g of mediprene® until molten (~210°C) before adding the amount of 95% progesterone masterbatch (table 31) to the molten mediprene® and mixing well.

*Table 31: Reagents used to prepare progesterone test polymers.*

| Dose equivalent (g/device) | Mass of progesterone masterbatch (g) | Mass of Mediprene® (g) | Total mass (g) | % progesterone |
|---|---|---|---|---|
| 0.5 | 0.52 | 11.1 | 11.62 | 4.3 |
| 1 | 1.04 | 11.1 | 12.14 | 8.2 |
| 1.38 | 1.44 | 11.1 | 12.54 | 11.0 |
| 1.7 | 1.77 | 11.1 | 12.87 | 13.2 |

Once suitable mixed, the molten material was pressed into sheets of approximately 2 mm thickness and allowed to cool. Two sheets from each preparation (8 polymer sheets) were cut into rectangles to obtain a uniform surface area whereby a total volume could be determined.

The mass of each sheet was recorded before placing the polymer in 500 mL of acetone equilibrated to 37°C in a temperature controlled water bath. A 1 mL aliquot was taken at 2, 4, 6, 8, 10, 15 and 20 minutes and at two additional points between 69 and 350 minutes. Samples were analysed by HPLC without further preparation.

**Results**

Evaluation of lab-made polymer blends at varied progesterone content revealed that initially, progesterone release is predominately a partition controlled process; however, after the drug diffuses out, progesterone release is primarily matrix controlled (time-dependent). An increase in cumulative amount of drug released was seen with initial drug loading in accordance to Higuchi. However, this was true only up until a limit of between 1.38 and 1.7 g of progesterone per mock device where the drug-released flux depends linearly on $(2A)^{1/2}$, characteristic of a matrix-type delivery system. This linear relationship describes the accumulated amount of the drug released from a unit of the surface area with the square root of time.

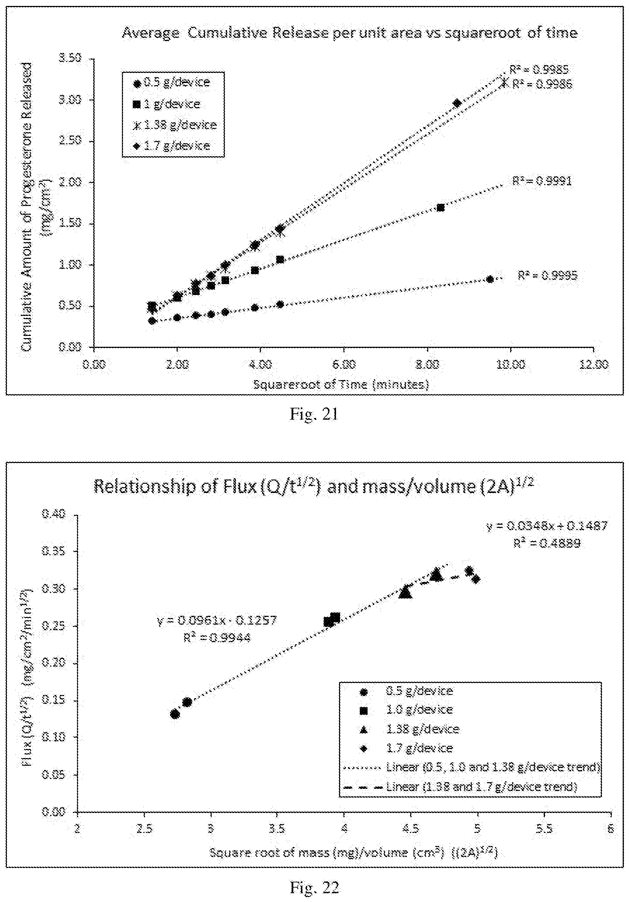

**Fig. 21** shows the Cumulative release of progesterone with increasing square root of time (minutes) with increasing loading dose. Generally, the rate of progesterone release increases as the progesterone concentration of the preparation increases. There is however, an upper limit to progesterone release rate that is reached between dose loadings of 1.38 g/device and 1.7 g/device.

**Fig. 22** shows the Flux plotted against the square root of mass/volume ratio for progesterone dose per device loadings. A linear relationship is seen between flux and square root of mass/volume ratio up to a maximum progesterone dose of value somewhere between 1.38 and 1.7 g/device.

**Summary:**

Jurox IVD and competitor product (CIDR - silicone polymer matrix) were found to follow zero-order release rate in SVF. As the CIDR® shows greatly increased progesterone release velocity in acetone compared with the IVD but more similar progesterone release in SVF and *in vivo,* it is highly likely that progesterone release in SVF and *in vivo* is solubility limited. This indicates that progesterone distribution or matrix structure differs greatly for the CIDR® and IVD.

**APPENDIX**

**Appendix 1: Plate set up, calibration curves and QC accuracy**

**Appendix 1.1. JX1302-K005 Part 2 Period 1 Calibration curves and QC samples**

**1.1.1 Plate 1**

*Table I: Plate 1 sample layout (Day 0).*

|   | 1 | 2 | 3 | 4 | 5 | 6 | 7 | 8 | 9 | 10 | 11 | 12 |
|---|---|---|---|---|---|---|---|---|---|----|----|----|
| a | d | d | blank 1 | QC-1 | Pre-Tx-1 | Pre-Tx-9 | 2h-5 | 4h-1 | 4h-9 | 6h-5 | 8h-1 | 8h-9 |
| b | e | e | blank 3 | QC-1 | Pre-Tx-2 | Pre-Tx-10 | 2h-6 | 4h-2 | 4h-10 | 6h-6 | 8h-2 | 8h-10 |
| c | f | f | blank 5 | QC-2 | Pre-Tx-3 | Pre-Tx-11 | 2h-7 | 4h-3 | 4h-11 | 6h-7 | 8h-3 | 8h-11 |
| d | g | g | blank 7 | QC-2 | Pre-Tx-4 | Pre-Tx-12 | 2h-8 | 4h-4 | 4h-12 | 6h-8 | 24h-1 | 8h-12 |
| e | h | h | blank 8 | QC-3 | Pre-Tx-5 | 2h-1 | 2h-9 | 4h-5 | 6h-1 | 6h-9 | 8h-5 | 8h-4 |
| f | i | i | blank 11 | QC-3 | Pre-Tx-6 | 2h-2 | 2h-10 | 4h-6 | 6h-2 | 6h-10 | 8h-6 | QC3 |
| g | j | j | WFI | QC-4 | Pre-Tx-7 | 2h-3 | 2h-11 | 4h-7 | 6h-3 | 6h-11 | 8h-7 | QC4 |
| h | blank | blank | QC6 | QC-4 | Pre-Tx-8 | 2h-4 | 2h-12 | 4h-8 | 6h-4 | 6h-12 | 8h-8 | QC5 |

*Table II: Plate 1 sample absorbance (Day 0).*

|   | 1 | 2 | 3 | 4 | 5 | 6 | 7 | 8 | 9 | 10 | 11 | 12 |
|---|---|---|---|---|---|---|---|---|---|----|----|----|
| a | 0.578 | 0.723 | 1.615 | 0.702 | 1.317 | 1.578 | 0.665 | 0.484 | 0.573 | 0.593 | 0.59 | 0.63 |
| b | 0.901 | 0.708 | 1.188 | 0.725 | 1.151 | 0.929 | 0.69 | 0.445 | 0.474 | 0.606 | 0.573 | 0.487 |
| c | 0.836 | 0.777 | 1.511 | 0.822 | 1.05 | 1.462 | 0.521 | 0.598 | 0.442 | 0.474 | 0.56 | 0.475 |
| d | 0.974 | 0.966 | 0.929 | 0.723 | 1.29 | 1.353 | 0.453 | 0.732 | 0.486 | 0.48 | 0.586 | 0.485 |

(continued)

|   | 1 | 2 | 3 | 4 | 5 | 6 | 7 | 8 | 9 | 10 | 11 | 12 |
|---|---|---|---|---|---|---|---|---|---|---|---|---|
| e | 1.133 | 1.143 | 1.351 | 0.393 | 1.438 | 0.518 | 0.595 | 0.65 | 0.537 | 0.629 | 0.646 | 0.865 |
| f | 1.23 | 1.241 | 1.555 | 1.11 | 1.456 | 0.476 | 0.561 | 0.645 | 0.503 | 0.524 | 0.662 | 0.643 |
| g | 1.321 | 1.272 | 2.084 | 1.252 | 1.043 | 0.639 | 0.547 | 0.558 | 0.614 | 0.464 | 0.513 | 0.761 |
| h | 1.462 | 1.449 | 0.287 | 1.265 | 1.323 | 0.72 | 0.525 | 0.475 | 0.897 | 0.496 | 0.561 | 1.008 |

*Table III: Plate 1, Time points from Day 0.*

| Thereretical conc ng/mL | Measured conc ng/mL | Accuracy % | Average conc ng/mL | Ave accuracy % |
|---|---|---|---|---|
| 10.0 | 12.3 | 123 | 12.8 | 122 |
| 10.0 | 12.2 | 122 | | |
| 5.0 | 7.4 | 149 | 9.8 | 196 |
| 5.0 | 12.2 | 244 | | |
| 2.0 | 13.0 | 651 | 7.7 | 386 |
| 2.0 | 2.4 | 122 | | |
| 1.0 | 1.0 | 95 | 0.9 | 91 |
| 1.0 | 0.9 | 88 | | |

### 1.1.2 Plate 2

*Table IV: Plate 2 sample layout (Day 1 and Day 2).*

|   | 1 | 2 | 3 | 4 | 5 | 6 | 7 | 8 |
|---|---|---|---|---|---|---|---|---|
| a | cal a | cal a | QC-1 | 24h-1 | 24h-9 | 2d-5 | 8h-1 | 8h-9 |
| b | cal b | cal b | QC-1 | 24h-2 | 24h-10 | 2d-6 | 8h-2 | 8h-10 |
| c | cal c | cal c | QC-2 | 24h-3 | 24h-11 | 2d-7 | 8h-3 | 8h-11 |
| d | cal d | cal d | QC-2 | 24h-4 | 24h-12 | 2d-8 | 8h-4 | 8h-12 |
| e | cal e | cal e | QC-3 | 24h-5 | 2d-1 | 2d-9 | 8h-5 | qc3 |
| f | cal f | cal f | QC-3 | 24h-6 | 2d-2 | 2d-10 | 8h-6 | qc4 |
| g | cal g | cal g | QC-4 | 24h-7 | 2d-3 | 2d-11 | 8h-7 | qc5 |
| h | blank | blank | QC-4 | 24h-8 | 2d-4 | 2d-12 | 8h-8 | qc6 |

*Table V: Plate 2 sample absorbance (Day 1 and Day 2).*

|   | 1 | 2 | 3 | 4 | 5 | 6 | 7 | 8 |
|---|---|---|---|---|---|---|---|---|
| a | 0.601 | 0.704 | 0.886 | 0.461 | 0.733 | 0.972 | 0.635 | 1.313 |
| b | 0.973 | 0.846 | 0.77 | 0.687 | 0.577 | 1.074 | 0.625 | 0.821 |
| c | 1.062 | 1.033 | 0.903 | 0.574 | 0.602 | 0.63 | 0.775 | 0.656 |
| d | 1.33 | 1.254 | 0.938 | 0.772 | 0.616 | 0.747 | 1.161 | 0.639 |
| e | 1.531 | 1.448 | 1.423 | 0.911 | 0.888 | 0.862 | 0.812 | 0.668 |
| f | 1.598 | 1.655 | 1.302 | 0.883 | 0.815 | 0.614 | 0.931 | 0.845 |

(continued)

|   | 1 | 2 | 3 | 4 | 5 | 6 | 7 | 8 |
|---|---|---|---|---|---|---|---|---|
| g | 1.71 | 1.639 | 1.557 | 0.57 | 0.677 | 0.695 | 0.621 | 1.063 |
| h | 1.916 | 1.808 | 1.694 | 0.777 | 0.756 | 0.902 | 0.655 | 1.529 |

*Table VI: Plate 2, Time points from Day 1 and 2.*

| Theroretical conc ng/mL | Measured conc ng/mL | Accuracy % | Average conc ng/mL | Ave accuracy % |
|---|---|---|---|---|
| 10.0 | 10.2 | 102 | 10.8 | 108 |
| 10.0 | 11.4 | 114 | | |
| 5.0 | 10.1 | 202 | 9.9 | 197 |
| 5.0 | 9.7 | 193 | | |
| 2.0 | 2.9 | 145 | 3.7 | 182 |
| 2.0 | 4.4 | 220 | | |
| 1.0 | 1.6 | 159 | 1.1 | 105 |
| 1.0 | 0.5 | 52 | | |

**1.1.3 plate 3**

*Table VII: Plate 3 sample layout (Day 3 to Day 8).*

|   | 1 | 2 | 3 | 4 | 5 | 6 | 7 | 8 | 9 | 10 | 11 | 12 |
|---|---|---|---|---|---|---|---|---|---|---|---|---|
| a | cal a | cal a | QC10 | 3d-1 | 3d-9 | 4.5d-5 | 6d-1 | 6d-9 | 7pre-5 | 7d12h-1 | 7d12h-9 | 8d-5 |
| b | cal b | cal b | QC10 | 3d-2 | 3d-10 | 4.5d-6 | 6d-2 | 6d-10 | 7pre-6 | 7d12h-2 | 7d12h-10 | 8d-6 |
| c | cal c | cal c | QC7 | 3d-3 | 3d-11 | 4.5d-7 | 6d-3 | 6d-11 | 7pre-7 | 7d12h-3 | 7d12h-11 | 8d-7 |
| d | cal d | cal d | QC7 | 3d-4 | 3d-12 | 4.5d-8 | 6d-4 | 6d-12 | 7pre-8 | 7d12h-4 | 7d12h-12 | 8d-8 |
| e | cal e | cal e | QC5 | 3d-5 | 4.5d-1 | 4.5d-9 | 6d-5 | 7pre-1 | 7pre-9 | 7d12h-5 | 8d-1 | 8d-9 |
| f | cal f | cal f | QC5 | 3d-6 | 4.5d-2 | 4.5d-10 | 6d-6 | 7pre-2 | 7pre-10 | 7d12h-6 | 8d-2 | 8d-10 |
| g | cal g | cal g | QC2 | 3d-7 | 4.5d-3 | 4.5d-11 | 6d-7 | 7pre-3 | 7pre-11 | 7d12h-7 | 8d-3 | 8d-11 |
| h | blank | blank | QC2 | 3d-8 | 4.5d-4 | 4.5d-12 | 6d-8 | 7pre-4 | 7pre-12 | 7d12h-8 | 8d-4 | 8d-12 |

*Table VIII: Plate 3 sample absorbance (Day 3 to Day 8).*

|   | 1 | 2 | 3 | 4 | 5 | 6 | 7 | 8 | 9 | 10 | 11 | 12 |
|---|---|---|---|---|---|---|---|---|---|---|---|---|
| a | 0.942 | 0.756 | 0.824 | 0.929 | 1.136 | 1.083 | 1.062 | 0.988 | 0.85 | 1.546 | 1.888 | 2.036 |
| b | 1.088 | 1.086 | 0.668 | 0.933 | 0.577 | 1.295 | 1.051 | 0.556 | 1.281 | 1.465 | 0.678 | 2.045 |
| c | 1.185 | 1.156 | 0.804 | 0.726 | 0.894 | 0.736 | 0.846 | 0.965 | 0.757 | 1.534 | 1.664 | 1.442 |

(continued)

|   | 1 | 2 | 3 | 4 | 5 | 6 | 7 | 8 | 9 | 10 | 11 | 12 |
|---|---|---|---|---|---|---|---|---|---|---|---|---|
| d | 1.344 | 1.304 | 0.752 | 0.954 | 0.912 | 0.935 | 1.033 | 0.947 | 1.015 | 1.684 | 1.814 | 1.766 |
| e | 1.46 | 1.446 | 0.913 | 1.002 | 1.085 | 1.013 | 1.058 | 1.083 | 1.08 | 1.819 | 1.708 | 1.939 |
| f | 1.503 | 1.472 | 0.896 | 1.135 | 0.942 | 0.556 | 1.154 | 1.022 | 0.522 | 1.816 | 1.515 | 0.681 |
| g | 1.482 | 1.445 | 1.268 | 0.741 | 0.939 | 0.823 | 0.642 | 1.051 | 0.851 | 1.059 | 1.291 | 1.907 |
| h | 1.644 | 1.573 | 1.368 | 1.018 | 1.12 | 0.863 | 1.071 | 1.251 | 1.058 | 1.507 | 1.942 | 2.149 |

*Table IX: Plate 3, Time points from Day 3 to 8.*

| Theroretical conc ng/mL | Measured conc ng/mL | Accuracy % | Average conc ng/mL | Ave accuracy % |
|---|---|---|---|---|
| 10.0 | 13.0 | 130 | 13.6 | 136 |
| 10.0 | 14.3 | 143 | | |
| 7.0 | 13.2 | 188 | 13.4 | 191 |
| 7.0 | 13.6 | 194 | | |
| 5.0 | 12.1 | 242 | 12.2 | 243 |
| 5.0 | 12.3 | 245 | | |
| 2.0 | 6.5 | 324 | 5.1 | 257 |
| 2.0 | 3.8 | 189 | | |

**1.1.4 Plate 4**

*Table X: Plate 4 sample layout (Day 9).*

|   | 1 | 2 | 3 | 4 | 5 |
|---|---|---|---|---|---|
| a | cal a | cal a | QC7.5 | 9d-1 | 9d-9 |
| b | cal b | cal b | QC7.5 | 9d-2 | 9d-10 |
| c | cal c | cal c | QC5 | 9d-3 | 9d-11 |
| d | cal d | cal d | QC5 | 9d-4 | 9d-12 |
| e | cal e | cal e | QC2 | 9d-5 | blank |
| f | cal f | cal f | QC2 | 9d-6 | test 1 |
| g | cal g | cal g | qc1 | 9d-7 | test 2 |
| h | blank | blank | qc1 | 9d-8 | test 3 |

*Table XI: Plate 4 sample absorbance (Day 9).*

|   | 1 | 2 | 3 | 4 | 5 |
|---|---|---|---|---|---|
| a | 0.731 | 0.775 | 0.981 | 1.775 | 2.029 |
| b | 0.841 | 0.821 | 0.998 | 1.601 | 0.873 |
| c | 1.005 | 0.843 | 1.148 | 1.451 | 1.895 |
| d | 1.201 | 1.11 | 0.918 | 2.01 | 2.095 |
| e | 1.16 | 1.186 | 1.438 | 1.981 | 2.209 |

(continued)

|   | 1 | 2 | 3 | 4 | 5 |
|---|---|---|---|---|---|
| f | 1.287 | 1.223 | 1.427 | 2.007 | 1.443 |
| g | 1.319 | 1.258 | 1.143 | 1.632 | 1.955 |
| h | 1.354 | 1.259 | 1.027 | 1.814 | 2.109 |

*Table XII: Plate 4, Day 9 time points.*

| Theoretical conc. ng/mL | Measured conc. ng/mL | Accuracy % | Average conc ng/mL | Ave accuracy % |
|---|---|---|---|---|
| 7.0 | 5.3 | 76 | 5.1 | 72 |
| 7.0 | 4.8 | 69 | | |
| 5.0 | 2.1 | 42 | 4.8 | 96 |
| 5.0 | 7.6 | 151 | | |
| 2.0 | 0.4 | 20 | 0.4 | 21 |
| 2.0 | 0.4 | 22 | | |
| 1.0 | 2.1 | 214 | 3.1 | 312 |
| 1.0 | 4.1 | 891 | | |

**Appendix 1.2. JX1302-K005 Part 2 Period 2 Standard curve and QC samples.**

**1.2.1 Plate 5**

*Table XIII: Plate 5 sample layout (Day 0 to Day 1).*

|   | 1 | 2 | 3 | 4 | 5 | 6 | 7 | 8 | 9 | 10 | 11 | 12 |
|---|---|---|---|---|---|---|---|---|---|----|----|----|
| a | d | d | QC-1 | Pre-Tx-1 | Pre-Tx-9 | 2h-5 | 4h-1 | 4h-9 | 6h-5 | 8h-1 | 8h-9 | 24h-5 |
| b | e | e | QC-1 | Pre-Tx-2 | Pre-Tx-10 | 2h-6 | 4h-2 | 4h-10 | 6h-6 | 8h-2 | 8h-10 | 24h-6 |
| c | f | f | QC-2 | Pre-Tx-3 | Pre-Tx-11 | 2h-7 | 4h-3 | 4h-11 | 6h-7 | 8h-3 | 8h-11 | 24h-7 |
| d | g | g | QC-2 | Pre-Tx-4 | Pre-Tx-12 | 2h-8 | 4h-4 | 4h-12 | 6h-8 | 8h-4 | 8h-12 | 24h-8 |
| e | h | h | QC-3 | Pre-Tx-5 | 2h-1 | 2h-9 | 4h-5 | 6h-1 | 6h-9 | 8h-5 | 24h-1 | 24h-9 |
| f | i | i | QC-3 | Pre-Tx-6 | 2h-2 | 2h-10 | 4h-6 | 6h-2 | 6h-10 | 8h-6 | 24h-2 | 24h-10 |
| g | j | j | QC-4 | Pre-Tx-7 | 2h-3 | 2h-11 | 4h-7 | 6h-3 | 6h-11 | 8h-7 | 24h-3 | 24h-11 |
| h | blank | blank | QC-4 | Pre-Tx-8 | 2h-4 | 2h-12 | 4h-8 | 6h-4 | 6h-12 | 8h-8 | 24h-4 | 24h-12 |

*Table XIV: Plate 5 sample absorbance (Day 0 to Day 1).*

| | 1 | 2 | 3 | 4 | 5 | 6 | 7 | 8 | 9 | 10 | 11 | 12 |
|---|---|---|---|---|---|---|---|---|---|---|---|---|
| **a** | 0.736 | 0.679 | 0.858 | 1.412 | 2.049 | 0.473 | 0.392 | 0.363 | 0.515 | 0.625 | 0.421 | 0.787 |
| **b** | 0.817 | 0.756 | 0.925 | 1.597 | 1.483 | 0.637 | 0.499 | 0.662 | 0.694 | 0.528 | 0.662 | 0.879 |
| **c** | 0.949 | 0.889 | 1.228 | 1.41 | 1.94 | 0.702 | 0.548 | 0.586 | 0.728 | 0.586 | 0.533 | 0.574 |
| **d** | 1.007 | 0.893 | 1.117 | 1.342 | 1.94 | 0.56 | 0.712 | 0.584 | 0.522 | 0.693 | 0.678 | 0.641 |
| **e** | 1.086 | 1.05 | 1.44 | 1.996 | 0.583 | 0.49 | 0.602 | 0.556 | 0.514 | 0.637 | 0.539 | 0.597 |
| **f** | 1.489 | 1.377 | 1.362 | 1.978 | 0.502 | 0.621 | 0.683 | 0.501 | 0.633 | 0.737 | 0.634 | 0.77 |
| **g** | 1.66 | 1.542 | 1.319 | 1.714 | 0.608 | 0.597 | 0.689 | 0.603 | 0.612 | 0.705 | 0.57 | 0.78 |
| **h** | 1.772 | 1.158 | 1.341 | 1.519 | 0.965 | 0.535 | 0.547 | 0.717 | 0.619 | 0.562 | 0.772 | 0.674 |

**Table XV: Plate 5 QC sample accuracy (for Day 0 and Day 1 data).**

| Theoretical conc. ng/mL | Measured conc. ng/mL | Accuracy % | Average conc ng/mL | Ave accuracy % |
|---|---|---|---|---|
| 10 | 8.71 | 87 | 7.6 | 76 |
| 10 | 6.58 | 66 | | |
| 5 | 2.98 | 60 | 3.4 | 68 |
| 5 | 3.85 | 77 | | |
| 2 | 1.78 | 89 | 2.0 | 99 |
| 2 | 2.18 | 109 | | |
| 1 | 2.42 | 242 | 2.4 | 235 |
| 1 | 2.29 | 229 | | |

### 1.2.2 Plate 6

*Table XVI: Plate 6 sample layout (Day 2 to Day 7).*

| | 1 | 2 | 3 | 4 | 5 | 6 | 7 | 8 | 9 | 10 | 11 | 12 |
|---|---|---|---|---|---|---|---|---|---|---|---|---|
| **a** | d | d | QC-1 | 2d-1 | 2d-9 | 3d-5 | 4d-1 | 4d-9 | 5.5d-5 | 7d_pre-1 | 7d_pre-9 | 7d_12h-5 |
| **b** | e | e | QC-1 | 2d-2 | 2d-10 | 3d-6 | 4d-2 | 4d-10 | 5.5d-6 | 7d_pre-2 | 7d pre-10 | 7d 12h-6 |
| **c** | f | f | QC-2 | 2d-3 | 2d-11 | 3d-7 | 4d-3 | 4d-11 | 5.5d-7 | 7d_pre-3 | 7d_pre-11 | 7d_12h-7 |
| **d** | g | g | QC-2 | 2d-4 | 2d-12 | 3d-8 | 4d-4 | 4d-12 | 5.5d-8 | 7d_pre-4 | 7d_pre-12 | 7d_12h-8 |
| **e** | h | h | QC-3 | 2d-5 | 3d-1 | 3d-9 | 4d-5 | 5.5d-1 | 5.5d-9 | 7d_pre-5 | 7d_12h-1 | 7d_12h-9 |
| **f** | i | i | QC-3 | 2d-6 | 3d-2 | 3d-10 | 4d-6 | 5.5d-2 | 5.5d-10 | 7d_pre-6 | 7d_12h-2 | 7d_12h-10 |
| **g** | j | j | QC-4 | 2d-7 | 3d-3 | 3d-11 | 4d-7 | 5.5d-3 | 5.5d-11 | 7d_pre-7 | 7d_12h-3 | 7d_12h-11 |
| **h** | blank | blank | QC-4 | 2d-8 | 3d-4 | 3d-12 | 4d-8 | 5.5d-4 | 5.5d-12 | 7d_pre-8 | 7d_12h-4 | 7d_12h-12 |

*Table XVII: Plate 6 sample absorbance (Day 2 to Day 7).*

|   | 1 | 2 | 3 | 4 | 5 | 6 | 7 | 8 | 9 | 10 | 11 | 12 |
|---|---|---|---|---|---|---|---|---|---|----|----|----|
| a | 1.199 | 1.151 | 1.575 | 1.554 | 1.206 | 1.962 | 1.389 | 1.379 | 2.047 | 1.865 | 1.779 | 3.135 |
| b | 1.292 | 1.059 | 1.523 | 1.265 | 1.544 | 1.878 | 1.479 | 1.506 | 1.607 | 1.702 | 1.511 | 3.05 |
| c | 1.429 | 1.205 | 1.903 | 0.891 | 1.349 | 1.414 | 1.194 | 1.57 | 1.151 | 1.595 | 1.883 | 2.042 |
| d | 1.547 | 1.392 | 1.732 | 1.357 | 1.319 | 1.374 | 1.362 | 1.252 | 1.345 | 1.836 | 1.631 | 3 |
| e | 1.704 | 1.682 | 2.222 | 1.35 | 1.552 | 1.223 | 1.606 | 1.315 | 1.472 | 1.943 | 2.7 | 2.966 |
| f | 2.124 | 2.068 | 2.157 | 1.789 | 1.374 | 1.7 | 1.722 | 1.198 | 1.149 | 2.03 | 2.354 | 2.604 |
| g | 2.242 | 2.195 | 2.205 | 1.105 | 1.421 | 1.588 | 1.23 | 1.095 | 1.329 | 1.298 | 2.524 | 2.946 |
| h | 2.477 | 2.455 | 2.321 | 1.305 | 1.582 | 1.66 | 1.7 | 1.272 | 1.596 | 1.778 | 4.494 | 3.025 |

*Table XVIII: Plate 6 QC sample accuracy (for Day 2 to Day 7 data).*

| Theoretical conc. ng/mL | Measured conc. ng/mL | Accuracy % | Average conc ng/mL | Ave accuracy % |
|---|---|---|---|---|
| 10 | 5.12 | 51 | 5.3 | 53 |
| 10 | 5.55 | 56 | | |
| 5 | 2.86 | 57 | 3.4 | 68 |
| 5 | 3.94 | 79 | | |
| 2 | 1.09 | 54 | 1.3 | 63 |
| 2 | 1.43 | 71 | | |
| 1 | 1.18 | 118 | 0.9 | 89 |
| 1 | 0.59 | 59 | | |

**1.2.1 Plate 7**

*Table XIX: Plate 7 sample layout (Day 8 to Day 9).*

|   | 1 | 2 | 3 | 4 | 5 | 6 |
|---|---|---|---|---|---|---|
| a | d | d | QC-1 | 8d-1 | 8d-9 | 9d-5 |
| b | e | e | QC-1 | 8d-2 | 8d-10 | 9d-6 |
| c | f | f | QC-2 | 8d-3 | 8d-11 | 9d-7 |
| d | g | g | QC-2 | 8d-4 | 8d-12 | 9d-8 |
| e | h | h | QC-3 | 8d-5 | 9d-1 | 9d-9 |
| f | i | i | QC-3 | 8d-6 | 9d-2 | 9d-10 |
| g | j | j | QC-4 | 8d-7 | 9d-3 | 9d-11 |
| h | blank | blank | QC-4 | 8d-8 | 9d-4 | 9d-12 |

*Table XX: Plate 7 sample absorbance (Day 8 to Day 9).*

|   | 1 | 2 | 3 | 4 | 5 | 6 |
|---|---|---|---|---|---|---|
| a | 0.737 | 0.62 | 0.838 | 1.524 | 1.753 | 1.487 |

(continued)

|   | 1 | 2 | 3 | 4 | 5 | 6 |
|---|---|---|---|---|---|---|
| b | 0.67 | 0.702 | 0.76 | 1.423 | 1.682 | 1.542 |
| c | 0.833 | 0.744 | 0.979 | 1.277 | 1.727 | 1.25 |
| d | 0.952 | 0.836 | 0.961 | 1.597 | 1.723 | 1.424 |
| e | 1.041 | 0.959 | 1.338 | 1.834 | 1.644 | 1.485 |
| f | 1.339 | 1.277 | 1.31 | 1.792 | 1.401 | 1.406 |
| g | 1.427 | 1.481 | 1.307 | 1.439 | 1.501 | 1.551 |
| h | 1.628 | 1.576 | 1.319 | 1.931 | 1.881 | 1.579 |

*Table XXI: Plate 7 QC sample accuracy (for Day 8 and Day 9 data).*

| Theoretical conc. ng/mL | Measured conc. ng/mL | Accuracy % | Average conc ng/mL | Ave accuracy % |
|---|---|---|---|---|
| 10 | 6.7 | 67 | 7.7 | 77 |
| 10 | 8.7 | 87 | | |
| 5 | 4.6 | 92 | 4.7 | 94 |
| 5 | 4.8 | 96 | | |
| 2 | 1.7 | 85 | 1.8 | 89 |
| 2 | 1.9 | 94 | | |
| 1 | 1.9 | 189 | 1.9 | 185 |
| 1 | 1.8 | 181 | | |

The present embodiments are to be considered in all respects as illustrative and not restrictive.

**Claims**

1. A drug release device comprising a drug release component and a support, wherein:

the drug release component comprises one or more thermoplastic elastomer (TPE) polymers and one or more polyolefins, wherein at least one of the TPE polymers is polystyrene-*block*-poly(ethylene-*co*-butylene)-*block*-polystyrene (SEBS);
the drug release component has a hardness in the range of 20-90 Shore A, as measured by a Type A Durometer in accordance with ASTM D2240, and a melt point in the range of about 80 to 250°C,
the drug release component is impregnated with one or more drugs;
the support has a melt point in the range of about 70-200°C; and
the drug release component is moulded onto the support.

2. The device according to claim 1 wherein the drug release component has a hardness in the range 20-55 Shore A, as measured by a Type A Durometer in accordance with ASTM D2240.

3. The device of claim 1 or 2, wherein the drug release component includes:

a polyolefin selected from the group consisting of polyethylene (PE), polypropylene (PP), polymethylpentene (PMP) and polybutene-1 (PB-1);
one or more drugs selected from the group consisting of: progesterone, megesterol acetate, progestogens, progestins, norethandrolone, delmadinone; antimicrobials including: penicillins, sulfonamides, tetracyclines, lincosamides, neomycin; anthelmintics including: pyrantel, piperizine, praziquantal, seditives such as medetomidine, acepromazine, chlorpromazine, diazepam, thiopentone sodium; analgesics including: fentanyl; and corticosteroids including: flumethasone, Triamcinolone acetonide and prednisolone;

optionally one or more non-TPE polymers selected from the group consisting of polyolefins selected from the group consisting of polyethylene (PE), polypropylene (PP), polymethylpentene (PMP) and polybutene-1 (PB-1); polystyrene, polyesters, polylactic acid, poly(L-lactic acid), polycarbonate, poly(ethylene-*alt*- terephthalate), poly(butylene-*alt*-terephthalate), silicone oil, silicone resin, polydimethylsiloxane, phenyl vinyl methyl silicone, poly[1-(trimethylsilyl)-1-propyne], polyether aryl ketones, poly(ether ether ketone), poly(ether-*alt*-imide), poly(amide-*alt*-imide), poly(ethylene-*co*-vinyl acetate), polycaprolactone, poly(trimethylene terephthalate), polyhydroxyalkanoate, polyhydroxybutyrate, poly(3-hydroxybutyrate-*co*-3-hydroxyvalerate), poly(glycolic acid), poly(lactic-*co*-glycolic acid), poly(vinyl chloride), poly(ether sulfone), polyacrylate, polymethylmethacrylate, polysulfone, polyphenylene sulphide, poly(hydroxylethyl acrylate), polyhydroxylethylmethacrylate, poly-*p*-xylene, polytetrafluoroethylene, fluorinated ethylene propylene, perfluoroalkoxy, ethylene chlorotrifluoroethylene, ethylene tetrafluoroethylene, polyvinyl fluoride, poly(acrylonitrile-*co*-butadiene-*co*-styrene), poly(styrene-*co*-acrylonitrile), cyclic olefin copolymer, poly(methacylate-*co*-acrylonitrile-*co*-butadiene-*co*-styrene), poly(styrene-co-butadiene), acrylics, polyurethanes, acetals, Nylon 6, Nylon 66, Nylon 12 and Nylon 6/12; and

optionally, one or more additives selected from the group consisting of: oils including paraffin, isobutylene oil and silicone oil; biological compatible plasticizers including paraffin oil, silicone oil, isobutylene oil and phthalates; antioxidants including hindered phenols with thiodipropionate synergists; UV stabilisers including benzotriazoles, titanium dioxide or carbon black and/or anti-ozonates including ethylene vinyl acetate (EVA) and microcrystalline waxes; fillers, including calcium carbonate and/or talc; and static dissipative agents including carbon black, carbon nanotubes and carbon fibres.

4.  The device of any one of claim 1-3, wherein the drug release component is adhered to the support.

5.  The device of any one of claims 1-4, wherein the drug release component is adhered to the support by partial melting of the support.

6.  The device according to any one of claims 1-5 wherein the drug release component comprises block copolymer SEBS in an amount of about 30-90 % w/w; one or more thermoplastic polymers selected from the group consisting of polyethylene (PE), polypropylene (PP), polymethylpentene (PMP), polybutene-1 (PB-1) in an amount of about 3-40%w/w; an oil selected from the group consisting of paraffin oil, iosbutylene oil and silicone oil in an amount of about 5-60% w/w; and wherein the drug release component is impregnated with progesterone.

7.  The device according to any one of claims 1-6, wherein the support partially or completely includes a non-brittle plastic with high elastic memory and substantially high flexural modulus, wherein the non-brittle plastic includes one of the following: polypropylene, polyethylene, low density polyethylenes (LDPE), high density polyethylenes (HDPE), poly vinyl chloride (PVC), polystyrene, polyesters, polylactic acid, poly(L-lactic acid), polycarbonate, poly(ethylene-*alt*-terephthalate), poly(butylene-*alt*-terephthalate), silicone oil, silicone resin, polydimethylsiloxane, phenyl vinyl methyl silicone, poly[1-(trimethylsilyl)-1-propyne], polyether aryl ketones, poly(ether ether ketone), poly(ether-*alt*-imide), poly(amide-*alt*-imide), poly(ethylene-*co*-vinyl acetate), polycaprolactone, poly(trimethylene terephthalate), polyhydroxyalkanoate, polyhydroxybutyrate, poly(3-hydroxybutyrate-co-3-hydroxyvalerate), poly(glycolic acid), poly(lactic-*co*-glycolic acid), poly(vinyl chloride), poly(ether sulfone), polyacrylate, polymethylmethacrylate, polysulfone, polyphenylene sulphide, poly(hydroxylethyl acrylate), polyhydroxylethylmethacrylate, poly-*p*-xylene, polytetrafluoroethylene, fluorinated ethylene propylene, perfluoroalkoxy, ethylene chlorotrifluoroethylene, ethylene tetrafluoroethylene, polyvinyl fluoride, poly(acrylonitrile-*co*-butadiene-*co*-styrene), poly(styrene-co-acrylonitrile), cyclo olefin copolymer, poly(methacylate-*co*-acrylonitrile-*co*-butadiene-*co*-styrene), poly(styrene-*co*-butadiene), acrylics, polyurethanes, acetals, Nylon 6, Nylon 66, Nylon 12 and Nylon 6/12.

8.  The device according to any one of claims 1-7, wherein the support is in the form of a spine and the spine optionally has one or more raised nodules that contact the drug release component.

9.  The device according to any one of claims 1-8, wherein the drug release component does not include a rate control barrier.

10. The device according to any one of claims 1-9, wherein the device is configured for insertion into the vaginal cavity of an animal.

11. The device according to any one of claims 1-10, wherein the device comprises

- an elongate body extending from a first end to a second end, and
- two arms attached to and extending from the first end of the elongate body, wherein the two arms have arm tips and are moveable relative to the elongate body.

12. The device according to claim 11 wherein the drug release device is substantially "T" or "Y" shaped in its unrestrained configuration such that the arms are moveable relative to the elongate body in the plane of the "T" or Y" shape.

13. The device according to any one of claims 1 to 12, wherein the device has a surface area in the range of about 50-150 cm$^2$.

14. The device according to any one of claims 1-13, when used as a subcutaneous, intramuscular or intra-vaginal implant.

15. The device according to any one of claims 1-13, for use in delivering a drug to an animal selected from the group consisting of: pigs, hind gut fermenters including horses, ruminant animals including cows, goats, alpacas and smaller animals including dogs and cats and humans.

**Patentansprüche**

1. Arzneimittelfreisetzungsvorrichtung, die eine Arzneimittelfreisetzungskomponente und einen Träger umfasst, wobei:

die Arzneimittelfreisetzungskomponente ein oder mehrere thermoplastische Elastomer- (TPE-) Polymere und ein oder mehrere Polyolefine umfasst, wobei zumindest eines der TPE-Polymere Polystyrol-Block-Poly(ethylen-co-butylen)-Block-Polystyrol (SEBS) ist;
die Arzneimittelfreisetzungskomponente eine Härte im Bereich von 20 bis 90 Shore A, wie mittels Typ-A-Durometer gemäß ASTM D2240 gemessen, und einen Schmelzpunkt im Bereich von etwa 80 bis 250 °C aufweist,
die Arzneimittelfreisetzungskomponente mit einem oder mehreren Arzneimitteln imprägniert ist;
der Träger einen Schmelzpunkt im Bereich von etwa 70 bis 200 °C aufweist; und
die Arzneimittelfreisetzungskomponente auf den Träger aufgeformt ist.

2. Vorrichtung nach Anspruch 1, wobei die Arzneimittelfreisetzungskomponente eine Härte im Bereich von 20 bis 55 Shore A aufweist, wie mittels Typ-A-Durometer gemäß ASTM D2240 gemessen.

3. Vorrichtung nach Anspruch 1 oder 2, wobei die Arzneimittelfreisetzungskomponente Folgendes umfasst:

ein Polyolefin, das aus der aus Polyethylen (PE), Polypropylen (PP), Polymethylpenten (PMP) und Polybuten-1 (PB-1) bestehenden Gruppe ausgewählt ist;
ein oder mehrere Arzneimittel, die aus der aus folgenden bestehenden Gruppe ausgewählt sind: Progesteron, Megesterolacetat, Progestogenen, Progestinen, Norethandrolon, Delmadinon; Antimikrobiotika, einschließlich: Penicillinen, Sulfonamiden, Tetracyclinen, Lincosamiden, Neomycin; Anthelmintika, einschließlich: Pyrantel, Piperizin, Praziquantal, Sedativa, wie z.B. Medetomidin, Acepromazin, Chlorpromazin, Diazepam, Thiopentonnatrium; Analgetika, einschließlich: Fentanyl; und Kortikosteroiden, einschließlich: Flumethason, Triamcinolonacetonid und Prednisolon;
gegebenenfalls ein oder mehrere Nicht-TPE-Polymere, die aus der aus Polyolefinen bestehenden Gruppe ausgewählt sind, die aus der aus folgenden bestehenden Gruppe ausgewählt sind: Polyethylen (PE), Polypropylen (PP), Polymethylpenten (PMP) und Polybuten-1 (PB-1); Polystyrol, Polyestern, Polymilchsäure, Poly(L-milchsäure), Polycarbonat, Poly-(ethylen-alt-terephthalat), Poly(butylen-alt-terephthalat), Silikonöl, Silikonharz, Polydimethylsiloxan, Phenylvinylmethylsilikon, Poly[1-(trimethylsilyl)-1-propin], Polyetherarylketonen, Poly(etheretherketon), Poly(ether-alt-imid), Poly(amid-alt-imid), Poly(ethylen-co-vinylacetat), Polycaprolacton, Poly(trimethylenterephthalat), Polyhydroxyalkanoat, Polyhydroxybutyrat, Poly(3-hydroxybutyrat-co-3-hydroxyvalerat), Poly(glykolsäure), Poly(milch-co-glykolsäure), Poly(vinylchlorid), Poly(ethersulfon), Polyacrylat, Polymethylmethacrylat, Polysulfon, Polyphenylensulfid, Poly(hydroxyethylacrylat), Polyhydroxyethylmethacrylat, Poly-p-xylol, Polytetrafluorethylen, fluoriertem Ethylenpropylen, Perfluoralkoxy, Ethylenchlortrifluorethylen, Ethylentetrafluorethylen, Polyvinylfluorid, Poly(acrylnitril-co-butadien-co-styrol), Poly(styrol-co-acrylnitril), zyklischem Olefincopolymer, Poly(methacylat-co-acrylnitril-co-butadien-co-styrol), Poly(styrol-co-butadien), Acryl, Polyurethanen, Acetalen, Nylon 6, Nylon 66, Nylon 12 und Nylon 6/12; und
gegebenenfalls ein oder mehrere Additive, die aus der aus folgenden bestehenden Gruppe ausgewählt sind: Ölen, einschließlich Paraffin, Isobutylenöl und Silikonöl; biologisch verträglichen Weichmachern, einschließlich Paraffinöl, Silikonöl, Isobutylenöl und Phthalaten; Antioxidationsmitteln, einschließlich gehinderten Phenolen mit Thiodipropionatsynergisten; UV-Stabilisatoren, einschließlich Benzotriazolen, Titandioxid oder Carbon Black und/oder Antiozonaten, einschließlich Ethylenvinylacetat (EVA) und mikrokristallinen Wachsen; Füllstoffen, einschließlich Calciumcarbonat und/oder Talk; und statischen dissipativen Mitteln, einschließlich Carbon Black, Kohlenstoffnanoröhrchen und Kohlenstofffasern.

4. Vorrichtung nach einem der Ansprüche 1 bis 3, wobei die Arzneimittelfreisetzungskomponente an dem Träger anhaftet.

5. Vorrichtung nach einem der Ansprüche 1 bis 4, wobei die Arzneimittelfreisetzungskomponente an dem Träger durch teilweises Schmelzen des Trägers anhaftet.

6. Vorrichtung nach einem der Ansprüche 1 bis 5, wobei die Arzneimittelfreisetzungskomponente das SEBS-Blockcopolymer in einer Menge von etwa 30 bis 90 Gew.-%, ein oder mehrere thermoplastische Polymere, das/die aus der aus Polyethylen (PE), Polypropylen (PP), Polymethylpenten (PMP), Polybuten-1 (PB-1) bestehenden Gruppe ausgewählt ist/sind, in einer Menge von etwa 3 bis 40 Gew.-%, ein aus der aus Paraffinöl, Isobutylenöl und Silikonöl bestehenden Gruppe ausgewähltes Öl in einer Menge von etwa 5 bis 60 Gew.-% umfasst; und wobei die Arzneimittelfreisetzungskomponente mit Progesteron imprägniert ist.

7. Vorrichtung nach einem der Ansprüche 1 bis 6, wobei der Träger teilweise oder vollständig einen nichtspröden Kunststoff mit hohem Formgedächtnis und im Wesentlichen hohem Biegemodul umfasst, wobei der nichtspröde Kunststoff eines der folgenden umfasst: Polypropylen, Polyethylen, Polyethylen mit niedriger Dichte (LDPE), Polyethylen mit hoher Dichte (HDPE), Polyvinylchlorid (PVC), Polystyrol, Polyester, Polymilchsäure, Poly(L-milchsäure), Polycarbonat, Poly(ethylen-alt-terephthalat), Poly(butylen-alt-terephthalat), Silikonöl, Silikonharz, Polydimethylsiloxan, Phenylvinylmethylsilikon, Poly[1-(trimethylsilyl)-1-propin], Polyetherarylketone, Poly(etheretherketon), Poly(ether-alt-imid), Poly(amid-alt-imid), Poly-(ethylen-co-vinylacetat), Polycaprolacton, Poly(trimethylenterephthalat), Polyhydroxyalkanoat, Polyhydroxybutyrat, Poly(3-hydroxybutyrat-co-3-hydroxyvalerat), Poly(glykolsäure), Poly-(milch-co-glykolsäure), Poly(vinylchlorid), Poly(ethersulfon), Polyacrylat, Polymethylmethacrylat, Polysulfon, Polyphenylensulfid, Poly(hydroxylethylacrylat), Polyhydroxylethylmethacrylat, Poly-p-xylol, Polytetrafluorethylen, fluoriertes Ethylenpropylen, Perfluoralkoxy, Ethylenchlortrifluorethylen, Ethylentetrafluorethylen, Polyvinylfluorid, Poly(acrylnitril-co-butadien-co-styrol), Poly(styrol-co-acrylnitril), Cycloolefincopolymer, Poly(methacylat-co-acrylnitril-co-butadien-co-styrol), Poly(styrol-co-butadien), Acryl, Polyurethane, Acetale, Nylon 6, Nylon 66, Nylon 12 und Nylon 6/12.

8. Vorrichtung nach einem der Ansprüche 1 bis 7, wobei der Träger in Form eines Gerüsts vorliegt und das Gerüst gegebenenfalls ein oder mehrere erhöhte Knötchen aufweist, die die Arzneimittelfreisetzungskomponente kontaktieren.

9. Vorrichtung nach einem der Ansprüche 1 bis 8, wobei die Arzneimittelfreisetzungskomponente keine Freisetzungsratensteuerbarriere umfasst.

10. Vorrichtung nach einem der Ansprüche 1 bis 9, wobei die Vorrichtung zum Einbringen in den Vaginalhohlraum eines Tiers ausgelegt ist.

11. Vorrichtung nach einem der Ansprüche 1 bis 10, wobei die Vorrichtung Folgendes umfasst:

- einen länglichen Korpus, der sich von einem ersten Ende zu einem zweiten Ende erstreckt; und
- zwei Schenkel, die an dem ersten Ende des länglichen Korpus befestigt sind und sich von diesem erstrecken,

wobei die zwei Schenkel Schenkelspitzen aufweisen und in Bezug auf den länglichen Korpus bewegbar sind.

12. Vorrichtung nach Anspruch 11, wobei die Arzneimittelfreisetzungsvorrichtung in ihrer nichtblockierten Anordnung im Wesentlichen "T"- oder "Y"-förmig ist, so dass die Schenkel in Bezug auf den länglichen Korpus in der Ebene der "T"- oder "Y"-Form bewegbar sind.

13. Vorrichtung nach einem der Ansprüche 1 bis 12, wobei die Vorrichtung eine Oberfläche im Bereich von etwa 50 bis 150 cm$^2$ aufweist.

**14.** Vorrichtung nach einem der Ansprüche 1 bis 13, die als subkutanes, intramuskuläres oder intravaginales Implantat verwendet wird.

**15.** Vorrichtung nach einem der Ansprüche 1 bis 13 zur Verwendung zur Zufuhr eines Arzneimittels an ein Tier, das aus der aus Schweinen, Caecumverdauern, umfassend Pferde, Wiederkäuern, umfassend Kühe, Ziegen, Alpakas, und kleineren Tieren, umfassend Hunde und Katzen, und Menschen bestehenden Gruppe ausgewählt ist.

**Revendications**

**1.** Dispositif de libération de médicament comprenant un composant de libération de médicament et un support, dans lequel :

le composant de libération de médicament comprend un ou plusieurs polymères élastomères thermoplastiques (TPE) et une ou plusieurs polyoléfines, dans lequel au moins l'un des polymères TPE est un polystyrène-*séquence*-poly(éthylène-*co*-butylène)-block-polystyrène (SEBS) ;

le composant de libération de médicament a une dureté comprise dans la plage allant de 20 à 90 Shore A, telle que mesurée par un duromètre de type A conformément à la norme ASTM D2240, et un point de fusion compris dans la plage allant d'environ 80 à 250 °C,

le composant de libération de médicament est imprégné avec un ou plusieurs médicaments ;

le support a un point de fusion compris dans la plage allant d'environ 70 à 200 °C ; et

le composant de libération de médicament est moulé sur le support.

**2.** Dispositif selon la revendication 1, dans lequel le composant de libération de médicament a une dureté comprise dans la plage allant de 20 à 55 Shore A, telle que mesurée par un duromètre de type A conformément à la norme ASTM D2240.

**3.** Dispositif selon la revendication 1 ou 2, dans lequel le composant de libération de médicament comprend :

une polyoléfine choisie dans le groupe constitué par le polyéthylène (PE), le polypropylène (PP), le polyméthylpentène (PMP) et le polybutène-1 (PB-1) ;

un ou plusieurs médicaments choisis dans le groupe constitué par : la progestérone, l'acétate de mégestérol, les progestatifs, les progestines, la noréthandrolone, la delmadinone ; les antimicrobiens incluant : les pénicillines, les sulfamides, les tétracyclines, les lincosamides, la néomycine ; les anthelminthiques incluant : le pyrantel, la pipérizine, le praziquantal, les sédatifs tels que la médétomidine, l'acépromazine, la chlorpromazine, le diazépam, le thiopentone sodique ; les analgésiques incluant : le fentanyl ; et les corticostéroïdes incluant : la fluméthasone, l'acétonide de triamcinolone et la prednisolone ;

éventuellement un ou plusieurs polymères non TPE choisis dans le groupe constitué par les polyoléfines choisies dans le groupe constitué par le polyéthylène (PE), le polypropylène (PP), le polyméthylpentène (PMP) et le polybutène-1 (PB-1) ; un polystyrène, les polyesters, un acide polylactique, un acide poly(L-lactique), un polycarbonate, un poly(éthylène-*alt*-téréphtalate), un poly(butylène-*alt*-téréphtalate), une huile de silicone, une résine de silicone, un polydiméthylsiloxane, une phénylvinylméthylsilicone, un poly[1-(triméthylsilyl)-1-propyne], les polyéther-arylcétones, une poly(éther-éther-cétone), un poly(éther-*alt*-imide), un poly(amide-*alt*-imide), un poly(éthylène-co-acétate de vinyle), une polycaprolactone, un poly(téréphtalate de triméthylène), un polyhydroxyalcanoate, un polyhydroxybutyrate, un poly(3-hydroxybutyrate-co-3-hydroxyvalérate), un poly(acide glycolique), un poly(acide lactique-co-glycolique), un poly(chlorure de vinyle), une poly(éthersulfone), un polyacrylate, un polyméthacrylate de méthyle, une polysulfone, un polysulfure de phénylène, un poly(acrylate d'hydroxyléthyle), un polyméthacrylate d'hydroxyléthyle, un poly-p-xylène, un polytétrafluoroéthylène, un éthylène-propylène fluoré, un perfluoroalcoxy, un éthylène-chlorotrifluoroéthylène, un éthylène-tétrafluoroéthylène, un polyfluorure de vinyle, un poly(acrylonitrile-*co*-butadiène-*co*-styrène), un poly(styrène-*co*-acrylonitrile), un copolymère d'oléfine cyclique, un poly(méthacrylate-co-butadiène-*co*-styrène), un poly(styrène-*co*-butadiène), les composés acryliques, les polyuréthanes, les acétals, le nylon 6, le nylon 66, le nylon 12 et le nylon 6/12 ; et éventuellement, un ou plusieurs additifs choisis dans le groupe constitué par : les huiles incluant la paraffine, une huile d'isobutylène et une huile de silicone ; les plastifiants biologiques compatibles incluant une huile de paraffine, une huile de silicone, une huile d'isobutylène et les phtalates ; les antioxydants incluant les phénols encombrés avec des synergistes de type thiodipropionate ; les stabilisants UV incluant les benzotriazoles, le dioxyde de titane ou le noir de carbone et/ou les anti-ozonates incluant l'éthylène-acétate de vinyle (EVA) et les cires microcristallines ; les charges incluant le carbonate de calcium et/ou le talc ; et les agents dissipatifs statiques incluant le noir de carbone, les nanotubes de carbone et les fibres de carbone.

4. Dispositif selon l'une quelconque des revendications 1 à 3, dans lequel le composant de libération de médicament adhère au support.

5. Dispositif selon l'une quelconque des revendications 1 à 4, dans lequel le composant de libération de médicament adhère au support par fusion partielle du support.

6. Dispositif selon l'une quelconque des revendications 1 à 5, dans lequel le composant de libération de médicament comprend un SEBS copolymère séquencé en une quantité d'environ 30 à 90 % m/m ; un ou plusieurs polymères thermostatiques choisis dans le groupe constitué par le polyéthylène (PE), le polypropylène (PP), le polyméthylpentène (PMP), le polybutène-1 (PB-1) en une quantité d'environ 3 à 40 % m/m ; une huile choisie dans le groupe constitué par une huile de paraffine, une huile d'isobutylène et une huile de silicone en une quantité d'environ 5 à 60 % m/m ; et dans lequel le composant de libération de médicament est imprégné avec de la progestérone.

7. Dispositif selon l'une quelconque des revendications 1 à 6, dans lequel le support inclut partiellement ou totalement un plastique non cassant ayant une mémoire élastique élevée et un module de flexion sensiblement élevé, dans lequel le plastique non cassant inclut l'un des éléments suivants : un polypropylène, un polyéthylène, les polyéthylènes basse densité (LDPE), les polyéthylènes haute densité (HDPE), un polychlorure de vinyle (PVC), un polystyrène, les polyesters, un acide polylactique, un acide poly(L-lactique), un polycarbonate, un poly(éthylène-*alt*-téréphtalate), un poly(butylène-*alt*-téréphtalate), une huile de silicone, une résine de silicone, un polydiméthylsiloxane, une phénylvinylméthylsilicone, un poly[1-(triméthylsilyl)-1-propyne], les polyéther-arylcétones, une poly(éther-éther-cétone), un poly(éther-*alt*-imide), un poly(amide-*alt*-imide), un poly(éthylène-*co*-acétate de vinyle), une polycaprolactone, un poly(téréphtalate de triméthylène), un polyhydroxyalcanoate, un polyhydroxybutyrate, un poly(3-hydroxybutyrate-co-3-hydroxyvalérate), un poly(acide glycolique), un poly(acide lactique-co-glycolique), un poly(chlorure de vinyle), une poly(éthersulfone), un polyacrylate, un polyméthacrylate de méthyle, une polysulfone, un polysulfure de phénylène, un poly(acrylate d'hydroxyléthyle), un polyméthacrylate d'hydroxyléthyle, un poly-p-xylène, un polytétrafluoroéthylène, un éthylène-propylène fluoré, un perfluoroalcoxy, un éthylène-chlorotrifluoroéthylène, un éthylène-tétrafluoroéthylène, un polyfluorure de vinyle, un poly(acrylonitrile-*co*-butadiène-*co*-styrène), un poly(styrène-*co*-acrylonitrile), un copolymère d'oléfine cyclique, un poly(méthacrylate-*co*-butadiène-co-styrène), un poly(styrène-*co*-butadiène), les composés acryliques, les polyuréthanes, les acétals, le nylon 6, le nylon 66, le nylon 12 et le nylon 6/12.

8. Dispositif selon l'une quelconque des revendications 1 à 7, dans lequel le support est sous la forme d'une tige et la tige a éventuellement un ou plusieurs nodules surélevés qui sont en contact avec le composant de libération de médicament.

9. Dispositif selon l'une quelconque des revendications 1 à 8, dans lequel le composant de libération de médicament n'inclut pas de barrière de régulation du débit.

10. Dispositif selon l'une quelconque des revendications 1 à 9, dans lequel le dispositif est configuré pour une insertion dans la cavité vaginale d'un animal.

11. Dispositif selon l'une quelconque des revendications 1 à 10, dans lequel le dispositif comprend

- un corps allongé s'étendant d'une première extrémité jusqu'à une seconde extrémité, et
- deux bras liés à et s'étendant depuis la première extrémité du corps allongé,
dans lequel les deux bras ont des bouts de bras et sont mobiles par rapport au corps allongé.

12. Dispositif selon la revendication 11, dans lequel le dispositif de libération de médicament a essentiellement la forme d'un « T » ou d'un « Y » dans sa configuration sans retenue de sorte que les bras sont mobiles par rapport au corps allongé dans le plan de la forme « T » ou « Y ».

13. Dispositif selon l'une quelconque des revendications 11 à 12, dans lequel le dispositif a une surface comprise dans la plage allant d'environ 50 à 150 cm$^2$.

14. Dispositif selon l'une quelconque des revendications 11 à 13, lorsqu'il est utilisé en tant qu'implant sous-cutané, intramusculaire ou intra-vaginal.

**15.** Dispositif selon l'une quelconque des revendications 11 à 13, pour une utilisation dans l'administration d'un médicament à un animal choisi dans le groupe constitué par : les porcs, les fermenteurs du gros intestin incluant les chevaux, les animaux ruminants incluant les vaches, les chèvres, les alpagas et les petits animaux incluant les chiens et les chats et les humains.

I

IV

V

II

SECTION B-B

VI

VII

D

DETAIL D

SECTION E-E

III

Fig. 1A

I

D

D

SECTION D-D

A

A

SECTION A-A

B

B

SECTION B-B

FLOW CHANNELS

II

Fig. 1B

150 mm

145 mm

A

D | D

A

Overmold
Polymer Spine

SECTION A-A

III

II

IV

I

V

SECTION D-D

Fig. 1C

SECTION F-F

Fig. 2A

SECTION A-A

Fig. 2B

Fig. 2C

Fig. 3

Fig. 4

Fig. 5

Fig. 6

Fig. 7

Fig. 8

Fig. 9

Fig. 10

Fig. 11

Fig. 12

Fig. 13

Fig. 14

(a)                              (b)

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

JX1302-K005 Part 2 Period 2
Plate 6 Calibration Curve

$y = 7\text{E-}05x^3 + 0.0082x^2 - 0.2139x + 2.4458$

Fig. 29

JX1302-K005 Part 2 Period 2
Plate 7 Calibration Curve

$y = -0.0005x^3 + 0.0174x^2 - 0.2169x + 1.6584$

Fig. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2016900002
- US 2015342894 A
- US 3364549 A
- US 3670633 A
- US 3700633 A
- US 4764572 A
- US 3299174 A

**Non-patent literature cited in the description**

- Styrenic Block Co-polymers. **JIRI GEORGE DROBNY.** Handbook of Thermoplastic Elastomers. Elsivier Inc, 2014
- **DOUGLAS M. BRYCE.** Plastic Injection Moulding: Manufacturing Process Fundamentals. Society of Manufacturing Engineers, 1996, vol. 1
- **H.O. OWEN ; D. F. KATZ.** *Contraception,* 1999, vol. 59, 91
- **LEBLANC, S.J. ; BROES, A.** *Can. Vet. J.,* 2014, vol. 55, 582-584
- **RATHBONE, M. J. et al.** *J. Control. Release,* 1994, vol. 54, 117-148